# EUROPEAN PATENT SPECIFICATION

(11) **EP 3 681 478 B1**
(45) Date of publication and mention of the grant of the patent: **10.09.2025**
(21) Application number: 18853900.1
(22) Date of filing: 10.09.2018
(51) Int. Cl.: A61K 9/06, A61K 9/00, A61K 31/138, A61K 47/06, A61K 47/10, A61K 47/12, A61K 47/14, A61K 47/20, A61K 47/44, A61K 45/06

(54) **TOPICAL COMPOSITIONS AND METHODS FOR TREATMENT**
TOPISCHE ZUSAMMENSETZUNGEN UND VERFAHREN ZUR BEHANDLUNG
COMPOSITIONS TOPIQUES ET MÉTHODES DE TRAITEMENT

(30) Priority: 11.09.2017 US 201762556884 P; 11.09.2017 US 201762556920 P; 11.09.2017 US 201762556799 P; 31.01.2018 US 201862624787 P; 03.07.2018 US 201862693885 P
(43) Date of publication of application: 22.07.2020
(73) Proprietor: Atossa Therapeutics, Inc., Seattle, WA 98125 (US)
(72) Inventor: QUAY, Steven, C., Seattle WA 98104 (US); KUSHWAHA, Avadhesh, S., San Diego CA 92130 (US); KISAK, Edward, T., San Diego CA 92101 (US); NEWSAM, John, M., La Jolla CA 92037 (US)
(74) Representative: Titmus, Craig Edward
(86) International application number: PCT/US2018/050197
(87) International publication number: WO 2019/051370

(56) References cited:
- EP-A1- 3 202 420
- WO-A1-2016/168021
- US-A1- 2007 190 019
- US-A1- 2007 269 379
- US-A1- 2008 319 092
- US-A1- 2012 164 075
- US-A1- 2016 346 230
- US-B2- 8 454 945
- CHOON SIONG MAH ET AL: "A miniaturized flow-through cell to evaluate skin permeation of endoxifen", INTERNATIONAL JOURNAL OF PHARMACEUTICS, vol. 441, no. 1-2, 2013, pages 433 - 440, XP055101012, ISSN: 0378-5173, DOI: 10.1016/j.ijpharm.2012.11.011
- MANISHA PRASHAR ET AL: "SYNERGISTIC ACTION OF PENETRATION ENHANCERS IN TRANSDERMAL DRUG DELIVERY", JOURNAL OF DRUG DELIVERY AND THERAPEUTICS, vol. 4, no. 3, 15 May 2014 (2014-05-15), pages 45 - 51, XP055705360, ISSN: 2250-1177, DOI: 10.22270/jddt.v4i3.824

## Description

### CROSS-REFERENCE TO RELATED APPLICATIONS

This application claims the benefit of U.S. Patent Application No. 62/556,884, filed September 11, 2017; U.S. Patent Application No. 62/556,920, filed September 11, 2017; U.S. Patent Application No. 62/556,799, filed September 11, 2017; U.S. Patent Application No. 62/624,787, filed January 31, 2018; and U.S. 62/693,885, filed July 3 2018.

### BACKGROUND

Breast cancer is by far the most common form of cancer in women, and it is the second leading cause of cancer death in humans. Despite advances in diagnosing and treating breast cancer, the prevalence of this disease has been steadily rising at a rate of about 1% per year since 1940. Today, the likelihood that a woman living in North America will develop breast cancer during her lifetime is one in eight. In addition to breast cancers, other breast disorders affecting a large number of women include benign but often precancerous lesions, such as ductal hyperplasia, lobular hyperplasia, atypical ductal hyperplasia, and atypical lobular hyperplasia.

Current best practice for the treatment of breast cancer is to diagnose breast cancer with mammography and then treat the patient with surgery, radiation therapy, and chemotherapy. The current widespread use of mammography has resulted in improved detection of breast cancer. Although breast cancer is about 100 times less common among men than among women, the lifetime risk of men getting breast cancer is about 1 in 1,000. About 2,470 new cases of invasive breast cancer will be diagnosed in men and about 460 men will die from breast cancer. American Cancer Society estimates that in 2017, 255,180 new cases of breast cancer will be diagnosed in both men and women combined and 41,070 subjects will die of breast cancer. Nonetheless, the death rate due to breast cancer has remained relatively unchanged at about 21 deaths per 100,000 women and 0.4 deaths per 100,000 men. All too often, breast cancer is discovered at a stage that is too far advanced, when therapeutic options and survival rates are severely limited.

Breast cancers include any malignant tumor of breast cells. There are several types of breast cancer. Exemplary breast cancers include, but are not limited to, ductal carcinoma in situ, lobular carcinoma in situ, invasive (or infiltrating) ductal carcinoma, invasive (or infiltrating) lobular carcinoma, inflammatory breast cancer, triple-negative breast cancer, ER+ breast cancer, HER2+ breast cancer, adenoid cystic (or adenocystic) carcinoma, low-grade adenosquamous carcinoma, medullary carcinoma, mucinous (or colloid) carcinoma, papillary carcinoma, tubular carcinoma, metaplastic carcinoma, and micropapillary carcinoma. A single breast tumor can be a combination of these types or be a mixture of invasive and in situ cancer.

Tamoxifen is a selective estrogen receptor modulator that is used for the treatment of women with endocrine responsive breast cancer, i.e. hormone-dependent or hormone-sensitive breast cancer. Adjuvant therapy primarily via oral delivery of tamoxifen is known to have severe side effects in some subjects such as vasomotor symptoms, for example hot flashes, and reproductive tract (gynecologic) cancers. Patient compliance remains a problem with tamoxifen therapy. Further, the majority of the individuals on adjuvant tamoxifen therapy do not respond to the drug and 30 - 50% of the patients subsequently die of their disease.

Several cytochrome P450 (CYP) mutations have been proposed to cause reduced conversion of tamoxifen to its active metabolite, endoxifen, and reduce tamoxifen efficacy and increase resistance to the drug. So far, over 140 allelic variants of CYP2D6 have been described and a substantial part of these are associated with reduced or absent activity of the encoded enzyme. Based on the combination of the carried alleles, each individual subject can be classified into one of four phenotypic groups: poor metabolizer (PM), intermediate metabolizer (IM), extensive metabolizer (EM) and ultra-rapid metabolizer (UM), reflecting variations in levels of serum endoxifen. However, changes in the CYP genotype do not fully explain the tamoxifen resistance and the reduced endoxifen levels observed in as many as 50% of the subjects.

Therefore, several alternatives to tamoxifen are being developed for the treatment of breast cancer, which include various selective estrogen receptor modulators (SERDs) (such as raloxifene, toremifene, and tamoxifen's active metabolites, afimoxifene (*see*, U.S. Patent Nos. 7,485,623; 7,507,769; 7,704,516; 7,786,172; 7,968,532; and 8,048,927), endoxifen (*see*, US9333190; U.S. Patent Nos. 9,220,680; 9,090,640; and 9,200,045; U.S. Publication Nos. 2009/0291134 and US20100112041), and their derivatives (*see*, U.S. Patent Nos. 8,063,249; U.S. Publication Nos. 2015/0080339 and 2014/0193334)), selective estrogen receptor downregulators (SERDs) such as fulvestrant, and aromatase inhibitors (AI) such as exemestane, letrozole, and anastrozole.

It is widely accepted that (Z)-endoxifen is the main active metabolite responsible for the clinical efficacy of tamoxifen. Hydrochloride and citrate salts of endoxifen are known in the art primarily for oral administration and currently under evaluation for metastatic cancer (*See*, *e.g.*, Fauq et al., Bioorganic & Medicinal Chemistry Letters. 20 (2010) 3036 - 3038; Stearns et al., J. Natl. Cancer Inst. Vol 95, No. 23, 2003; US Publication Nos. 2009/0291134 and 2010/0112041; Clinical Trials Gov. Identifier Nos. NCT01273168 and NCT02311933; Goetz et al., 2015, San Antonio Cancer Symposium; Ahmad et al., Clinical Pharmacology & Therapeutics. 88(6) 814-817, 2010; and J Clin. Oncol. 30, 2012 (suppl; abstr 3089)).

Topical endoxifen offers the possibility of achieving local therapeutic benefits while reducing or eliminating systemic side effects such as those observed with tamoxifen. However, skin is a significant barrier to drug permeation and despite several decades of significant research, the success of transdermal or topical drug delivery remains fairly limited to mostly lipophilic drugs with only a small number of transdermal/topical drugs commercially available. Various interactions in connection with topical or transdermal dosage forms can affect the release of active ingredients from topical dosage forms, including drug-skin interaction, drug-vehicle interaction, and vehicle-skin interaction (Robert, M.S., Structure-permeability considerations in percutaneous absorption. In Prediction of Percutaneous Penetration. ed. by R. C. Scott et al., vol. 2, pp. 210-228, IBC Technical Services, London, 1991). Further, various factors can thus affect absorption rates and penetration depth including the active ingredient, the vehicle, the pH, and the relative solubility of the active in the vehicle versus the skin (Ostrenga J. et al., Significance of vehicle composition I: relationship between topical vehicle composition, skin penetrability, and clinical efficacy, Journal of Pharmaceutical Sciences, 60: 1175-1179 (1971)). More specifically, permeability can be affected by drug attributes such as solubility, size and charge, as well as, vehicle attributes such as the drug dissolution rate, spreading-ability, adhesion, and ability to alter membrane permeability.

US 2012/0164075 provides compositions containing endoxifen, formulations and liposomes of endoxifen, methods of preparation of such agents and formulations, and use of such agents and formulations for the treatment of a subject having or at risk for psychiatric and neurodegenerative diseases, infectious diseases, fertility disorders, osteoporosis, osteoarthritis, and/or cardiovascular diseases.

There is very little prior work on skin-applied formulations of endoxifen (Lee et. al., Cancer Chemother Pharmacol 2015, 76:1235-1246; Breast Cancer (Dove Med Press) 2011, 3:61-70; and Mah et. Al., Int J Pharm 2013, 441:433-440). (Z)-endoxifen converts to its inactive and deleterious form, the (E)-isomer, in aqueous conditions, generally remaining at equilibrium at about 1:1 ratio. While Ahmad et al. also disclose certain oils and hydroalcoholic gels and solutions comprising lipid complexes of endoxifen free base and salts (U.S. Patent Pub. No. 20100112041), there remains an unmet medical need for new topical compositions comprising (Z)-endoxifen and salts and solvates thereof wherein there is reduced conversion of (Z)-endoxifen to (E)-endoxifen, and methods for the treatment and/or prevention of hormone-dependent breast and reproductive tract disorders using such compositions.

### SUMMARY OF THE DISCLOSURE

It is an object of the present disclosure to provide novel topical compositions comprising endoxifen, and salts and solvates thereof.

It is another object of the present disclosure to provide novel topical compositions for topical, transdermal, transpapillary, and intraductal administration of endoxifen, and salts and solvates thereof.

It is another object of the present disclosure to provide novel and stable compositions of (Z)-endoxifen, and salts and solvates thereof.

It is another object of the present disclosure to provide novel topical compositions of endoxifen, and salts and solvates thereof capable of permeating through the skin into underlying tissues with low systemic exposure.

It is another object of the present disclosure to provide methods of making novel and stable compositions of endoxifen, and salts and solvates thereof.

Described herein are topical compositions for use in methods for treatment of subjects having or at risk of having a hormone-dependent breast disorder, a hormone-dependent reproductive tract disorder, or both.

Accordingly, the present invention is directed to a topical composition comprising:
a. endoxifen or a salt or a solvate thereof;
b. a first compound; and
c. a second compound; and
wherein the first compound comprises:
i. diethyleneglycol monoethyl ether, and the second compound comprises a penetration enhancer selected from the group consisting of dimethyl sulfoxide (DMSO), diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof; or
ii.DMSO and the second compound comprises a penetration enhancer selected from the group consisting of diethyleneglycol monoethyl ether, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof.

In various embodiments, the total concentration of the first compound and the second compound is up to about 95%, up to about 90%, up to about 85%, up to about 80%, up to about 75%, up to about 70%, up to about 65%, up to about 60%, up to about 55%, up to about 50%, up to about 45%, up to about 40%, up to about 35%, up to about 30%, up to about 25%, up to about 20%, up to about 15%, up to about 10%, or up to about 5% w/w of the topical composition.

In other embodiments, the total concentration of the first compound and the second compound ranges from 10% to 90% w/w of the composition.

In still other embodiments, the ratio of the first compound to second compound ranges from 1:9 to 9:1. In yet other embodiments, the ratio of the first compound to second compound ranges from 1:4 to 4:1. In further embodiments, the ratio of the first compound to second compound ranges from 1:2 to 2:1. In still further embodiments, the ratio of the first compound to second compound is about 1:1.

In other embodiments, the total concentration of first compound ranges from 10% to 90% (w/w) of the final composition, and the total concentration of the second compound ranges from 5% to 80% (w/w) of the final composition.

In some embodiments, the topical composition comprises 0.01% to 10% (w/w) of (Z)-endoxifen free base or a salt or a solvate thereof. In other embodiments, the endoxifen salt is endoxifen gluconate, endoxifen HCl and endoxifen citrate, or solvates thereof.

In certain embodiments, the present disclosure relates to topical compositions wherein the endoxifen or a salt or a solvate thereof comprises: (a) at least 40% (Z)-endoxifen free base (w/w) with respect to total endoxifen in the final composition; or (b) (Z)-endoxifen and (E)-endoxifen at a Z:E ratio ranging from 99:1 to 1:99, from 90:10 to 10:90, from 85:15 to 15:85, from 80:20 to 20:80, from 75:25 to 25:75, from 70:30 to 30:70, from 65:35 to 35:65, from 60:40 to 40:60, from 55:45 to 45:55, and about 50:50.

In certain embodiments, the topical composition of the present embodiments comprises: (a) 0.01% to 10% (w/w) of (Z)-endoxifen free base or salts or solvates thereof; (b) a first compound comprising diethyleneglycol monoethyl ether; (c) a second compound comprising isopropanol and mineral oil.

In certain other embodiments, the topical composition of the present disclosure comprises: (a) 0.01% to 10% (w/w) of (Z)-endoxifen free base or salts or solvates thereof; (b) a first compound comprising diethyleneglycol monoethyl ether; (c) a second compound comprising isopropanol and caprylic/capric glycerides.

In some embodiments, the topical composition of the present disclosure comprises: (a) 0.01% to 10% (w/w) of (Z)-endoxifen free base or salts or solvates thereof; (b) a first compound comprising 15% to 45% (w/w) diethyleneglycol monoethyl ether; (c) a second compound comprising 5% to 30% (w/w) isopropanol; 25% to 45% (w/w) caprylic/capric trigycerides; and (d) q.s. 100% (w/w) with mineral oil.

In other embodiments, the topical compositions of the present disclosure comprise any of Sample Nos. 1 to 8 and Sample Nos. 11 to 14 of Table No. 4.

In an aspect of the present disclosure, the topical compositions have a water content of less than 1% or wherein the topical compositions have a dielectric constant of less than 50 or both.

In another aspect, the topical compositions further comprise a second therapeutic agent. In various embodiment, the second therapeutic agent selected from the group consisting of bicalutamide, enzalutamide, abiraterone acetate, an oncology drug such as antineoplastics such as capecitabine (Xeloda), carboplatin (Paraplatin), cisplatin (Platinol), cyclophosphamide (Neosar), docetaxel (Docefrez, Taxotere), doxorubicin (Adriamycin), pegylated liposomal doxorubicin (Doxil), epirubicin (Ellence), fluorouracil (5-FU, Adrucil), gemcitabine (Gemzar), methotrexate (multiple brand names), paclitaxel (Taxol), protein-bound paclitaxel (Abraxane), vinorelbine (Navelbine), eribulin (Halaven), ixabepilone (Ixempra), goserelin acetate, trastuzumab, ado-trastuzumab, bevacizumab, everolimus, check point inhibitors (such as pembrolizumab (Keytruda^{™}), nivolumab (Opdivo^{™}), atezolizumab (Tecentriq^{™}), durvalumab (Imfinzi^{™}), and avelumab (Bavencio^{™}), and inhibitors of ABC-binding cassette reporters such as BCRP inhibitors and P-gp inhibitors.

In some embodiments, the topical compositions of the present disclosure further comprise a pharmaceutically acceptable excipient.

In another aspect, the topical compositions of the present disclosure further comprise a thickening agent, a penetration enhancer, an emollient, a surfactant, an antioxidant, an antimicrobial, a controlled-release agent, a skin care active, or a combination thereof.

In an aspect, the present disclosure relates to the stability of the topical compositions disclosed herein. In some embodiments, the topical composition is stable at ambient temperature for at least 18 months.

In one aspect, the topical composition comprising endoxifen or a salt or a solvate thereof is formulated at a unit dose of endoxifen or a salt or a solvate thereof of 0.01 mg, 0.05 mg, 0.1 mg, 0.25 mg, 0.5 mg, 0.75 mg, 1 mg, 1.5 mg, 2 mg, 3 mg, 4 mg, 5 mg, 6 mg, 7 mg, 8 mg, 9 mg, 10 mg, 15 mg, 20 mg, 25 mg, 50 mg, 75 mg, 100 mg, and 200 mg.

In another aspect, the present disclosure relates to the topical composition according to claim 1 for use in a method of treating a subject having or at risk of having a hormone-dependent breast disorder, a hormone-dependent reproductive tract disorder, or both, comprising administering a topical composition disclosed herein. In some embodiments, the hormone-dependent breast disorder or the hormone-dependent reproductive tract disorder is a benign breast disorder, hyperplasia, atypia, atypical ductal hyperplasia, atypical lobular hyperplasia, increased breast density, gynecomastia, McCune-Albright Syndrome, precocious puberty, DCIS, LCIS, breast cancer, endometrial cancer, ovarian cancer, uterine cancer, cervical cancer, vaginal cancer, or vulvar cancer. In some embodiments, the subject has prostate cancer; and the subject has or is at risk of having gynecomastia; or the subject has initiated or is about to initiate chemotherapy.

Described herein is a patient population that is benefited by the topical compositions disclosed herein. In some embodiments, the present disclosure relates to the topical composition according to the claims for use in a method of treating a subject having or at risk of having a hormone-dependent breast disorder, a hormone-dependent reproductive tract disorder, or both, wherein the subject having or at risk of having the hormone-dependent breast disorder or hormone-dependent reproductive tract disorder is tamoxifen-refractory or tamoxifen resistant.

In some embodiments, the present disclosure relates to the topical composition according to the claims for use in a method of treating a subject having or at risk of having a hormone-dependent breast disorder, a hormone-dependent reproductive tract disorder, or both, wherein a topical composition comprising endoxifen or a salt or a solvate thereof is administered to a subject at a unit dose of endoxifen or a salt or a solvate thereof ranging from 0.01 mg to 200 mg.

In other embodiments, the present disclosure relates to the topical composition according to the claims for use in a method of treating a subject having or at risk of having a hormone-dependent breast disorder, a hormone-dependent reproductive tract disorder, or both, wherein a topical composition comprising (Z)-endoxifen or a salt or a solvate thereof is administered to the subject at a unit dose of (Z)-endoxifen or a salt or a solvate thereof ranging from 0.01 mg to 100 mg.

In yet other embodiments, the present disclosure relates to the topical composition according to the claims for use in a method of treating a subject having or at risk of having a hormone-dependent breast disorder, a hormone-dependent reproductive tract disorder, or both, wherein a topical composition comprising endoxifen or a salt or a solvate thereof is administered to the subject at a dose of (Z)-endoxifen or a salt or a solvate thereof of 2.0 mg, 6 mg, and 10 mg.

In still other embodiments, the present disclosure relates to the topical composition according to the claims for use in a method of treating a subject having or at risk of having a hormone-dependent breast disorder, a hormone-dependent reproductive tract disorder, or both, wherein a topical composition comprising endoxifen or a salt or a solvate thereof is administered to the subject at a dose of (Z)-endoxifen or a salt or a solvate thereof of 1.0 mg, 3.0 mg, and 5 mg per breast.

In another aspect, the present disclosure relates to the topical composition according to the claims for use in a method of treating a subject having or at risk of having a hormone-dependent breast disorder, a hormone-dependent reproductive tract disorder, or both, wherein the administration of a composition comprising endoxifen or a salt or a solvate thereof to a subject maintains the subject's plasma endoxifen at a steady state level of ≤ 30 nM.

In one aspect, not according to the claims, herein provided is a method for making a topical composition comprising combining: (a) endoxifen or a salt or a solvate thereof; (b) a first compound; and (c) a second compound; and wherein the first compound and second compound are different, and each is selected from the group consisting of DMSO, diethyleneglycol monoethyl ether, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof; and wherein the composition is stirred until the composition combines into a clear homogeneous solution.

Described herein is a kit for treatment or prevention of hormone-dependent breast disorder or hormone dependent reproductive tract disorder, in a subject in need thereof comprising: (a) endoxifen free base or a salt or a solvate thereof; (b) a sealed container for housing the composition; and c) instructions for use of the composition.

In one aspect, the present disclosure provides the topical composition according to the claims for use in a method for the treatment of a subject having or at risk of having a hormone-dependent breast disorder, a hormone-dependent reproductive tract disorder, or both, the use comprising administering the composition provided in a kit disclosed herein in accordance with the instruction provided in the kit.

In another aspect, the present disclosure provides for the topical composition disclosed herein for use in the treatment of a subject having or at risk of having a hormone-dependent breast disorder, a hormone-dependent reproductive tract disorder, or both, the use comprising: (a) dosing the subject with a first composition comprising tamoxifen; (b) determining or having determined the subject's tamoxifen-metabolites profile in a test sample obtained from the subject; (c) determining or having determined if the subject's plasma endoxifen level is low based on subject's tamoxifen-metabolites profile as to compared to a level of plasma endoxifen in a reference tamoxifen-metabolites profile; and (d) administering the topical composition to the subject; and wherein the composition does not comprise tamoxifen. In some embodiments, upon administration of the topical composition, the subject's plasma endoxifen level is maintained at a steady level of ≤ 30 nM.

### DESCRIPTION OF THE DRAWINGS

The foregoing aspects and many of the attendant advantages of this disclosure will become more readily appreciated as the same become better understood by reference to the following detailed description, when taken in conjunction with the accompanying drawings, wherein:

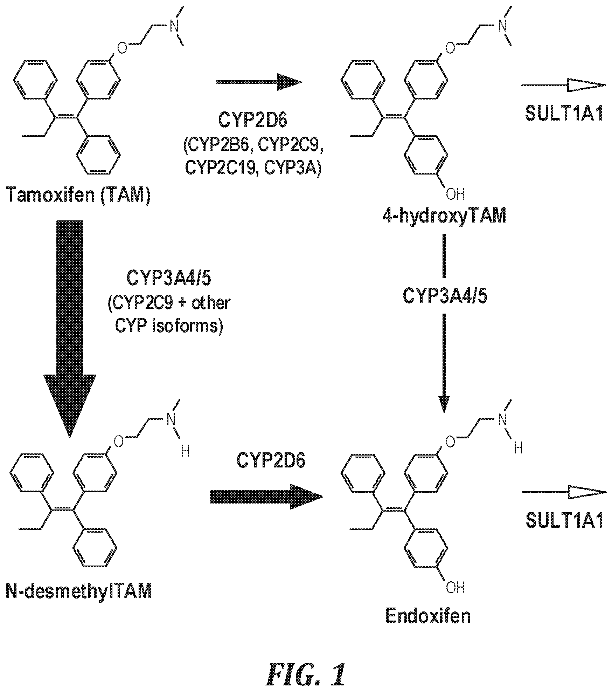

FIGURE 1 is a scheme depicting the metabolic route of tamoxifen to endoxifen and other metabolites. The plasma concentrations of 4-hydroxytamoxifen are low relative to those of N-desmethyl tamoxifen, indicating that the primary route for metabolism of the parent drug via N-demethylation by cytochrome P450 3A4 (CYP3A4) to N-desmethyl tamoxifen, followed by hydroxylation by Cytochrome P450 2D6 (CYP2D6).

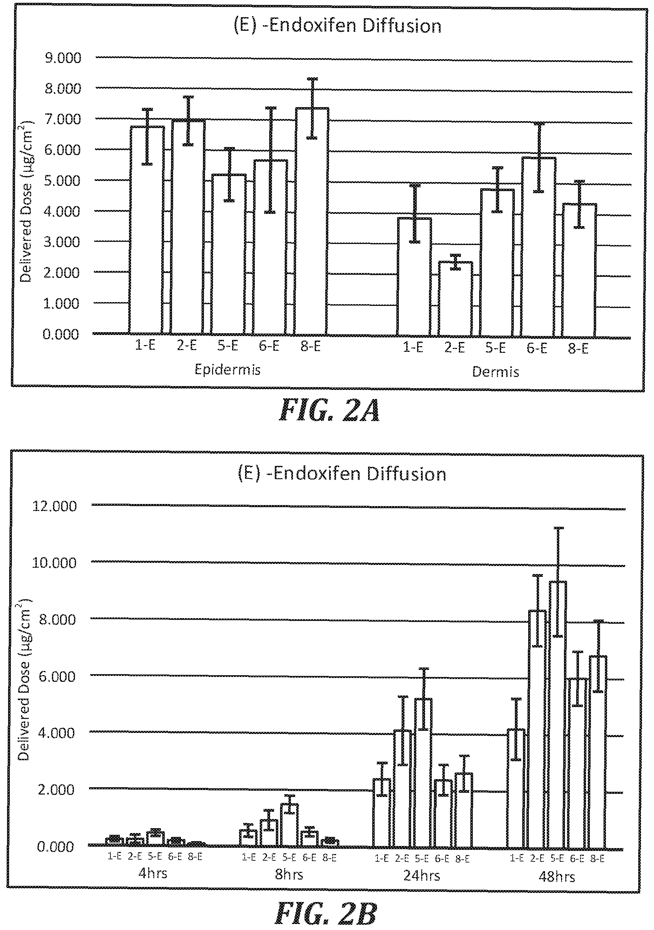

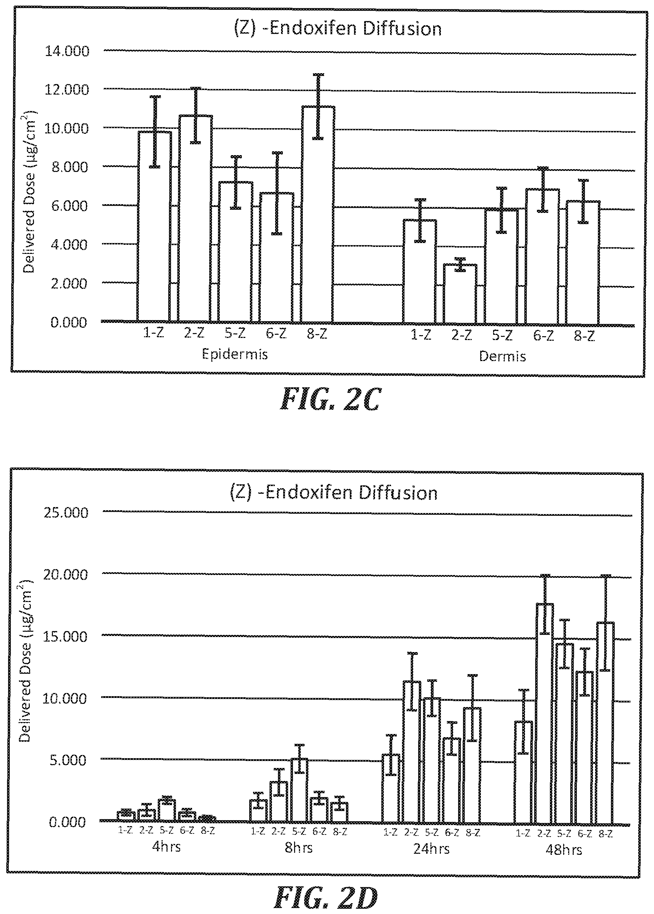

FIGURE 2 shows the results of skin permeation study conducted using Franz Diffusion Cells apparatus wherein the ability of various topical compositions to diffuse into and across the skin (epidermis and dermis) layers are provided. FIG 2A and 2B show the diffusion of (E)-endoxifen into the epidermis and dermis and across the skin tissue to the underlying solution. FIG 2C and 2D show the diffusion of (Z)-endoxifen into the epidermis and dermis and across the skin tissue to the underlying solution.

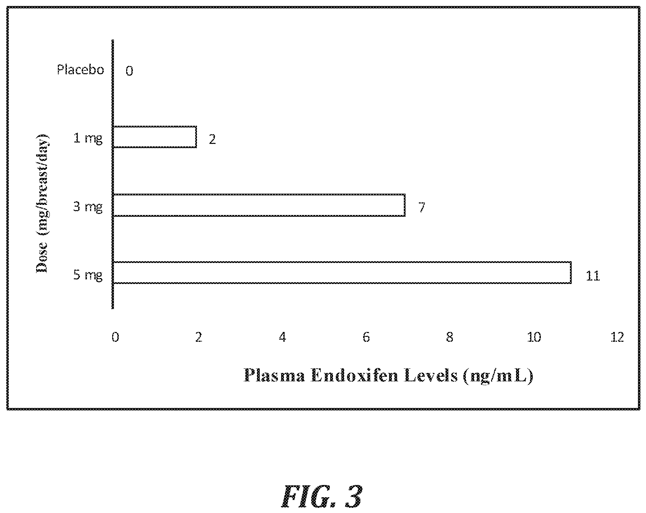

FIGURE 3 shows the number of plasma samples with plasma endoxifen levels ≥ 2 ng/mL in subjects dosed with placebo (0 mg/breast/day), 1 mg/breast/day (total 2 mg/day), 3mg/breast/day (6 mg/day), or 5 mg (10 mg/day) topical endoxifen as described in **Example 9.** Samples from each subject in each dosage group were analyzed to determine the plasma endoxifen levels by Quest Laboratory. Tamoxifen-metabolite LCMS/MS assay having endoxifen analyte sensitivity level of 0.15 ng/mL.

### DETAILED DESCRIPTION

As used herein, the terms "a," "an," and "the" include plural reference unless the context dictates otherwise.

As used herein, the terms "active pharmaceutical ingredient", "active ingredient", "API," "drug substance," "active," "actives," "active agent," or "therapeutic agent" may be used interchangeably to refer to the pharmaceutically active compound(s) in a pharmaceutical composition. This is in contrast to other ingredients in the compositions, such as excipients, which are substantially or completely pharmaceutically inert. The therapeutic agent as used herein includes the active compound as free base and its salts or solvates thereof.

As used herein the term "drug" means a compound intended for use in diagnosis, cure, mitigation, treatment, and/or prevention of disease in man or other animals, and a "Drug Substance" means a finished product which comprises the API.

As used herein, "adjuvant therapy" refers to a therapy that follows a primary therapy and that is administered to subjects at risk of relapsing. Adjuvant systemic therapy in case of breast cancer or reproductive tract cancer, for example with tamoxifen, usually begins soon after primary therapy to delay recurrence, prolong survival or cure a subject.

As used herein, the term "tamoxifen" refers to (Z)-2-[4-(1,2-diphenyl-1-butenyl)phenoxy]-N,N-dimethylethanamine. Tamoxifen can also refer to E-isomer or a combination of E-isomer and Z-isomer.

As used herein, the terms "4-hydroxytamoxifen," "afimoxifene," and "4-OHT" used interchangeably refer to 4-1-[4-[2-(dimethylamino)ethoxy]phenyl]-2-phenylbut-1-enyl]phenol.

As used herein, the term "clomifene" refers to 2-[4-(2-Chloro-1,2-diphenylethenyl)phenoxy]-N,N-dimethylethanamine.

As used herein, the term "droloxifene" refers to 3- [(IE)- 1-[4- [2- (Dimethylamino)ethoxy]phenyl]-2-phenyl- 1 -butenyl]phenol.

As used herein, the term "endoxifen" refers to 4-hydroxy-N-desmethyl-tamoxifen.

As used herein, the term "raloxifene" refers to [6-Hydroxy-2-(4-hydroxyphenyl)benzo[b]thien-3-yl][4-[2-(1-piperidinyl)eth- oxy]-phenyl]methanone.

As used herein, the term "toremifene" refers to 2-[4-(1Z)-4-Chloro-1,2-diphenyl-1-butenyl)phenoxy]-N,N-dimethylethanamine.

As used herein, the term "fulvestrant" refers to 7a-[9-(4,4,5,5,5-Pentafluoropentylsulphhinyl)nonyl]oestra- 1 ,3,-5 (10)-triene-3 , 17 β-diol, (7a, 17β)-7- { 9-[(4,4,5,5,5pentafluoropentyl)sulfinyl] nonyl}estra-l,3,5(10)-triene-3,17-diol or ICI 182,780.

As used herein, the term "anastrozole" refers to 2,2'-[5-(1H-1,2,4-triazol-l-ylmethyl)- 1 ,3-phenylene]bis(2-methylpropanenitrile).

As used herein and in the claims, the terms "comprising," "containing," and "including" are inclusive, open-ended and do not exclude additional unrecited elements, compositional components or method steps. Accordingly, the terms "comprising" and "including" encompass the more restrictive terms "consisting of" and "consisting essentially of"

As used herein, the term "dosage form" means the form in which the compounds or compositions of the present disclosure are delivered to a patient. The dosage form refers to the compounds or compositions of the present disclosure delivered to a subject in any form suitable for its route of administration or delivery, for example, without limitation, topical, transdermal, transpapillary and intraductal delivery.

As used herein, the term "Combination Therapy" refers to the use of an inventive composition described herein in combination with one or more of any prophylactic agent, therapeutic agent or treatment. The combination can refer to inclusion of a second therapeutic or prophylactic agent in a same composition as an inventive composition disclosed herein (for example, in the same topical formulation) or in separate compositions (for example, in 2 different topical formulations). The separate composition may be in a different dosage form (for example, in one topical formulation and the other an oral capsule). Treatment in Combination Therapy can be any treatment such as chemotherapy, radiotherapy, surgery and the like. The use of the terms "Combination Therapy" and "in combination with" does not restrict the order in which an inventive composition described herein and prophylactic and/or therapeutic agent and/or treatment are administered to a subject in need thereof. Compositions of the present disclosure can be administered prior to (e.g., 1 minute (min), 5 min, 15 min, 30 min, 45 min, 1 hour (h), 2 h, 4 h, 6 h, 8 h, 10 h, 12 h, 24 h, 36 h, 48 h, 72 h, 96 h, 1 week (wk), 2 wk, 3 wk, 4 wk, 5 wk, 6 wk, 8 wk, 12 wk, 6 months (m), 9 m, or 1 year before), concomitant with, or subsequent to (e.g., 1 minute (min), 5 min, 15 min, 30 min, 45 min, 1 hour (h), 2 h, 4 h, 6 h, 8 h, 10 h, 12 h, 24 h, 36 h, 48 h, 72 h, 96 h, 1 week (wk), 2 wk, 3 wk, 4 wk, 5 wk, 6 wk, 8 wk, 12 wk, 6 months (m), 9 m, or 1 year after) administration of one or more prophylactic and/or therapeutic agents or treatment to a subject in thereof.

As used herein, the terms "controlled-release" and "modified release" refer to the release of a therapeutic agent in a way that deviates from immediate (conventional) release. Such modified release includes sustained or extended release, targeted release, delayed release (delayed onset), pulsatile (fractional) release. As used herein, the terms "sustained-release" and "extended-release" are used interchangeably and refer to the slower release of the administered therapeutically active agent over a longer period of time than a comparable immediate/conventional release formulation, resulting in levels of the therapeutically active agent or an API in the affected tissues elevated over baseline for a longer period of time than for a comparable immediate-release formulation.

As used herein, the terms "condition," "disorder," and "disease," may be used interchangeably.

As used herein, the terms "test sample" means sample of blood obtained from a subject. It is to be understood that when blood sample is obtained from a subject, subject's blood is used for determining the subject's endoxifen levels and/or other biomarkers that may be measured or tested.

As used herein "plasma endoxifen" is used to refer to endoxifen levels in the subject's test sample whether the test is conducted on whole blood, plasma or serum.

As used herein, the terms "biologically acceptable," "pharmaceutically acceptable" or "pharmacologically acceptable" means materials, compositions, or vehicles that are compatible with other ingredients of the formulation and that they do not substantially produce adverse reactions, e.g., toxic, allergic, or immunological reactions, when administered to a subject. They may be approved by a regulatory agency, e.g., of the U.S. Federal or state government or listed in the U.S. pharmacopeia or other generally recognized pharmacopeia for use in animals, and more particularly in humans.

As used herein, the terms "biologically acceptable carrier," "pharmaceutically acceptable carrier" or "carrier" means a pharmaceutically acceptable material, composition, or vehicle, such as a liquid or solid filler, diluent, excipient, solvent, or encapsulating material, involved in carrying or transporting one or more of the compounds of the present disclosure from one tissue, organ, or portion of the body or across the skin.

As used herein, the term "pharmaceutically acceptable salt" refers to any salt (e.g., obtained by reaction with an acid or a base) of a compound of the present disclosure that is physiologically tolerated in a subject (e.g., a mammal, and/or in vivo, ex-vivo, in vitro cells, tissues, or organs). "Salts" of the compound of the present disclosure may be derived from inorganic or organic acids and bases. Suitable anion salts include, arecoline, besylate, bicarbonate, bitartarate, butylbromide, citrate, camysylate, gluconate, glutamate, glycollylarsanilate, hexylresorcinate, hydrabamine, hydrobromide, hydrochloride, hydroxynapthanoate, isethionate, malate, mandelate, mesylate, methylbromide, methylbromide, methylnitrate, methylsulfate, mucate, napsylate, nitrate, pamaoate (Embonate), pantothenate, phosphate/diphosphate, polygalacuronate, salicylate, stearate, sulfate, tannate, teoclate, fatty acid anions, and triethiodide. Suitable cations salts include benzathine, clemizole, chloroprocaine, choline, diethylamine, diethanolamine, ethylenediamine, meglumine, piperazine, procaine, aluminum, barium, bismuth, lithium, magnesium, potassium, and zinc.

For the purposes of this application, the salts of the compounds of the present disclosure are considered to be pharmaceutically acceptable for therapeutic uses. However, salts of acids and bases that are non-pharmaceutically acceptable may also find use, for example, in the preparation or purification of a pharmaceutically acceptable compound.

As used herein, the term "pharmaceutical composition" means a combination of the active agent (e.g., an active pharmaceutical compound or ingredient, API) with a carrier, inert or active (e.g., a phospholipid, a triglyceride), making the compositions suitable for diagnostic or therapeutic uses *in vitro*, *in vivo*, or *ex vivo.* The active agents of the pharmaceutical compositions of the present disclosure are not combined with a lipid carrier that will form a heterogeneous liposomal structure.

As used herein "primary therapy" refers to a first line of treatment upon initial diagnosis of a hormone-dependent breast disorder or a hormone-dependent reproductive tract disorder or both in a subject. Exemplary primary therapies may involve surgery, a wide range of chemotherapies, and radiotherapy.

As used herein, a "route of administration" or "route of delivery" for the compound or compositions of present disclosure refers to the topical, transdermal, transpapillary, and intraductal pathway for delivering the compound or compositions of the present disclosure to a subject.

As used herein, the terms "subject," "patient," and "individual," may be used interchangeably herein and refer to a mammal such as a human. Mammals also include pet animals such as dogs, cats, laboratory animals, such as rats, mice, and farm animals such as cows and horses. Unless otherwise specified, a mammal may be of any gender or sex.

As used herein, the term "skin care active" means all compounds or substances now known or later demonstrated to provide benefit when applied to skin and all compounds now claimed or in the future claimed to provide benefit when applied to skin.

As used herein, the term "tamoxifen resistance" refers to two classes of resistance: (a) de novo resistance, i.e., non-responsiveness to tamoxifen therapy from the beginning of the treatment, and (b) acquired resistance, i.e., non-responsiveness to tamoxifen therapy after initial responsiveness or tamoxifen-dependent growth /stimulated growth while continuing to express estrogen receptors (Minsun Chang. Biomol. Ther. 20(3), 256-267 (2012). The acquired resistance to tamoxifen may develop as early as 3 m to 1 year to as late as 5 to 10 years. As used herein, the term "tamoxifen refractory" refers to subjects that have been dosed daily with tamoxifen for at least 2 days and have a steady state level of plasma endoxifen of less than 30 nM (e.g., less than 20 nM, less than 25 nM, or less than 30 nM).

As used herein the term "reference plasma endoxifen level" refers to a value of 30 nM.

As used herein, the terms "topical" or "topically," refer to application of the compositions of the present disclosure to the surface of the skin and tissues (e.g., alveolar, buccal, lingual, masticatory, and other tissues that line the hollow organs or body cavities, including mucosa. Topical formulations can be used for topical, transdermal, transpapillary, and intraductal administration. "Transdermal" refers to administration through the skin and may be delivered by any means suitable such as a patch, a tape, a bandage, aerosolized and non-aerosolized sprays, pumps with metered dosing, and other transdermal delivery systems, etc. As used herein, the term "transpapillary" refers to the application of the compositions of the present disclosure to the surface of the skin on the mammary papilla(e), wherein the compositions are delivered through the skin of the mammary papilla(e) to the underlying tissues, including into the breast milk ducts. As used herein, the term "intraductal" or "intraductally" refers to administration of a composition or a formulation disclosed herein directly into a breast milk duct of a subject. This may be done by any means that permits direct administration into the milk duct, such as using a cannula, syringe, catheters, microcatheters, probes, etc. The terms topical and transdermal are to be given the broadest possible usage for the purpose of the present disclosure.

As used herein, the term "unit dosage form" refers to physically discrete units suitable for unitary dosages for subjects, each unit containing a predetermined quantity of active material calculated to produce the desired therapeutic effect, in association with a suitable pharmaceutical excipient.

As used herein, a "unit dose" is a dose of any therapeutic or active agent administered in one dose/at one time/single route/single point of contact, i.e., one administration event. As used herein, "split dose" refers to (1) dosing regimens in which one or more active agents are administered to a patient at least twice daily; (2) once daily administration of a pharmaceutical composition containing one or more active agent in which a portion of the active agent is formulated for immediate release and a portion of the active agent is formulated for delayed or pulsatile release; and (3) once daily administration of a pharmaceutical composition containing an active agent formulated for controlled or sustained release.

It is specifically understood that any numerical value cited herein includes all values from the lower value to the upper value, i.e., all possible combination of numerical values between the lowest value and the highest value enumerated are to be considered to be expressly stated in this application. For example, if a concentration range or beneficial range is stated as 1% to 50%, it is intended that values such as 2% to 40%, 10% to 30%, or 1% to 3% etc., are expressly enumerated in this specification. As another example, a stated concentration of "20%" or "about 20%" is intended to include values from 19% to 21%. Yet another example, if a ratio of 1:10 to 10:1 is stated, then it is intended that ratios such as 1:9 to 9:1, from 1:8 to 8:1, from 1:7 to 7:1, from 1:6 to 6:1, from 1:5 to 5:1, from 1:4 to 4:1, from 1:3 to 3:1, from 1:2 to 2:1, from 1:1 to 2:1 or from 2:5 to 3:5 etc. are specifically intended. It is also to be understood that if a concentration or dose is stated as a specific value, such as 1 mg or 10 mg, it is intended to include 10% variation from the stated value. It is to be further understood that if the active agent is present as both (Z) and (E) isoforms, that the dose is based on the amount of (Z) isoform. There are only some examples of what is specifically intended. Unless specified otherwise, the values of the constituents or components of the compositions are expressed in weight percent of each ingredient in the component.

As used herein, the terms "hormone-dependent breast disorder," "hormone-dependent reproductive tract disorder," "hormone-dependent breast and reproductive tract disorder" each and collectively include, without limitation, any breast or reproductive tract (gynecologic) disorder that is related to or is sensitive to high estrogen or normal estrogen levels that need to be reduced, disorders with estrogen-receptor positive (ER+) and/or progesterone-receptor positive (PR+) disorders, for example, breast disorders, endometriosis, uterine fibroids (also called leiomyomas) etc. Reproductive tract disorders include endometrial, ovarian, cervical, uterus, vaginal, and vulvar, cancers. The terms "estrogen-related disorder" and "estrogen-receptor related disorder" may be used interchangeably to refer to the foregoing hormone dependent disorders. The disorders may be presented primarily or secondarily to an underlying disease, for example, prostate cancer or other disorders such as liver diseases. Hormone-dependent breast and reproductive tract disorder for example, include McCune-Albright syndrome which is a disorder caused by a mutation in the GNAS gene affecting bones, skin, and several hormone-producing (endocrine) tissues, often resulting in abnormal scar-like (fibrous) tissue in their bones, a condition called polyostotic fibrous dysplasia, hyperthyroidism in individuals carrying such mutations, and in girls often resulting in precocious puberty.

As used herein, "breast disorder" means any aberration or a constellation of aberrations in the breast. Such aberration may be proliferative, non-proliferative, benign or malignant. Breast disorders include benign lesions of the breast (e.g., hyperplasia), increased breast density, gynecomastia, mastalgia, breast cancer, McCune-Albright Syndrome and precious puberty. Benign breast lesions include, but are not limited to, hyperplasia, atypia, ductal hyperplasia, lobular hyperplasia, atypical ductal hyperplasia (ADH), and atypical lobular hyperplasia (ALH). While not cancerous, ADH and ALH may be indicative of a predisposition for breast cancer.

Breast density is a breast disorder identified by visual techniques such as mammography and reflects increased fibroglandular tissue within the breast, i.e., overgrowth of stromal and epithelial cells in the breast. Breast density is classified in 4 classes Class A, B, C and D based on the degree of severity of the density. It is an independent risk factor for breast cancer. At least 23 states in USA require physicians to inform subjects if they have dense breast(s). There is currently no treatment for dense breasts, although subjects are reminded to make healthy lifestyle choices and undergo regular mammograms to monitor changes in breast.

Gynecomastia is a common male breast condition reflecting increased hyperplasia of the breast tissue, including epithelial hyperplasia, with prevalence of asymptomatic gynecomastia is 60% to 90% in neonates, 50% to 60% in adolescents, and up to 70% in men aged 50 to 69 years (Therapeutics and Clinical Risk Management 2011:7, 145 - 148). Newborn gynecomastia usually resolves itself within 4 weeks of birth and at least half of adolescent males experience gynecomastia with typical onset of 13 to 14 years of age (Tanner stage 3 or 4). However, subjects showing symptoms of gynecomastia for over 12 to 18 months appear to have irreversible tissue and structural changes (Lemaine et al. Semin Plast Surg. 2013 Feb; 27(1): 56-61). Gynecomastia has been proposed to be a risk factor for male breast cancer.

Further, gynecomastia often presents itself secondarily to an underlying disorder such as prostate cancer, cirrhosis and liver disease, male hypogonadism, hyperthyroidism, renal failure and in patients undergoing hemodialysis, type I diabetes mellitis, etc. Further, medications, such as anti-androgen medications or certain hypertensives, anti-psychotics, etc., themselves have been reported to cause up to 25% of cases of gynecomastia and can be categorized by their hormone-like action. For e.g., the most common side effects attributed to bicalutamide, a nonsteroidal antiandrogen used for treatment of prostate cancer, are gynecomastia and breast pain.

As used herein, "breast cancer" means any malignant tumor of breast cells. Breast cancer may be at any stage of breast cancer, including stages of a pre-cancer, an early stage cancer, a non-metastatic cancer, a pre-metastatic cancer, a locally advanced cancer, and a metastatic cancer. There are several types of breast cancer. Exemplary breast cancers include, but are not limited to, ductal carcinoma in situ (DCIS), lobular carcinoma in situ (LCIS), invasive (or infiltrating) lobular carcinoma (ILC), invasive (or infiltrating) ductal carcinoma (IDC), microinvasive breast carcinoma (MIC), inflammatory breast cancer, ER-positive (ER+) breast cancer, ER-negative (ER-) breast cancer, HER2+ breast cancer, triple negative breast cancer (TNBC), adenoid cystic (adenocystic) carcinoma, low-grade adenosquamatous carcinoma, medullary carcinoma, mucinous (or colloid) carcinoma, papillary carcinoma, tubular carcinoma, metaplastic carcinoma, or micropapillary carcinoma. A single breast cancer tumor can be a combination of these types or be a mixture of invasive and in situ cancer.

DCIS is the most common non-invasive breast cancer. It involves the cell lining the breast ducts. In DCIS, the cells have not spread beyond the walls of the duct into the surrounding breast tissue. About 1 in 5 new breast cancer cases will be DCIS. LCIS is a precancerous neoplasia. It may be indicative of a predisposition for invasive cancer. LCIS only accounts for about 15% of the in situ (ductal or lobular) breast cancers.

IDC is the most invasive breast cancer. As the name applies, it is a carcinoma that begins in the breast ducts and then invades the surrounding fatty tissue. About 8 to 10 invasive breast cancers are infiltrating ductal carcinomas. IDC is often treated by surgery to excise the cancerous tissue, and radiation therapy. In addition, chemotherapy combined with immunotherapy (e.g., tamoxifen and tratuzumab) is often used to treat IDC. If the tumor is larger than 4 cm, then a radical mastectomy may be performed.

ILC is a cancer that develops in the lobules of the breast and has invaded the surrounding tissue. About 1 in 10 invasive breast cancer is an ILC. ILC is treated by surgery to excise the cancerous tissue, and radiation therapy. In addition, chemotherapy and immunotherapy combination (e.g., tamoxifen and tratuzumab) is often used as an adjuvant therapy to treat ILC.

Inflammatory breast cancer accounts for about 1% to 3% of all breast cancers. In inflammatory breast cancer, cancer cells block lymph vessels in the skin resulting in the beast turning red and feeling warm. The affected breast may become larger or firmer, tender, or itchy. Inflammatory breast cancer is treated with chemotherapy, immunotherapy, radiation therapy and in some cases, surgery.

Estrogen Receptor positive (ER+) breast cancer is characterized by the presence of estrogen receptors on the surface of the cancerous cells. Growth of ER+ cancer cells is associated with the availability of estrogen (hormone-dependent or hormone sensitive breast cancer). Approximately, 80% of all breast cancers are ER+ breast cancers. Treatment options for ER+ breast cancer include chemotherapeutic agents that block estrogen (e.g., tamoxifen).

The compositions of the present disclosure are formulated to be administered to a subject in need thereof topically. The topical compositions of the present disclosure are capable of being administered topically, transdermally, transpapillarily, and intraductally. In certain embodiments, the compositions disclosed herein are administered topically. In some embodiments, the compositions disclosed herein are administered to a breast duct via transpapillary or intraductal routes or modes of administration. Accordingly, the compositions comprising endoxifen are formulated to be compatible with its intended route of administration.

The topical formulations disclosed herein are designed to provide maximum delivery and uptake locally in the affected tissues with rate of release as needed (for example, controlled release, slow, rapid, delayed, pulsatile, or sustained release) throughout the region to be treated with little to no increase in the systemic blood levels of the therapeutic agents. Topical formulations disclosed herein also may be depot formulations allowing sustained and gradual release of an active agent. Such formulations can avoid hepatic metabolism. Topical administration includes percutaneous absorption of the topical compositions through the skin. The term topical application denotes any mode of delivery of a composition from the surface of a subject's skin, through stratum corneum, epidermis, and dermis layers, and into microcirculation. This is typically achieved by diffusion down a concentration gradient through and between cells, through the hair follicles, sweat and sebaceous glands, or any combination thereof.

The compositions may be administered to a subject by any means suitable. Accordingly, the compositions may be applied to any location to the surface of the skin for example to breast skin, manually without or with applicators such as droppers, pipettes, swabs, brushes, cloths, pads, sponges, or with any other applicator or device known in the art or using transdermal delivery systems such as patch, tape, dressing, sprays such as aerosolized sprays or non-aerosolized sprays, devices using pressurized containers or non-pressurized containers, and the like. The delivery may in metered doses or unmetered doses. In some embodiments, a preparation is applied directly over the spot of suspected hormone-dependent disorder. In certain embodiments, the composition may be applied directly on the nipple or mammary papilla(e). In other embodiments, the composition is applied directly on the mammary papilla and forced into a breast duct under pressure. In other embodiments, any of iontophoretic current, heat, microneedles or high voltage bursts of electricity (electroporation) may be applied after topical application of the compositions disclosed herein to further aid the penetration of the active compounds.

The present disclosure provides that compositions comprising a chemical compound to enhance penetration or permeation of the active agents such as endoxifen free base and salts thereof into or through the skin, i.e., "molecular penetration enhancer," "MPE^{™}", "chemical penetration enhancer," "penetration enhancer" or "permeation enhancer." These terms are used interchangeably throughout this disclosure. A penetration enhancer may be a pure or a single compound or may comprise a mixture of different chemical entities. As used herein, multiplexed molecular penetration enhancers (MMPE^{™}) refer to two or more substances the compositions disclosed herein comprise a combination of penetration enhancers wherein each of the substances is also a penetration enhancer. The topical compositions according to the invention comprise a first compound; and a second compound; wherein the first compound comprises: i. diethyleneglycol monoethyl ether, and the second compound comprises a penetration enhancer selected from the group consisting of DMSO, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof; or ii. DMSO and the second compound comprises a penetration enhancer selected from the group consisting of diethyleneglycol monoethyl ether, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof.

In some embodiments, the penetration enhancer can be diethyl sebacate, DMSO, diisopropyl adipate, dipropylene glycol, polyethylene glycols such as PEG300, PEG 400, diethylene glycol monoethyl ether (Transcutol^{®}), capric triglyceride, caprylic triglyceride, caprylic/capric triglyceride (tradename: Crodamol GTCC), isopropanol, mineral oil, cetyl alcohol, stearic acid, or a combination thereof. Such penetration enhancer can also serve as solvents and co-solvents for active agents.

In another aspect, the topical composition prepared with multiplexed penetration enhancers also serve to stabilize the topical compositions of the present disclosure. The applicants have surprisingly and unexpectedly discovered that certain combinations of compounds are excellent penetration enhancers, and as such can be incorporated into topical formulations to facilitate one or more active agents. The increased penetration enhancement can lead to a decrease in the total concentration of skin irritants in a formulation and provide stability to the compositions comprising at least one active agent. The active agents of the pharmaceutical compositions of the present disclosure are not combined with a lipid carrier that will form a heterogeneous liposomal structure.

In some embodiments, the present disclosure discloses that the first compound comprises diethyleneglycol monoethyl ether and the second compound comprises a penetration enhancer selected from the group consisting of DMSO, diethyl sebacate, diisopropyl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof.

In other embodiments, the present disclosure discloses that the first compound comprises DMSO and the second compound comprises a penetration enhancer selected from the group consisting of diethyleneglycol monoethyl ether, diethyl sebacate, diisopropyl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof.

The present disclosure discloses the following certain embodiments:
the first compound comprises diethyleneglycol monoethyl ether and the second compound comprises diethyl sebacate;
the first compound comprises diethyleneglycol monoethyl ether and the second compound comprises diisopropryl adipate;
the first compound comprises diethyleneglycol monoethyl ether and the second compound comprises dipropylene glycol;
the first compound comprises diethyleneglycol monoethyl ether and the second compound comprises polyethylene glycols such as PEG 300, PEG 400 and the like;
the first compound comprises diethyleneglycol monoethyl ether and the second compound comprises isopropanol;
the first compound comprises diethyleneglycol monoethyl ether and the second compound comprises t-butanol;
the first compound comprises diethyleneglycol monoethyl ether and the second compound comprises polyethylene glycol dodecyl ether (Brij L4);
the first compound comprises diethyleneglycol monoethyl ether and the second compound comprises cetyl alcohol;
the first compound comprises diethyleneglycol monoethyl ether and the second compound comprises mineral oil;
the first compound comprises diethyleneglycol monoethyl ether and the second compound comprises caprylic triglyceride;
the first compound comprises diethyleneglycol monoethyl ether and the second compound comprises capric triglyceride;
the first compound comprises diethyleneglycol monoethyl ether and the second compound comprises caprylic/capric triglycerides such as Crodamol GTCC;
the first compound comprises diethyleneglycol monoethyl ether and the second compound comprises stearic acid;
the first compound comprises DMSO and the second compound comprises diethyleneglycol monoethyl ether;
the first compound comprises DMSO and the second compound comprises diethyl sebacate;
the first compound comprises DMSO and the second compound comprises diisopropryl adipate;
the first compound comprises DMSO and the second compound comprises dipropylene glycol;
the first compound comprises DMSO and the second compound comprises polyethylene glycols;
the first compound comprises DMSO and the second compound comprises isopropanol;
the first compound comprises DMSO and the second compound comprises t-butanol;
the first compound comprises DMSO and the second compound comprises polyethylene glycol dodecyl ether;
the first compound comprises DMSO and the second compound comprises cetyl alcohol;
the first compound comprises DMSO and the second compound comprises mineral oil;
the first compound comprises DMSO and the second compound comprises caprylic triglyceride;
the first compound comprises DMSO and the second compound comprises capric triglyceride;
the first compound comprises DMSO and the second compound comprises caprylic/capric triglycerides (tradename: Crodamol GTCC);
the first compound comprises DMSO and the second compound comprises stearic acid;
the ratio of the first compound to second compound ranges from 1:9 to 9:1, from 1:4 to 4:1, 1:3 to 3:1, from 1:2 to 2:1, and 1:1;
the ratio of the first compound to second compound ranges from 1:9 to 9:1;
the ratio of the first compound to second compound ranges from 1:4 to 4:1;
the ratio of the first compound to second compound ranges from 1:2 to 1:2; and
the ratio of the first compound to second compound is about 1:1.

In another aspect, the present disclosure provides that the total concentration of the first compound and the second compound is up to 90%, up to 85%, up to 80%, up to 75%, up to 70%, up to 65%, up to 60%, up to 55%, up to 50%, up to 45%, up to 40%, up to 35%, up to 30%, up to 25%, up to 20%, up to 15%, up to 10%, or up to 5% w/w volume of the composition.

In some embodiments, the total concentration of the first compound and the second compound ranges from 10% to 90% w/w of the topical composition.

In some embodiments, the total concentration of the first compound ranges from 10% to 90% (w/w) of the topical composition; and the total concentration of the second compound ranges from 5% to 80% (w/w) of the topical composition.

Further, desirable amounts of penetration enhancers according to claim 1 (Samples 1 to 3, Samples 5 to 11 and Samples 13 to 15) shown in **Table 1** may be combined or multiplexed to serve as MMPEs as shown in **Table 4.** The topical formulations of the present disclosure may also be formulated to include other chemical penetration enhancers which have significant ability to enhance transport of actives. Such substances may have the character of surfactants, azone-like compounds, solvents, alcohols, fatty acids, fatty esters, aliphatic thiols and the like. Examples of chemical penetration enhancers are reported in the paper of Santus et al. (Santus, C. G. and Baker, R. W., Transdermal enhancer patent literature. Journal of Controlled Release 1993.25:1-20.)

In another aspect, the present disclosure provides that the topical compositions further comprise one or more cosmetically or pharmaceutically acceptable carriers/excipient. Excipients/carriers are known in the art and can include but are not limited to skin care actives, solvents, co-solvents, penetration enhancers, thickening agents, emollients (for example, moisturizers and humectants), controlled-release agents, surfactants, solubilizers such as C2 to C8 straight and branched chain alcohols, diols and triols and emulsifiers such as cetyl alcohol, stearyl alcohol, and the like.

The topical compositions further comprise a thickening agent to increase the viscosity of the composition or formulation or to function as a solubilizing agent. The thickening agents suitable for the purpose of the present disclosure include polyacrylamides, cellulose derivatives, ethyl cellulose, hydroxypropyl cellulose (HPC), hydroxyethyl cellulose, hydroxypropyl methyl cellulose (HPMC), and carboxymethyl cellulose (CMC), polyacrylic acid, carboxypolymethylene, carboxyvinyl polymers (carbomers), poloxamers, poloxamines, polyvinylpyrrolidone, polyvinyl alcohol, chitosan, dextran, pectins, natural gums, modified clays, polycarbophils, acrylic copolymers such as acrylate/alkyl acrylate copolymers, bentones, fatty acid metal salts such as aluminum stearates and hydrophobic silica, ethylcellulose, polyethylene and a combination thereof. Thickening agents such as polyacrylic acid and its derivatives are available commercially as Carbopol^{®}, and its derivatives such as Carbopol^{®}. Ultrez 10, Carbopol^{®}. 940, Carbopol^{®}. 941, Carbopol^{®}. 954, Carbopol^{®}. 980, Carbopol^{®}. 981, Carbopol^{®}. ETD 2001, Carbopol^{®}. EZ-2 and Carbopol^{®}. EZ3 from B.F. Goodrich specialty Polymers and Chemicals Div. of Cleveland, Ohio. Additional thickening agents, enhancers, adjuvants may generally be found in Remington's The Science and Practice of Pharmacy, Meade Publishing Co., United States Pharmacopeia/National Formulary. In some embodiments, the compositions comprising endoxifen free base and salts thereof further comprise 0.001% to 10% (w/w) of a thickening agent.

In an aspect, the present disclosure provides topical compositions further comprising at least one surfactant. Suitable examples of surfactant include the commercially available surfactants such as polysorbate-20 (low peroxide), polysorbate-40 (low peroxide), polysorbate-60 (low peroxide), polysorbate-80 (low peroxide), polysorbate-85 (low peroxide), phospholipids, e,g., plural oleique, TX-100, AOT-Tween 80, AOT-DOLPA, AOT-OPE4, CTAB-TRPO, CTAB (cetyltrimethylammonium bromide), surfactants such as sodium laureth sulfate, sorbitan monolaurate, lecithin, polyethylene glycol alkyl ethers such as diethylene glycol monoethyl ethers for example, polyethylene glycol dodecyl ether (Brij L4), Brij S20, and Brij Oso, and combinations thereof. Additional surfactants that may be used are supplied under the tradenames of Kolliphore EL (Sigma-Aldrich), a polyethoxylated castor oil formerly known as Cremaphor EL or Etocas, and include without limitation, Cremaphor EL and RH 40, and Etocas 35 and 40, Cremaphor RH140 and Etocas 40. In some emboiments, the surfactants may be present in a composition ranging from 0.01% to 15% w/w of the composition.

Additional penetration enhancers can be ethanol, propanol, cetostearyl alcohol, PEG 3395, PEG 4550, propylene glycol, oleic acid, methyl oleate, glycolic ethers, terpenes (such as eugenol and farnesol), amides (such as urea, dimethyl acetamide (DMA), dimethyl formamide (DMF), 2-pyrolidone, 1-methyl-2-pyrolidone, ethanolamine, diethanolamine and triethanolamine, menthol, pyrrolidones such as N-methylpyrrolidone), azones (such as 1-dodecylazepan-2-one, 2-nonyl-1,3-dioxolane (SEPA 009), Span20, and dimethylaminopropanoate (DDAIP)) and the like.

Additional solvents and co-solvents can be polar organic solvents such as a sulfoxide (such as decylmethyl sulfoxide), a glycolic ether (such as ethylene glycol monomethyl ether, diethylene glycol monomethyl ether, ethylene glycol monoethyl ether and the like), a fatty acid ester (such as di(lower)alkyl esters of C6-C22 fatty acids, isopropyl palmitate, methylpropiopnate, butyl stearate, methyl laurate, and ethyl oleate), or a fatty alcohol (such as oleyl alcohol and lauryl alcohol), or a combination thereof.

Skin care actives may provide benefits, or claimed benefits, in areas such as one or more of wrinkle removal or wrinkle reduction, firming of skin, exfoliation of skin, skin lightening, treatment of dandruff, treatment of acne, skin conditioning, development of tans and artificial tans, improvement of skin moisture content, improvement of skin barrier properties, control of sweat, anti-aging, reduction or avoidance of irritation and reduction or avoidance of inflammation. Examples of skin care actives include molecules such as peptides, proteins, oligonucleotides, fullerenes as well as small molecules. Skin care actives may be protease and/or enzyme inhibitors, anti-coenzymes, chelating agents, antibodies, antimicrobials, emollients (moisturizers and humectants), vitamins, skin protectants, antioxidants and/or skin soothing agents, plant extracts and the like. Examples of skin care actives include but are not limited to vitamin C, vitamin E (alpha tocopherol), retinoids, soy derivatives (e.g. isoflavones), green tea polyphenols, alpha hydroxy acids (e.g. glycolic and lactic acids), beta hydroxy acids (e.g. salicylic acid), poly hydroxy acids, alpha lipoic acid, hemp oil (glycerides), niacinamide, dimethyl amino ethanol, coenzyme Q10, kinetin (plant growth hormone), dimethyl sulfone and botulinum toxin. Other examples of skin care actives may be found in The Perricone Prescription by Nicholas Perricone, Harper Collins Publishers Inc., New York, 2002.

Additional examples of emollients include, without limitation, moisturizers and humectants such as glycerine, amino acids and amino acid derivatives, polyaminoacids and derivatives, pyrrolidone carboxylic acids and their salts and derivatives, mineral oil, petroleum, polydene, isohexadecane, fatty acids such as perlagonic, lauric, stearic, isostearic, hydroxystearic, oleic, linoleic, and ricinoleic, coca butter, safflower oil, olive oil, sunflower oil, cod liver oil, avocado oil, palm oil, sesame oil, soybean oil, silicone oil, polyethylene glycol, squalene, dimethicones, Q7-9120 (Dow Corning), cyclomethicones, and the like. In some embodiments, compositions comprise emollients that are liquid at room temperature.

Additional examples of antioxidants include alpha-tocopherol (vitamin E), butylhydroxyanisoles (BHA), butylhydroxytoluene (BHT), ascorbic acid, and pharmaceutically acceptable salts and esters thereof, propyl gallate, citric acid, malic acid, and pharmaceutically acceptable salts thereof, sulfite salts and mixtures thereof.

In another aspect, the topical compositions disclosed herein may also comprise an antimicrobial agent. The action of the antimicrobial agent can be bacteriostatic wherein the antibiotic arrests the proliferation of, but does not necessarily kill, the microorganism or the activity of the antibiotic can be bactericidal and kill the organism or a combination of the activities. Antibiotics suitable for use include beta-lactams (penicillin and cephalosporin), vancomycins, bacitracins, macrolides (erythromycins), lincosamides (clindomycin), chloramphenicols, tetracyclines, aminoglycosides (gentamycins), amphoterecins, cefazolins, clindamycins, mupirocins, sulfonamides, and trimethoprim, rifampicins, metronidazoles, quinolones, novobiocins, polymixins, and gramicidins and like and any salts or variants thereof. Antiseptic agents may also be used and include ethyl para-hydroxybenzoate, propyl para-hydroxybenzoate, and butyl para-hydroxybenzoate.

In one aspect, the present disclosure provides that the topical compositions disclosed herein may comprise a controlled release agent. Examples of controlled release agent suitable for use include, without limitation, polyethoxylated castor oil derivatives, hydrogenated oils, glyceryl mono-, di-, tribenates, glyceryl monostearate, glyceryl distearate, long chain alcohols, such as stearyl alcohol, cetyl alcohol, and polyethylene glycol; waxes, including synthetic waxes, microcrystalline waxes, paraffin wax, carnauba wax, and beeswax; and mixtures thereof. In some embodiments, the controlled release reagent is one or more long chain alcohol. In at least one embodiment, the controlled release reagent is cetyl alcohol. Accordingly, the topical compositions may be convention/immediate release, delayed release, sustained or extended release, or pulsatile release formulations.

Other examples of suitable excipients are listed in Remington's Pharmaceutical Sciences, 18th Edition, Ed. Alfonso Gennaro, Mack Publishing Co. Easton, Pa., 1995 and Handbook of Pharmaceutical Excipients, 3rd Edition, Ed. Arthur H. Kibbe, American Pharmaceutical Association, Washington, D.C. 2000.

### Active Agents

The topical compositions/formulations disclosed herein are particularly suitable for formulation of topical compositions comprising hydrophobic therapeutically active agents, such as SERMs (such as tamoxifen, afimoxifene, raloxifene, endoxifen, toremifene, N-desmethyl-tamoxifen, clomefine, ormeloxifene, lasofoxifene, and ospemifene), SERDs (such as fulvestrant), aromatase inhibitors (such as letrozole, anastrozole, and exemestane), and salts and solvates thereof. According to the invention, the at least one active agent is selected from the group consisting of endoxifen and salts and solvates thereof.

In some embodiments, the at least one active agent ranges from 0.01% to 20% (w/w) of the composition. In other embodiments, the at least one active agent ranges from 0.1% to 10% (w/w) of the composition.

### Endoxifen

The present disclosure relates to topical compositions wherein the at least one active agent is endoxifen free base or a salt or a solvate thereof. In an aspect, the present disclosure relates to compositions comprising endoxifen and salts thereof comprising 0.01% to 20% endoxifen free base and salts thereof weight by weight (w/w) or weight by volume (w/v) of the final composition. In some embodiments, composition comprising endoxifen free base and salts thereof comprise 0.01%, 0.1%, 0.2%, 0.3%, 0.4%, 0.5%, 1%, 2%, 3%, 4%, 5%, 6%, 7%, 8%, 9%, 10%, 11%, 12%, 13%, 14%, 15%, 16%, 17%, 18%, 19%, and 20% of endoxifen free base and salts thereof (w/w) or (w/v) of the final composition.

Endoxifen comprised in compositions of the present disclosure may be (Z)-endoxifen, (E)-endoxifen or a combination thereof, or a salt or a solvate thereof.

### Endoxifen Free Base

In one aspect, the present disclosure relates to topical compositions comprising (Z)-endoxifen free base.

In certain embodiments, the topical compositions comprise at least 0.1%, at least 0.2%, at least 0.3%, at least 0.4%, at least 0.5%, at least 1%, at least 5%, at least 10%, at least 20%, at least 25%, at least 30%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, at least 99.99% and 100% of (Z)-endoxifen free base (w/w) of total endoxifen in the final composition. In at least one embodiment, compositions comprising endoxifen comprise at least 40% of (Z)-endoxifen free base (w/w) of total endoxifen in the final composition.

In certain embodiments, compositions comprising endoxifen comprise at least 60%; at least 66%; at least 67%; at least 68%; at least 69%; at least 70%; at least 71%; at least 72%; at least 73%; at least 74%; and at least 75% of (Z)-endoxifen free base (w/w) of total endoxifen in the final composition. In some embodiments, the topical compositions comprise endoxifen predominantly (defined as at least 60%) as (Z)-endoxifen free base.

In an aspect, the present disclosure provides that the topical compositions comprise 0.01% to 10% (Z)-endoxifen w/w or w/v of the final composition. In various other embodiments, the compositions comprise 0.01%, 0.02%, 0.03%, 0.04%, 0.05%, 0.06%, 0.07%, 0.08%, 0,09%, 0.1%, 0.2%, 0.3%, 0.4%, 0.5%, 0.6%, 0.7%, 0.8%, 0.9%, 1%, 2%, 3%, 4%, 5%, and 10% of (Z)-endoxifen free base w/w or w/v of the final composition.

In some embodiments, the endoxifen or a salt or a solvate thereof in a topical composition comprises (Z)-endoxifen and (E)-endoxifen at a Z:E ratio ranging from 99:1 to 1:99, from 90:10 to 10:90, from 85:15 to 15:85, from 80:20 to 20:80, from 75:25 to 25:75, from 70:30 to 30:70, from 65:35 to 35:65, from 60:40 to 40:60, from 55:45 to 45:55 and about 50:50. In other embodiments, the endoxifen in the composition is comprised at a ratio of (Z)-endoxifen to (E)-endoxifen (Z:E ratio) of 10:90, 15:85, 20:80, 25:75, 30:70, 40:60, 45:55; 50:50, 55:45, 60:40, 70:30, 80:20, 90:10, 95:5, 96:4, 97:3, 98:2, 99:1, 99.5:0.5, 99.9:0.1 wt/wt% in the composition. In other embodiments, the topical compositions comprise a mixture of (Z)-endoxifen and (E)-endoxifen (E/Z-endoxifen mixture or E/Z-mix) in equilibrium. In certain embodiments, the (Z)-endoxifen dissolved in a solvent reaches an equilibrium with (E)-endoxifen at E:Z ratios (wt/wt%) ranging from of 30:70 to 70:30, form 35:65 to 65:35, from 40:60 to 60:40, from 45:55 to 55:45, and about 1:1 (*See* **Tables 5** & **6).**

In certain embodiments, the at least one active agent, endoxifen, in the topical compositions is at a ratio of (Z)-endoxifen to (E)-endoxifen (Z/E ratio) ranging from 0.3 to 2, from 0.5 to 1.8, from 0.6 to 1.5, from 0.8 to 1.25, from 0.9 to 1.1, and from 1.1 to 1.9 (*See* **Tables 5 & 6).**

The applicants have surprisingly and unexpectedly also discovered that the combination of the compounds (penetration enhancers) disclosed herein are useful for the preparation of topical compositions comprising (Z)-endoxifen and salts and solvates thereof that show reduced interconversion from the active (Z)-endoxifen form to the inactive and deleterious (E)-endoxifen in solutions or liquid formulations. Accordingly, in some embodiments, the topical compositions comprising predominantly (Z)-endoxifen and salts and solvates thereof are stable for at least 6 months, at least 9 months, at least 12 months, and at least 18 months at ambient temperature.

### Endoxifen Salts

In some embodiments, the present disclosure provides compositions comprising pharmaceutically acceptable salts of endoxifen. Endoxifen salts known in the art are hydrochloride (Fauq et al., Bioorg Med Chem Lett. 2010 May 15;20(10):3036-3038) and citrate salts of endoxifen (U.S. Publication No. 2010/0112041).

In an aspect, the present disclosure provides compositions comprising various salts of endoxifen. In certain embodiments, the anion salts of endoxifen are selected from the group consisting of acetate, adipate, alginate, aspartate, arecoline, benzoate, benzenesulfonate, bisulfate, butyrate, besylate, bicarbonate, bitartarate, bromide, butylbromide, camysylate, chloride, citrate, camphorate, camphorsulfonate, cyclopentanepropionate, dodecylsulfate, ethanesulfonate, fumarate, flucoheptanoate, glycerophosphate, gluconate, glutamate, glycollylarsanilate, 2-hydroxyethanesulfonate, hemisulfate, heptanoate, hexanoate, hexylresorcinate, hydrabamine, hydrobromide, hydroxynapthanoate, iodide, isethionate, lactate, malate, maleate, mandelate, methanesulfonate, mesylate, methylbromide, methylbromide, methylnitrate, methylsulfate, mucate, napsylate, 2-naphthalenesulfonate, nicotinate, nitrate, oxalate, palmoate, pamaoate (Embonate), pantothenate, pectinate, persulfate, phenylpropionate, picrate, pivalate, propionate, phosphate/diphosphate, polygalacuronate, salicylate, stearate, sulfate, succinate, tartrate, thiocyanate, tosylate, tannate, teoclate, triethiodide, undecanoate and the like. In other embodiments, the cation salts of endoxifen are selected from the group consisting of benzathine, clemizole, chloroprocaine, choline, diethylamine, diethanolamine, ethylenediamine, meglumine, piperazine, procaine, aluminum, barium, bismuth, lithium, magnesium, potassium, and zinc.

In some embodiments, the salt is gluconate or a chemical equivalent thereof. Accordingly, in at least one embodiment the endoxifen comprised in a composition of the present disclosure is an endoxifen gluconate or a chemical equivalent thereof. Unless specified otherwise, it will be understood that when referring to endoxifen gluconate herein, its chemical equivalents are also embraced.

For the purposes of the present disclosure, chemical equivalents of endoxifen gluconate include all anionic, cationic, and non-ionic reaction complexes between the endoxifen molecule and the gluconate moiety. Such complexes typically react with the hydroxyl group of the endoxifen molecule. The gluconate moiety includes D-gluconic acid, gluconic acid, glycogenic acid, glycan-Δ-lactone, and the like. In some embodiments, the gluconate moiety is a pharmaceutically acceptable gluconate salt. Such salts include calcium gluconate, sodium gluconate, and salts of alkali metals and alkaline earth metals such as potassium gluconate, magnesium gluconate, lithium gluconate, and the like.

Both stereoisomers of gluconate, the D-form and the L-form, are encompassed in the present disclosure. In some embodiments, endoxifen gluconate comprised in the compositions of the present disclosure is selected from the group consisting of (Z)-endoxifen D-gluconate, (Z)-endoxifen L-gluconate, (E)-endoxifen D-gluconate, (E)-endoxifen L-gluconate and a combination thereof. In some embodiments, the pharmaceutical composition comprises (Z)-endoxifen L-gluconate. In certain embodiments, the pharmaceutical composition comprises (Z)-endoxifen D-gluconate. The compositions may be present as racemic mixtures, pure stereoisomers (e.g. enantiomers and diastereoisomers), stereo-enriched mixtures and the like unless otherwise indicated. When a particular stereoisomer is shown or named herein, it will be understood by those skilled in the art that minor amounts of other stereoisomers may be present in the compositions of the present disclosure unless otherwise indicated, provided that the utility of the composition as a whole is not eliminated by the presence of such other isomers. Individual isomers may be obtained by numerous methods that are well known in the art, including chiral chromatography using a suitable chiral stationary phase or support or by chemically converting them into diastereoisomers, separating the diastereoisomers by conventional means such as chromatography or recrystallization, then regenerating the original stereoisomer.

In other embodiments, the topical compositions comprise endoxifen predominantly as (Z)-endoxifen salts and solvates.

Provided herein in certain embodiments are compositions comprising 1%, 5%, 10%, 20%, 25%, 30%, 40%, 50%, 60%, 70%, 80%, 90%, 95%, 96%, 97%, 98%, 99%, 99.5%, 99.99% or 100% of endoxifen gluconate selected from the group consisting of (Z)-endoxifen D-gluconate, (E)-endoxifen D-gluconate, (Z)-endoxifen L-gluconate, (E)-endoxifen L-gluconate or a combination thereof w/w of total endoxifen gluconate salt in the final composition.

In some embodiments, topical compositions comprise 10% to 100% of (Z)-endoxifen D-gluconate w/w or w/v of total endoxifen gluconate in the final composition. In other embodiments, topical compositions comprise 10% to 100% of (Z)-endoxifen L-gluconate w/w or w/v of total endoxifen gluconate in the final composition.

In other embodiments, a composition comprising endoxifen gluconate is comprised of 10%, 20%, 30%, 40%, 50%, 60%, 65%, 70%, 75%, 80%, 85%, 90%, 91%, 92%, 93%, 94%, 95%, 96%, 97%, 98%, 99%, 99.5%, 99.75%, 99.99% and 100% of (Z)-endoxifen D-gluconate, (Z)-endoxifen L-gluconate or a combination thereof w/w of the endoxifen gluconate in the final composition.

Provided herein in some embodiments are compositions comprising (Z)-endoxifen D-gluconate and (E)-endoxifen D-gluconate. (Z)-endoxifen D-gluconate and (E)-endoxifen D-gluconate may be present in the compositions at ratios ranging from 1:99 to 99:1 w/w or v/v respectively. Provided herein in some embodiments are compositions comprising (Z)-endoxifen L-gluconate and (E)-endoxifen L-gluconate. In certain embodiments, the ratio of (Z)-endoxifen L-gluconate to (E)-endoxifen L-gluconate (w/w or v/v) is 99:1 to 1:99 w/w or v/ respectively

One of skill in the art will recognize that other combinations of endoxifen gluconate isomers are embraced by the present disclosure.

Unless specifically referred to by the prefix (Z), (E) or (Z/E), endoxifen used generally without a prefix is used herein to include to any or all endoxifen isoforms.

### Synthesis of (Z)-endoxifen Free Base and Salts Thereof

Several methods for the preparation of synthetic endoxifen are known in the art. For e.g., patents and patent applications describe methods of synthetic preparations of endoxifen and their prodrugs and salts in U.S. Patent No. 9333190B2 (Ahmad, Jina Pharmaceuticals); WO 2008/070463 (Ahmad, Jina Pharmaceuticals), U.S. Application No. 2010/0112041 (Ahmad, Jina Pharmaceuticals), WO 2012/050263 (Ahmad, Jina Pharmaceuticals), WO 2014/141292 (Desai, Intas Pharmaceuticals), WO2017/070651 (USA/Alchem Lab. Corp.); WO2009/120999A2 (Kushner), U.S. Patent No. 8,063,249 (Kushner, Olema Pharmaceuticals), U.S. Patent Nos. 7,531,578 and 8,119,695 (Forman and Yu), and WO2012/050263 (Song, CJ Cheiljedang Corp).

Methods of synthetic preparation of endoxifen have also been published in research literature. (Gauthier et al., J. Org. Chem, 61, 3890 - 3893 (1996), Fauq et al., Bioorg Med Chem Lett. 2010 May 15;20(10):3036-3038); Stearns et al., J. Natl. Cancer Inst. Vol 95, No. 23, 2003; Johnson et al., Breast Cancer Research and Treatment. 85:151-159, 2004; Ogawa, et al. Chem. Pharm. Bull. 39, 911 - 916, 1991). However, there remains an unmet need for large scale industrial scalable manufacturing. Stable (Z)-endoxifen free base may be prepared accordance to **Schema 1** and as further described below and in **Example 1.**

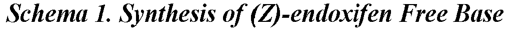

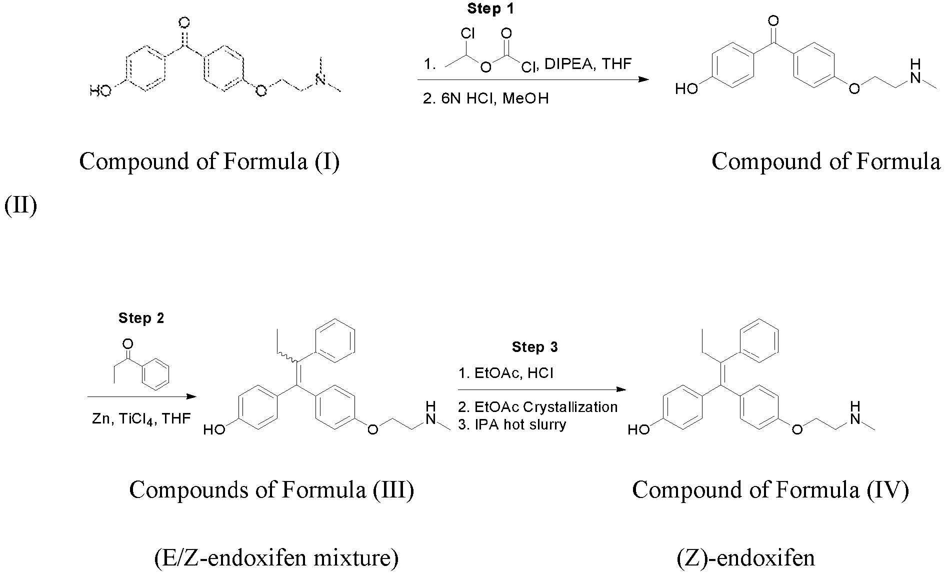

***Step 1. N-demethylation.*** The starting material [4-[2-(dimethylamino)ethoxy] phenyl](4-hydroxyphenyl)methanone, Compound of Formula (I), is n-demethylated by reacting the Compound of Formula (I) in a solvent such as tetrohydrofuran (THF) and a demethylating agent such as 1-chloroethyl-chloroformate in the presence of a proton acceptor such as N'N-diisopropyleamine (DIPEA) followed by extraction with methanol and 6N HCl. The reaction mixture is neutralized with NaOH and filtered. The resulting wet cake is washed with water and EtOAc to obtain the intermediate -product (4-Hydroxyphenyl)(4-(2-(methylamino)ethoxy) phenyl)methanone, **Compound of Formula (II).**

***Step 2. McMurry Reaction.*** The intermediate compound of Formula (II) 1011M01 is subjected to McMurry reaction by reacting compound of Formula (II) and propiophenone with Zn, in solvent THF and reducing agent such as TiCl4 followed by extraction with ethyl acetate (EtOAc) and n-Heptane to afford a mixture of (E)-endoxifen and (Z)-endoxifen free bases (E/Z-endoxifen), Compounds of Formula (III). The Compounds of Formula (III) may be further purified by crystallization or by column chromatography for use in pharmaceutical preparation as E/Z-endoxifen mixture.

The E/Z mixture of Step 2 may be subjected to further purification of (Z)-endoxifen free base as descried below in Step 3.

***Step 3. Purification of (Z)-endoxifen free base.*** The (E))/(Z)-endoxifen mixture, Compounds of Formula (III) is subjected to fractional crystallization with EtOAc followed by heating in isopropanol (IPA). The mixture is cooled and washed with isopropanol resulting in a wet cake which is dried to afford enriched white to off-white powder of (Z)-4-(1-(4-(2-(methylamino)ethoxy)phenyl)-2-phenylbut-1-enyl)phenol, **Z-Endoxifen free base, Compound of Formula (IV).** (Molecular weight 373.49; Molecular formula: C25H27NO2; Melting point 139°C - 143°C). Use of different solvents for fractional crystallization and recrystallization of the resulting solids is advantageous in purification of (Z)-endoxifen.

Additional methods of purification have been described in the art (Fauq et al., Bioorg Med Chem Lett. 2010 May 15;20(10):3036-3038; WO2012050263A1; J. Org Chem. Chem 1996, 61, 3890; Chem. Pharm. Bull. 1991, 911; (5); J. med. Chem, 2013, 56, 4611).

While the present disclosure provides compositions comprising synthetically prepared endoxifen free base and endoxifen free base enriched from commercially available synthesized endoxifen, compositions comprising isolated endoxifen, for example, by ex-vivo and in-vitro transformation of tamoxifen to its active metabolites using microbial culture, cell culture (e.g., whole-cell culture), organ explant culture, CYP enzyme bioreactors, and biocatalysts followed by isolation of endoxifen ("isolated endoxifen") are also encompassed in the present disclosure (See, Kebamo et al., J Drug Metab Toxicol 2015, 6(5), 196). Thus, in another aspect, the present disclosure relates to compositions comprising endoxifen free base and salts thereof prepared using ex-vivo and in-vitro isolated endoxifen. Ex-vivo and in-vitro isolated endoxifen can be further enriched or purified for (Z)-endoxifen free base as described herein. Such ex-vivo and in-vitro isolated endoxifen are also useful for the synthesis of endoxifen salts. A person of skill in the art will be able to apply ordinary skill and knowledge to isolate and purify endoxifen using techniques known in the art and disclosed herein.

In some embodiments, an endoxifen D-gluconate (such as (Z)-endoxifen D-gluconate) salt may be obtained by mixing an ethanolic slurry of the endoxifen (such as (Z)-endoxifen) as the free base with an aqueous solution of D-gluconic acid, obtained by hydrolyzing a 20% w/v solution of D-gluconolactone in water by heating at 70°C for 15 to 30 min. In some embodiments, a minimum volume of ethanol is used and 5 ml of the aqueous D-gluconic acid solution is added per 1 g of endoxifen free base. Stirring is then continued until a clear solution is obtained. The required volume of this solution is then added to one or more of other excipients to produce formulations in which "active ingredient" is endoxifen D-gluconate salt.

Purified (Z)-endoxifen free base or a mixture of (E)/(Z)-endoxifen are both useful for the purpose of preparation of the endoxifen gluconate salt. Solution of (Z)-endoxifen D-gluconate and (Z)-endoxifen L-gluconate may also be purified from a mixture of (E)/(Z)-endoxifen gluconate salt as described herein by fractional crystallization or recrystallization (Examples 1 and 2) to obtain a solid purified (Z)-endoxifen salt. The solid salt may be stored at -5°C until further use.

It will be understood by those skilled in the art that other endoxifen salts can also be used as starting material with gluconate moiety or gluconate salts to yield endoxifen gluconate or chemical equivalents thereof.

Endoxifen gluconate or chemical equivalent thereof may be prepared using starting reactants that are readily available. For example, endoxifen HCl (≥98% purity) and sodium gluconate are readily available from Sigma-Aldrich, USA.

Suitable solvents for making endoxifen gluconate or chemical equivalents thereof include, but are not limited to, organic solvents such as alcohols, acetones, DMSO, polyethylene glycol, fatty acids and fatty alcohols and their derivatives, hydroxyl acids, pyrrolidones, urea, vegetable oils, animal oils such as fish oils, essential oils, and the like or mixtures thereof, and water-miscible solvents such as water miscible alcohols, dimethylsulfoxide, dimethylformamide, water-miscible ether, for example, tetrahydrofuran, water-miscible nitrile, for example acrylonitrile, a water miscible ketone such as acetone or methyl ethyl ketone, an amide such as dimethylacetamide, propylene glycol, glycerin, polyethylene glycol 400, glycofurol, tetraglycol, and the like, or mixtures thereof.

Water-miscible solvents useful for the preparation of endoxifen gluconate are glycerin, ethanol, propanol, isopropanol, propylene glycol, polyethylene glycols, or mixtures thereof. Additional solvents that are useful include diglycol monoethyl ether (transcutol); alkelene glycols, such as dipropylene glycol, propylene glycol, polyethylene glycols such as PEG 300, 400, 3395, 4450 and the like; dimethyl isosorbide; and dehydrated alcohol. In some embodiments, the solvent is a dehydrated alcohol, such as absolute alcohol. In certain embodiments, the amount of solvent is sufficient to dissolve the endoxifen (free base, salts) and gluconate salts. The concentration of the solvent can also be adjusted as needed. The reaction can be carried out at room temperature and at atmosphere of pressure.

The amount of endoxifen free base or endoxifen salt (such as endoxifen HCl and the like) and gluconate salt (such as and sodium gluconate and the like) that can be used to make endoxifen gluconate or chemical equivalent thereof can vary depending on the amounts of reactants used. The resulting endoxifen gluconate will have endoxifen:gluconate moiety at about 1:1 ratio.

In some embodiments, the amount of endoxifen or endoxifen salt used for making endoxifen gluconate is from 0.01% to 40% (e.g., from 1% to 10%, or from 3% to 5%) by weight of the total composition (w/w). In some embodiments, the gluconate salt used for making endoxifen gluconate is from 0.01% to 40% (w/w) (e.g., from 1% to 10% (w/w), or from 3% to 5% (w/w)). One of skill in the art will be guided by skill and knowledge in the field and the present disclosure, including without limitation, amounts of reactants which are effective to achieve the desired yields.

Methods of making gluconate salts of therapeutic agents are known in the art (for example, U.S. Publication No. 2002/0127665).

Stereospecific synthesis of (Z) tamoxifen may be carried as described in J. Org. Chem., 1985, 50 (12), pp 2121-2123. Iodoxifene may be synthesized using the methods described in Organic Preparations and Procedures International, The New Journal for Organic Synthesis, Volume 26, 1994 - Issue 3. Raloxifene synthesis has been described in U.S. Patent Application No. 20070100147, Heterletters, Vol. 4: (4), 2014, 515-518. Toremifene and its salts may be synthesized by methods described in CN 201410415900 and Chem. Res. Toxicol., 2001, 14 (12), pp 1643-165. Droloxifene and its salts may be synthesized as described in Chem. Res. Toxicol., 2001, 14 (12), pp 1643-1653, and Tetrahedron Letters Volume 47, Issue 10, 6 March 2006, Pages 1631-1635. Clomephene may be synthesized as described in WO2015138340, U.S. Patent Nos. 2,914,563 and 3,848,030, and ISRN Oncology, Volume 2012 (2012), Article ID 581281. Ormeloxifene may be prepared as described in WO2009078029 and Journal of Chemical and Pharmaceutical Research, 2015, 7(7):736-741. Lasofoxifene and nafoxidine and their salts may be synthesized as described in Synthetic Communications; An International Journal for Rapid Communication of Synthetic Organic Chemistry, Volume 46, 2016 - Issue 4, p309-313. Ospemifene may be synthesized as described in WO 2014060640, Eur J Med Chem. 2014 Oct 30;86:211-8. Fulvestrant may be prepared as described in WO 2014064712 and EP 2350111A1. Letrozole may be synthesized as described in U.S. Patent No. 7705159 and WO2009069140A1. Anastrozole may be synthesized as described in US 8058302. Exemestane may be synthesized as described in EP 1709062 A1.

Additional methods of making tamoxifen, raloxifene, endoxifen, toremifene, N-desmethyl-tamoxifen, iodoxifene, droloxifene, clomefine (clomiphene), ormeloxifene, lasofoxifene, nafoxidine, ospemifene, fulvestrant, letrozole, anastrozole, and exemestane and their salts and solvates are known in the art.

Although, active agents (for example, endoxifen free base and salts thereof) can be applied directly to surface the skin, it is generally desirable to administer them to the skin as topical compositions or formulations, in combination with one or more excipients such as of pharmaceutically acceptable carriers such as polar organic solvents.

In an aspect, provided herein is the at least one active agent which is solubilized endoxifen free base and salts and solvates thereof in suitable polar organic solvents such as dimethyl sulfoxide, diethyl sebacate, diisopropyl adipate, di-propylene glycol, ethanol, isopropanol, polyethylene glycols such as PEG300 and PEG400, propylene glycol, diethylene glycol monoethyl ether, oleic acid, capric triglyceride, caprylic triglyceride, caprylic/capric triglycerides, mineral oil, cetyl alcohol, and stearic acid (*See* **Table 1).** The endoxifen free base and salts and solvates thereof remain homogeneously distributed and not enclosed in lipid-complexes in the compositions disclosed herein. Accordingly, in at least one embodiment, compositions comprise lipid complex-free and uniformly distributed endoxifen free base, and salts thereof.

In an aspect, provided herein is that 0.01% to 10% (w/w) of (Z)-endoxifen or a salt thereof is dissolved in any of Solvents 1 to 3, Solvents 5 to 11 and Solvents 13 to 15 of **Table 1,** or a combination thereof. Endoxifen free base and salts thereof dissolved in the solvents with a solubility range of total endoxifen (Z+E) from 2.13 mg/g to over 50 mg/g of the solution. Solubility of Z-endoxifen ranged from ~ 1 mg/g to over 30 mg/g of solution **(Table 1).** The polar organic solvents of **Table 1** also serve to function as penetration enhancers or MPEs.

Certain embodiments of the present disclosure comprise: 0.01% to 20% (w/w) of endoxifen free base as the at least one active agent or a salt or solvate thereof; a first compound comprising diethyleneglycol monoethyl ether; and a second compound, wherein the first compound and second compound are different, and second compound is a penetration enhancer selected from the group consisting of DMSO, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof;
0.01% to 20% (w/w) of endoxifen free base as the at least one active agent or a salt or solvate thereof; a first compound comprising DMSO; and a second compound, wherein the second compound is a penetration enhancer selected from the group consisting of diethyleneglycol monoethyl ether, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof;
0.01% to 10% (w/w) (Z)-endoxifen free base or salts or solvates thereof; a first compound comprising diethyleneglycol monoethyl ether; isopropanol; and a second compound comprising mineral oil;
0.01% to (w/w) 10% (Z)-endoxifen free base or salts or solvates thereof; a first compound comprising diethyleneglycol monoethyl ether; and a second compound comprising diethyl sebacate;
0.01% to (w/w) 10% (Z)-endoxifen free base or salts or solvates thereof; a first compound comprising diethyleneglycol monoethyl ether; and a second compound comprising diisopropryl adipate;
0.01% to 10% (w/w) (Z)-endoxifen free base or salts or solvates thereof; a first compound comprising diethyleneglycol monoethyl ether; and a second compound comprising dipropylene glycol;
0.01% to 10% (w/w) (Z)-endoxifen free base or salts or solvates thereof; a first compound comprising diethyleneglycol monoethyl ether; and a second compound comprising polyethylene glycols;
0.01% to 10% (w/w) (Z)-endoxifen free base or salts or solvates thereof; a first compound comprising diethyleneglycol monoethyl ether; and a second compound comprising isopropanol;
0.01% to 10% (w/w) (Z)-endoxifen free base or salts or solvates thereof; a first compound comprising diethyleneglycol monoethyl ether; and a second compound comprising t-butanol;
0.01% to 10% (w/w) (Z)-endoxifen free base or salts or solvates thereof; a first compound comprises diethyleneglycol monoethyl ether and a second compound comprises polyethylene glycol dodecyl ether;
0.01% to 10% (w/w) (Z)-endoxifen free base or salts or solvates thereof; a first compound comprises diethyleneglycol monoethyl ether and a second compound comprises cetyl alcohol;
0.01% to 10% (w/w) (Z)-endoxifen free base or salts or solvates thereof; a first compound comprises diethyleneglycol monoethyl ether and a second compound comprises mineral oil;
0.01% to 10% (w/w) (Z)-endoxifen free base or salts or solvates thereof; a first compound comprises diethyleneglycol monoethyl ether and a second compound comprises caprylic triglyceride;
0.01% to 10% (w/w) (Z)-endoxifen free base or salts or solvates thereof; a first compound comprises diethyleneglycol monoethyl ether and a second compound comprises capric triglyceride;
0.01% to 10% (w/w) (Z)-endoxifen free base or salts or solvates thereof; a first compound comprises diethyleneglycol monoethyl ether and a second compound comprises caprylic/capric triglycerides;
0.01% to 10% (w/w) (Z)-endoxifen free base or salts or solvates thereof; a first compound comprises diethyleneglycol monoethyl ether and a second compound comprises stearic acid;
0.01% to 10% (w/w) (Z)-endoxifen free base or salts or solvates thereof; a first compound comprises DMSO and a second compound comprises diethyleneglycol monoethyl ether;
0.01% to 10% (w/w) (Z)-endoxifen free base or salts or solvates thereof; a first compound comprises DMSO and a second compound comprises diethyl sebacate;
0.01% to 10% (w/w) (Z)-endoxifen free base or salts or solvates thereof; a first compound comprises DMSO and a second compound comprises diisopropryl adipate;
0.01% to 10% (w/w) (Z)-endoxifen free base or salts or solvates thereof; a first compound comprises DMSO and a second compound comprises dipropylene glycol;
0.01% to 10% (w/w) (Z)-endoxifen free base or salts or solvates thereof; a first compound comprises DMSO and a second compound comprises polyethylene glycols;
0.01% to 10% (w/w) (Z)-endoxifen free base or salts or solvates thereof; a first compound comprises DMSO and a second compound comprises isopropanol;
0.01% to 10% (w/w) (Z)-endoxifen free base or salts or solvates thereof; a first compound comprises DMSO and a second compound comprises t-butanol;
0.01% to 10% (w/w) (Z)-endoxifen free base or salts or solvates thereof; a first compound comprises DMSO and a second compound comprises polyethylene glycol dodecyl ether;
0.01% to 10% (w/w) (Z)-endoxifen free base or salts or solvates thereof; a first compound comprises DMSO and a second compound comprises cetyl alcohol;
0.01% to 10% (w/w) (Z)-endoxifen free base or salts or solvates thereof; a first compound comprises DMSO and a second compound comprises mineral oil;
0.01% to 10% (w/w) (Z)-endoxifen free base or salts or solvates thereof; a first compound comprises DMSO and a second compound comprises caprylic triglyceride;
0.01% to 10% (w/w) (Z)-endoxifen free base or salts or solvates thereof; a first compound comprises DMSO and a second compound comprises capric triglyceride;
0.01% to 10% (w/w) (Z)-endoxifen free base or salts or solvates thereof; a first compound comprises DMSO and a second compound comprises caprylic/capric triglycerides;
0.01% to 10% (w/w) (Z)-endoxifen free base or salts or solvates thereof; a first compound comprises DMSO and a second compound comprises and stearic acid;
0.01% to 10% (w/w) (Z)-endoxifen free base or salts or solvates thereof; diethyleneglycol monoethyl ether; isopropanol; and caprylic/capric triglycerides;
0.01% to 10% (w/w) (Z)-endoxifen free base or salts or solvates thereof; diethyleneglycol monoethyl ether; isopropanol; mineral and caprylic/capric triglycerides;
0.01% to 10% (w/w) (Z)-endoxifen free base or salts or solvates thereof; diethyleneglycol monoethyl ether; isopropanol; and capryic triglyceride;
0.01% to 10% (w/w) (Z)-endoxifen free base or salts or solvates thereof; diethyleneglycol monoethyl ether; isopropanol; capryic triglyceride; and soybean oil.
0.01% to 10% (w/w) (Z)-endoxifen free base or salts or solvates thereof; diethyleneglycol monoethyl ether; diethyl sebacate; diisopropyl adipate; polypropylene glycols and capric triglyceride;
0.01% to 10% (w/w) (Z)-endoxifen free base or salts or solvates thereof; a first compound comprising diethyleneglycol monoethyl ether; and a second compound comprising isopropanol; and mineral oil;
0.01% to 10% (w/w) (Z)-endoxifen free base or salts or solvates thereof; a first compound comprising diethyleneglycol monoethyl ether; and a second compound comprising isopropanol; mineral oil and caprylic/capric glycerides;
0.01% to 10% (w/w) (Z)-endoxifen free base or salts or solvates thereof; a first compound comprising 15% to 45% (w/w) diethyleneglycol monoethyl ether; and a second compound comprising 5% to 30% (w/w) isopropanol; 25% to 45% (w/w) caprylic/capric trigycerides; and q.s. 100% (w/w) with mineral oil;
0.01% to 10% (w/w) of (Z)-endoxifen or a salt thereof; a first compound comprising 25% to 65% (w/w) DMSO; and a second compound comprising 10% to 30% (w/w) of diethyl sebacate; 5% to 20% (w/w) of dipropylene glycol; 5% to 20% (w/w) of diisopropyl adipate; and q.s 100% (w/w) of polyethylene glycol such as PEG300;
0.01% to 10% (w/w) of (Z)-endoxifen or a salt thereof; a first compound comprising 25% to 55% (w/w) diethylene glycol monoethyl ether; and a second compound comprising 5% to 20% (w/w) of isopropanol; 20% to 40% (w/w) of capric triglyceride; and q.s. 100% (w/w) soybean oil;
0.01% to 10% (w/w) of (Z)-endoxifen or a salt thereof; a first compound comprising 25% to 65% (w/w) diethylene glycol monoethyl ether; and a second compound comprising 10% to 30% (w/w) of diethyl sebacate; 5% to 20% (w/w) of diisopropyl adipate; and q.s 100% (w/w) of polyethylene glycol such as PEG300;
0.01% to 10% (w/w) of (Z)-endoxifen or a salt thereof; a first compound comprising 25% to 65% (w/w) DMSO; and a second compound comprising 5% to 15% (w/w) of dipropylene glycol; 5% to 20% (w/w) of isopropanol; 0.01% to 10% surfactant such as polyethylene glycol dodecyl ether (Brij L4), Tween 20 and the like; and q.s. 100% (w/w) polypropylene glycols such as PEG 300;
0.01% to 10% (w/w) of (Z)-endoxifen or a salt thereof; a first compound comprising15% to 45% (w/w) DMSO; and a second compound comprising15% to 45% (w/w) diethylene glycol monoethyl ether; 5% to 15% (w/w) of dipropylene glycol; 5% to 20% (w/w) of diisopropyl adipate; 15% to 40% (w/w) of diethyl sebacate; and q.s. 100% (w/w) mineral oil;
0.01% to 10% (w/w) of (Z)-endoxifen or a salt thereof; a first compound comprising 10% to 30% (w/w) DMSO; and a second compound comprising 20% to 60% (w/w) of caprylic/capric triglyceride; 5% to 20% (w/w) of isopropanol; and q.s. 100% (w/w) soybean oil;
0.01% to 10% (w/w) of (Z)-endoxifen or a salt thereof; a first compound comprising10% to 40% (w/w) diethylene glycol monoethyl ether; and a second compound comprising 20% to 60% (w/w) of caprylic/capric triglyceride; 5% to 20% (w/w) of isopropanol; and q.s. 100% (w/w) mineral oil;
0.01% to 10% (w/w) of (Z)-endoxifen or a salt thereof; a first compound comprising10% to 40% (w/w) diethylene glycol monoethyl ether; a second compound comprising 20% to 60% (w/w) of caprylic/capric triglyceride; 5% to 20% (w/w) of isopropanol; 0.01% to 20% (w/w) cetyl alcohol; and q.s. 100% (w/w) mineral oil;
0.01% to 10% (w/w) of (Z)-endoxifen or a salt thereof; a first compound comprising 10% to 40% (w/w) diethylene glycol monoethyl ether; and a second compound comprising 20% to 60% (w/w) of caprylic/capric triglyceride; 5% to 20% (w/w) of isopropanol; 0.01% to 20% (w/w) cetyl alcohol; 0.01% to 20% (w/w) stearic acid; and q.s. 100% (w/w) mineral oil;
0.01% to 10% (w/w) of (Z)-endoxifen or a salt thereof; a first compound comprising10% to 40% (w/w) diethylene glycol monoethyl ether; and a second compound comprising 20% to 60% (w/w) of caprylic/capric triglyceride; 1% to 20% (w/w) of dimethyl sebacate; 5% to 20% (w/w) of isopropanol; and q.s. 100% (w/w) mineral oil.

It is an aspect of the present disclosure that the topical compositions disclosed herein are stable for at least 18 months. The stability of a drug product composition can have a significant impact on the length and cost of drug development, the nature of the studies required to support regulatory submissions, and the ultimate safety and approvability. It is important for instance to minimize the amount of impurities or degradation products that form over time due to interactions between the various ingredients in a composition. This can be particularly important in compositions that are designed to increase skin permeability.

It is an aspect of the present disclosure that (Z)-endoxifen or salts or solvates thereof present in the topical compositions undergo reduced interconversion from (Z)-endoxifen isoform to (E)-endoxifen isoform.

Elkins et al. provide t₉₀ of 149 days at room temperature and t₉₀ of 9 days at 45°C and observed degradation of (Z)-endoxifen HCl in aqueous medium from 98% to 75% at 45°C on day 15 (J Pharm Biomed Anal 2014, 88:174-179). In contrast to the published information, the topical compositions of the present disclosure provide surprising additional advantage of favorable stability at 15 weeks at 40°C (**Table 6**) as reflected in the lack of any substantial changes in the levels of (Z)-endoxifen (<10% drop in potency and (Z):(E) ratio remaining above 60:40), the absence of phase separation and crystallization at low temperatures, and a low level of impurities.

Accelerated stability tests at elevated temperatures are predictive of long term (at least 18 m) stability at ambient temperature. Ten (10) days accelerated stability studies and 3.5 months stability studies at 40°C (**Tables 5** & **6**) indicate that (Z)-endoxifen or salts or solvates thereof present in topical compositions disclosed herein are likely to remain stable at ambient temperature for the long term period. Thus, in some embodiments, the topical compositions comprising (Z)-endoxifen and salts and solvates thereof are stable at ambient temperature for at least 6 months, at least 9 months, at least 12 months, at least 15 months, and at least 18 months. In certain embodiments, the present disclosure provides topical compositions that degrades by less than 10%, less than 8%, less than 5%, less than 4%, less than 3%, less than 2% and less than 1% over the course of 18 months at ambient temperature. In certain embodiments, the rate of degradation is less than 0.9%, 0.8%, 0.7%, 0.6%, 0.5%, 0.4%, 0.3%, 0.2%, or less than 0.1%, and all fractions in between, over the course of 18 months at ambient temperature. Accordingly, in certain embodiments, the topical compositions comprising (Z)-endoxifen or salts or solvates thereof are stable at ambient temperature for at least 6 months, at least 9 months, at least 12 months, at least 15 months, and at least 18 months. The (Z):(E) ratio of endoxifen and salts and solvates thereof is maintained at least 60:40 at ambient temperature.

In another aspect of the present disclosure, the topical compositions of the present disclosure can include more than one active agent. In certain embodiments, the topical compositions of the present disclosure include one or more additional therapeutic agents. Combination of multiple active agents can reduce the number of medications a subject will need to ingest resulting potentially in improved patient compliance. For example, when a subject has prostate cancer and is on bicalutamide, enzalutamide, or abiraterone acetate therapy for the treatment of prostate cancer, the subject is likely to develop gynecomastia as a result of the therapy. The topical compositions comprising endoxifen and salts and solvates thereof and a prostate cancer chemotherapy drug such as bicalutamide, enzalutamide, or abiraterone acetate in a single composition can be administered to the subject having prostate cancer in order to prevent and/or treat gynecomastia.

As another example, a combination of any one or more of oncology drugs (such as trastuzumab, bevacizumab, everolimus, anti-neoplastics such as capecitabine, goserelin acetate, immune-oncology drugs such as nivolumab (Opdivo^{™}), pembrolizumab (Keytruda^{™}), atezolizumab (Tecentriq^{™}), durvalumab (Imfinzi^{™}), and avelumab (Bavencio^{™}) and other check point inhibitors known in the art, immunotherapeutics), and endoxifen or a salt or solvate thereof can be including into a single composition for the treatment of a subject with ER+/Her2+ positive breast cancer as a combination therapy.

Accordingly, in some embodiments, the compositions further comprise a second therapeutic agents such as bicalutamide, enzalutamide, abiraterone acetate, and oncology drugs such as antineoplastics such as capecitabine (Xeloda), carboplatin (Paraplatin), cisplatin (Platinol), cyclophosphamide (Neosar), docetaxel (Docefrez, Taxotere), doxorubicin (Adriamycin), pegylated liposomal doxorubicin (Doxil), epirubicin (Ellence), fluorouracil (5-FU, Adrucil), gemcitabine (Gemzar), methotrexate (multiple brand names), paclitaxel (Taxol), protein-bound paclitaxel (Abraxane), vinorelbine (Navelbine), eribulin (Halaven), ixabepilone (Ixempra), goserelin acetate, trastuzumab, ado-trastuzumab, bevacizumab, everolimus, check point inhibitors (such as pembrolizumab (Keytruda^{™}), nivolumab (Opdivo^{™}), atezolizumab (Tecentriq^{™}), durvalumab (Imfinzi^{™}), and avelumab (Bavencio^{™})).

In another aspect, a composition disclosed herein may comprise therapeutic agents that increase bioavailability of endoxifen in a subject. P-glycoprotein (P-gp, ABCB1) is a highly efficient drug efflux pump expressed in brain, liver, and small intestine, but also in cancer cells, that affects pharmacokinetics and confers therapy resistance for many anticancer drugs. Accordingly, in some embodiments, the compositions further comprise inhibitors of ATP-binding cassette (ABC family) transporters, such as inhibitors of breast cancer resistance protein (BCRP protein) and P-gp. Several inhibitors of BCRP protein and P-Gp are known in the art. For example, inhibitors of BCRP protein include cyclosporine, omeprazole, pantoprazole, saquinavir, and tacrolimus.

Non-limiting examples of P-gp inhibitors include first generation inhibitors such as Verapamil, cyclosporin A, reserpine, quinidine, yohimbine, tamoxifen and toremifene, second generation inhibitors such as Dexverapamil, dexniguldipine, valspodar (PSC 833), and Dofequidar fumarate (MS-209), third generation P-gp inhibitors such as Cyclopropyldibenzosuberane zosuquidar (LY335979), laniquidar (R101933), mitotane (NSC-38721), biricodar (VX-710), elacridar (GF120918/GG918), ONT-093, tariquidar (XR9576), and HM30181 and anti-P-gp monoclonal antibodies such as MRK-16).

Other active agents that may be included in the topical formulations disclosed herein are listed in 2000 MedAd News 19:56-60; and Physicians Desk Reference, 53rd Ed. Pages 792 - 796, Medical Economics company.

The at least one active agent, such as (Z)-endoxifen can remain homogeneously distributed and not enclosed in lipid complex in the topical compositions disclosed herein. Accordingly, in some embodiments, topical compositions comprise lipid complex-free and uniformly distributed endoxifen free base, and salts and solvates thereof.

The topical formulation of the present disclosure may be formulated by those skilled in the art as liquids, solutions, emulsions, creams, lotions, suspensions, triturates, gels, jellies, foams, pastes, ointments, shampoos, adhesives, and the like.

The penetration enhancing effect may be measured using techniques known in the art. An example of one measurement method is described in the Examples below.

The present disclosure provides a topical formulation for facilitating topical or transdermal administration of endoxifen free base and salts and solvates thereof. This enhanced effect is discussed further below and illustrated in certain embodiments of the disclosure described herein, including in the Examples. In another embodiment, the topical formulation of the present disclosure provides a means for targeting a therapeutically active agent to local tissue either in the skin or the underlying tissue. This latter embodiment may be particularly beneficially applied for treatment of conditions such as a hormone-dependent breast disorder or hormone-dependent reproductive tract disorder.

Transdermal administration can be achieved in different ways. One such non-limiting example includes mixing the composition of endoxifen with suitable pharmaceutical carriers and penetration enhancers to form solutions, ointments, emulsions, gel, lotion, creams or the like, where the topical composition is applied onto a certain area of the skin, for example breast skin. The topical formulations may be applied either occlusively or nonocclusively to the skin. In another embodiment of the disclosure, in applications where the objective is transdermal administration of a therapeutically active agent to the systemic circulation, the topical formulation is applied to the skin under occlusion in a transdermal patch. For example, the topical compositions comprising endoxifen and salts and solvates thereof can be incorporated into patches or other transdermal delivery systems according to the technology known in the art.

Accordingly, the present composition comprising endoxifen free base and salts thereof may be applied topically to the surface of skin by any means known and include delivery to a subject in need thereof topically or transdermally using transdermal delivery systems such as an applicator, brush, swab, a patch, a tape, sheet, a dressing, a spray device and an aerosolizer. Such transdermal delivery systems may be a fixed dose or a metered dose system for delivery of unit doses, such as a stored-energy metered dose pump or a manual metered dose pump. The drug delivery system may be a unit volume dispenser with or without a roll-on or other type of applicator. It may also be necessary to apply a number of dosages on untreated skin to obtain the desired result.

The drug delivery system is applied to the skin of the subject covering a delivery surface area between about 0.1 and 10 cm², between about 1 and 5 cm2, and about 1.5 to 2 cm². The drug delivery system may be a unit volume dispenser with or without a roll-on or other type of applicator. It may also be necessary to apply a number of dosages on untreated skin to obtain the desired result.

For example, in some embodiments, the patch for transdermal delivery comprises: (i) a backing layer; and (ii) an adhesive layer having a skin-contacting adhesive surface; and wherein the adhesive layer comprises a drug reservoir containing a composition comprising endoxifen free base and salts thereof as disclosed herein. In other embodiments, a patch will typically include 4 elements: a backing, an adhesive, a release liner, and the drug. In addition, the reservoir and multilaminate designs may include a membrane film that controls the rate of delivery from the patch.

In some embodiments, the patch may comprise (i) a solution-impermeable backing foil, (ii) a reservoir, (iii) a microporous or semipermeable membrane, (iv) a self-adhesive layer, and (v) optionally a removable backing film. The reservoir may be formed by the backing foil and the membrane.

In other embodiments, the patch comprises: (i) a backing layer; (ii) a drug reservoir disposed on a first layer; and (iii) a skin-contacting second layer comprising a pressure sensitive adhesive layer; and wherein the second layer is attached to a surface of the first layer opposed to a surface in contact with the backing layer, and wherein the second layer is a rate-controlling layer, and wherein the drug reservoir comprises a composition comprising endoxifen free base or salts thereof disclosed herein.

In still other embodiments, the patch comprises: (i) a backing layer; (ii) a drug reservoir disposed on a first layer; (iii) a second layer comprising a rate-controlling membrane, the membrane being attached to a surface of the first layer opposed to a surface in contact with the backing layer; and (iv) a skin-contacting third layer comprising a pressure sensitive adhesive attached to a surface of the membrane that is opposed to the surface of the rate-controlling membrane in contact with the first layer; and wherein the drug reservoir comprises a composition comprising endoxifen free base or a salt thereof that releases endoxifen or a salt thereof sufficient to treat a hormone dependent breast disorder and hormone dependent reproductive tract disorders for at least three days.

In some embodiment, the patch comprises sufficient amount of a composition disclosed herein to deliver endoxifen free base and salts thereof to a subject for at least 3 days, at least 7 days, at least 10 days, at least 14 days, at least 15 days, at least 30 days, at least 1 month, at least 3 months, and at least 6 months.

Methods and polymers used for making patches are known in the art (Kandavilli et al. Pharmaceutical Technology. May 2002, pages 62 to 80).

One of skill in the art will further recognize that compositions disclosed herein may comprise one or more of the excipients known in the art and disclosed herein in any combination appropriate for a desired formulation. Additional excipients may generally be found in Remington's The Science and Practice of Pharmacy, Meade Publishing Co. United States Pharmacopeia/National formulary. One of skill in the arts will be able to select suitable excipients necessary for the preparation of the formulations and appropriate dosage forms compatible with the route of administration based on his or her skill and knowledge in the art and the disclosures made herein. In all cases, the ultimate dosage form should be sterile and stable under the conditions of manufacture and storage.

### Compositions for Use

In an aspect, the present disclosure provides compositions according to the claims for use in methods of treatment of subjects in need
thereof wherein the subjects are administered one or more topical compositions disclosed herein.

In another aspect, topical compositions disclosed herein may be used for the treatment of subjects having or at risk of having a hormone-dependent breast disorder or hormone-dependent reproductive tract disorder or both.

The topical compositions of the present disclosure may be used as a primary therapy, as a part of a neo-adjuvant therapy (to primary therapy), or as part of adjuvant therapy regimen, where the intention is to ameliorate or cure a subject having or at risk of having a hormone-dependent breast disorder or hormone-dependent reproductive tract disorder or both. Accordingly, the present disclosure provides compositions according to the claims for use in methods of treating a subject having or at risk of having a hormone-dependent breast disorder or hormone-dependent reproductive tract disorder or both, wherein the subject is administered a topical composition disclosed herein.

In certain embodiments, the disorder is a hormone-dependent breast disorder. In other embodiments, the disorder is hormone-dependent reproductive tract disorder. In still other embodiments, the subject has both, a hormone-dependent breast disorder and a hormone-dependent reproductive tract disorder. In some embodiments, the hormone dependent disorder is a benign breast disorder, hyperplasia, atypia, atypical ductal hyperplasia, atypical lobular hyperplasia, increased breast density, gynecomastia, McCune-Albright Syndrome, precocious puberty, DCIS, LCIS, breast cancer, endometrial cancer, ovarian cancer, uterine cancer, cervical cancer, vaginal cancer or vulvar cancer.

In some embodiments, the breast disorder is increased breast density. For example, the breast disorder is a class B (formerly Class II), class C (formerly class III) or class D (formerly class IV) breast density. In some embodiments, subjects have increased mammographic breast density classified as class C or class D breast density.

In some embodiments, the hormone-dependent breast disorder or hormone-dependent reproductive tract disorder is precocious puberty. In other embodiments, the hormone-dependent breast disorder or hormone-dependent reproductive tract disorder is McCune-Albright Syndrome.

In some embodiments, the breast disorder is gynecomastia. In some embodiments, gynecomastia is presented secondarily to an underlying disease. Accordingly, in some embodiments the subject also has underlying disease selected from the group consisting of prostate cancer, cirrhosis and liver disease, male hypogonadism, hyperthyroidism, renal failure and in patients undergoing hemodialysis, or type I diabetes mellitis. In certain embodiments, the subject has prostate cancer as the underlying disease. In certain other embodiments, subject with prostate cancer undergoing or about to initiate chemotherapeutic treatment may present with or have a risk of developing drug-induced gynecomastia. In one aspect, the present disclosure provides compositions according to the claims for use in methods of treatment of a subject with prostate cancer having or at risk of having a hormone-dependent disorder such as gynecomastia. Subjects with gynecomastia undergo irreversible tissue changes within 12 to 18 months of showing symptoms of gynecomastia. Accordingly, the present disclosure provides compositions according to the claims for use in methods of treating a subject with gynecomastia within 12 months of symptomology (i.e., showing symptoms of gynecomastia).

Bannayan and Hadju (A. J. C. P. Vol. 57, 1972) have described three histological types of gynecomastia: florid, fibrous, and intermediate. The florid type is characterized by ductal hyperplasia and proliferation, with loose and edematous stroma. The fibrous type contains more stromal fibrosis and fewer ducts. As its name infers, the intermediate type of gynecomastia presents features of the two. In another aspect, the present disclosure provides compositions according to the claims for use in methods of treatment of subject showing florid and intermediate gynecomastia as determined by histological changes. In another aspect, the present disclosure provides compositions according to the claims for use in a treatment window for the subjects with fibrous type of gynecomastia. Accordingly, provided herein are compositions according to the claims for use in methods of treatment of subject with gynecomastia showing fibrous histological changes wherein the subject present with fibrous gynecomastia for less than 1 year.

In certain embodiments, the breast cancer is DCIS, LCIS, ILC, IDC, MIC, inflammatory breast cancer, ER-positive (ER+) breast cancer, HER2+ breast cancer, adenoid cystic (adenocystic) carcinoma, low-grade adenosquamatous carcinoma, medullary carcinoma, mucinous (or colloid) carcinoma, papillary carcinoma, tubular carcinoma, metaplastic carcinoma, or micropapillary carcinoma. In at least one embodiment, a single breast cancer tumor may be a combination of the foregoing or be a mixture of invasive and in situ cancer.

The present disclosure contemplates the compounds and compositions disclosed herein for use at various stages in tumor development and progression, including the treatment of advanced and/or aggressive neoplasia, i.e., overt disease in a subject that is not amenable to cure by local modalities of treatment such as surgery or radiotherapy, metastatic disease, locally advanced disease. Accordingly, in some embodiments the breast cancer is a pre-cancer, an early stage cancer, a non-metastatic cancer, a pre-metastatic cancer, or a locally advanced cancer. In at least one embodiment, the breast disorder is metastatic cancer. In some embodiments, the subject further has prostate cancer and has or is at risk of having gynecomastia; or has initiated or is about to initiate chemotherapy.

Tamoxifen remains the current choice for therapeutics for such disorders despite the various serious adverse effects, poor patient compliance and resistance to the drug due to low plasma endoxifen levels seen in subjects taking tamoxifen. Such subjects may have low endoxifen levels upon dosing with tamoxifen for any number of reasons such as having CYP gene mutations, for example, in CYP2D6, CYP3A4, CYP2C9 making them unable to metabolize tamoxifen to its active metabolite, endoxifen, or low or dysfunctional estrogen receptor preventing (or decreasing) sufficient tamoxifen uptake, for other reasons yet to be identified. Notwithstanding the mechanism underlying the low plasma endoxifen in a subject, the compositions of the present disclosure are useful for any condition wherein a subject has low endoxifen when dosed with tamoxifen or the subject has or is at a risk of having hormone-dependent breast disorder or hormone-dependent reproductive tract disorder. Therefore, the compositions of the present disclosure can be particularly important in the treatment of tamoxifen-resistant hormone-dependent breast disorders or hormone-dependent reproductive tract disorders.

Provided herein are patient populations for whom the pharmaceutical compositions are particularly useful. The compositions of the present disclosure are also particularly important in the treatment of subjects belong to tamoxifen-refractory patient population with hormone-dependent breast disorders or hormone-dependent reproductive tract disorders. Accordingly, in some embodiments, the compositions disclosed herein are useful for the treatment of tamoxifen refractory or tamoxifen resistant subjects having or at risk of having hormone-dependent breast disorders or hormone-dependent reproductive tract disorders or both. In some embodiments, topical compositions comprising endoxifen and salts and solvates thereof (such as topical (Z)-endoxifen free base, (E)/(Z)-endoxifen free base mixture, endoxifen gluconate, endoxifen HCl and endoxifen citrate) administered to such subject at the doses disclosed herein will be advantageous.

In some embodiments, topical administration of compositions disclosed herein will maintain a subjects' plasma endoxifen at levels ≤ 30 nM, ≤ 25 nM, ≤ 20 nM, ≤ 15 nM, ≤ 12 nM, ≤ 10 nM, ≤ 8 nM, ≤ 6 nM, ≤ 5 nM, ≤ 4 nM, ≤ 3 nM, ≤ 2 nM, or ≤ 1 nM. In some embodiments, the administration of topical compositions disclosed herein will maintain subjects' plasma endoxifen at steady state levels of ≤ 30 nM. In at least one embodiment, the administration of topical compositions disclosed herein will maintain subjects' plasma endoxifen at steady state levels of ≤15 nM. Administration of the topical compositions of the present disclosure to the breast tissues, such as breast skin or a breast duct is particularly advantageous for maximum delivery and uptake locally for treatment with little systemic blood levels of endoxifen thereby reducing the potential for adverse effects seen with tamoxifen *(See* **Example 9**). In certain embodiments, the administration of the compositions of the present disclosure via topical, transdermal, transpapillary and intraductal modes of delivery avoids hepatic metabolism.

Whether a subject is tamoxifen-refractory or tamoxifen resistant may be determined by dosing a subject with an initial dosage of tamoxifen (a first composition comprising tamoxifen) and determining the subject's plasma endoxifen level. Plasma endoxifen levels in a subject dosed with tamoxifen serves as a biomarker for the tamoxifen-refractory subjects. The plasma endoxifen levels (acute and/or steady state) may be determined by obtaining from the subject a test sample, which may be blood sample, collected from the subject after dosing the subject with tamoxifen. Plasma or serum may be obtained from blood samples for testing the biomarker endoxifen levels. The initial dosage may comprise administering tamoxifen daily for at least 1 day, 2 days, 3 days, 15 days, 1 week, 2 weeks, 4 weeks, 1 month, 2 months, 3 months, 4 months, 5 months, or 6 months. The subject may also be administered with a first composition comprising tamoxifen daily for at least 1 day, 2 days, 3 days, 15 days, 1 week, 2 weeks, 4 weeks, 1 month, 2 months, 3 months, 4 months, 5 months, 6 months, 1 year, 2 years, 3 years, 4 years, 5 years or 10 years.

Subject's plasma endoxifen level may be determined by measuring endoxifen in the test sample. The subject's plasma endoxifen levels are compared to a reference plasma endoxifen level. For the purposes of the present disclosure, the reference plasma level is 30 nM. If the subject's plasma endoxifen steady state level is determined to be lower than 30 nM, then subject is defined as tamoxifen-refractory. Such a tamoxifen-refractory subject who has or who may be at risk of having a hormone-dependent breast disorder or hormone-dependent reproductive tract disorder is treated by administering to the subject, a topical composition disclosed herein (such as a topical composition comprising endoxifen free base or a salt or solvate thereof disclosed herein). Such subjects may be administered a topical composition disclosed here topically, transdermally, transpapillarily or intraductally.

In some embodiments, the topical composition administered to such as subject comprises (Z)-endoxifen free base. In other embodiments, the compositions administered may comprise an endoxifen salt selected from the group consisting of acetate, adipate, alginate, aspartate, arecoline, benzoate, benzenesulfonate, bisulfate, butyrate, besylate, bicarbonate, bitartarate, bromide, butylbromide, camysylate, chloride, citrate, camphorate, camphorsulfonate, cyclopentanepropionate, dodecylsulfate, ethanesulfonate, fumarate, flucoheptanoate, glycerophosphate, gluconate, glutamate, glycollylarsanilate, 2-hydroxyethanesulfonate, hemisulfate, heptanoate, hexanoate, hexylresorcinate, hydrabamine, hydrobromide, hydroxynapthanoate, iodide, isethionate, lactate, malate, maleate, mandelate, methanesulfonate, mesylate, methylbromide, methylbromide, methylnitrate, methylsulfate, mucate, napsylate, 2-naphthalenesulfonate, nicotinate, nitrate, oxalate, palmoate, pamaoate (Embonate), pantothenate, pectinate, persulfate, phenylpropionate, picrate, pivalate, propionate, phosphate/diphosphate, polygalacuronate, salicylate, stearate, sulfate, succinate, tartrate, thiocyanate, tosylate, tannate, teoclate, triethiodide, undecanoate and the like. In other embodiments, the cation salts of endoxifen are selected from the group consisting of benzathine, clemizole, chloroprocaine, choline, diethylamine, diethanolamine, ethylenediamine, meglumine, piperazine, procaine, aluminum, barium, bismuth, lithium, magnesium, potassium, and zinc. In other embodiments, the composition comprising endoxifen is endoxifen HCl or endoxifen citrate. In other embodiments, the composition administered to such a subject comprises endoxifen gluconate selected from the group consisting of (Z)-endoxifen D-gluconate, (Z)-endoxifen L-gluconate, (E)-endoxifen D-gluconate, (E)-endoxifen L-gluconate, or a combination thereof.

The present disclosure further provides that the efficacy and safety of the compositions and the treatment regimen disclosed herein may be assessed during and after treatment. The present disclosure also provides that a subject's breast condition may be determined prior to prophylactic treatment with the topical compositions disclosed here. For example, the present disclosure provides that women with prior history and/or family history of breast disorders or estrogen-related disorders are tested for a risk of developing or for reoccurrence of hormone-dependent breast disorders or hormone-dependent reproductive tract disorders or both, and administered with the topical compositions disclosed herein for prevention and treatment of such disorders. Accordingly, the methods described herein may comprise assessing a subject's breast condition (such as risk for developing, recurrence, and prognosis of breast disorder and/or estrogen-related disorder) before, during and/or following treatment with the compositions disclosed herein. Methods described herein may comprise collecting biological samples from a subject, testing the biological samples, based on the test results predicting or diagnosing hormone-dependent breast disorders or hormone-dependent reproductive tract disorders or both in the subject, and administering to a subject in need thereof a topical composition of the present disclosure.

In an aspect, the present disclosure also provides that a subject's plasma endoxifen levels are tracked or monitored periodically or as necessary to monitor the occurrence or progression (or lack thereof) of disease. If required, a subject who has been administered with an initial dosage of tamoxifen may have his or her plasma endoxifen levels adjusted by administering a composition comprising endoxifen free base or salts thereof as disclosed herein on an ongoing basis based on the test results.

In some embodiments, the subject's tamoxifen-refractory status may be determined by determining the subject's tamoxifen-metabolites profile which is compared with a reference tamoxifen-metabolite profile as seen in control or normal subjects. Subjects with low plasma endoxifen levels in subject's tamoxifen-metabolite profile as compared to the reference tamoxifen-metabolite profile are administered a topical composition comprising endoxifen or a salt thereof disclosed herein. Such compositions may comprise synthetically prepared endoxifen or isolated endoxifen, or salts or solvates thereof.

The plasma endoxifen may be measured by any of the methods known in the art. The levels of plasma endoxifen in test sample may be determined based on subject's CYP genes, DNA, RNA, endoxifen protein, tamoxifen-metabolite profile or a combination thereof. The tamoxifen-metabolites profile can include a panel containing at least tamoxifen, 4-OHT, N-desmethyltamoxifen, and endoxifen in a test such as those offered by Quest Laboratories.

In some embodiments, the level of plasma endoxifen and/or tamoxifen-metabolite profile in the test sample is measured by High Performance Liquid Chromatography (HPLC), Gas Chromatography Mass Spectrometry (GC-MS), Liquid Chromatography Mass spectrometry (LC-MS), Liquid Chromatography Tandem Mass spectrometry (LC-MS/MS), immunohistochemistry (IHC), polymerase chain reaction (PCR), quantitative PCR (qPCR), and the like. In some embodiments, the tamoxifen-metabolites profile is predicted based on the subject's genetic composition. In some embodiments, the subject's CYP genotype includes, without limitation, CYP2D6, CYP3A4, CYP2C9 genes are analyzed. In some embodiments, subject's estrogen receptor levels may be analyzed. In other embodiments, the determination of plasma endoxifen may be done by third party laboratories.

In another aspect, the subjects may have their test samples tested for their biomarker profile that may be indicative of a hormone-dependent breast disorder or a hormone-dependent reproductive tract disorder or both or for monitoring the progress (or lack thereof or remission) of the hormone-dependent disorder(s) disclosed herein. Such biomarkers are known in the art, and include, by way of non-limiting examples, biomarkers such as CYP2D6, BRCA-1, BRCA-2, ER, PR, Her2, uPA, PAI, Tf, p53, Ki67, cytokeratins, cancer tumor antigens, and other biomarkers measured by Mammaprint, OncotypeDx, PAM50, EndoxPredict, MammoStrat, and other diagnostic and predictive tests. In at least one embodiment, the subject's biomarker is Ki67. A subject with biomarker profile indicating that the subject has or is at risk of having a of a hormone-dependent breast disorder or a hormone-dependent reproductive tract disorder or both, can be administered a topical composition disclosed herein.

In another aspect, the subject's biomarker profile may be monitored before, during and after the treatment period to determine the efficacy and prophylactic effect of the composition on patient's disease burden and risk of re-occurrence of the disease.

In some aspects, provided herein are compositions according to the claims for use in methods of treating a tamoxifen-refractory or tamoxifen-resistant subject, the method comprising administration to the subject a topical composition disclosed herein (i.e. a topical composition comprising endoxifen free base, or a salt or a solvate thereof). In some embodiments disclosed herein are compositions according to the claims for use in methods of treating a tamoxifen-refractory subject having or at risk for having a hormone-sensitive breast disorder or hormone-sensitive reproductive tract disorder or both, the method comprising administration to the subject a topical composition disclosed herein (i.e. a topical composition comprising endoxifen free base, or a salt or a solvate thereof), wherein the subject has plasma endoxifen level of ≤30 nM, <25 nM, <20 nM, <15 nM, <10 nM, <5 nM or <1 nM. In certain embodiments, the topical composition comprising endoxifen is endoxifen gluconate, endoxifen HCl, or endoxifen citrate. In some embodiments, topical compositions comprising at least 40% (Z)-endoxifen free base w/w in endoxifen in the final composition are administered to the subject. In other embodiments, the topical compositions comprising 0.01% to 10% (Z)-endoxifen free base or a salt or a solvate thereof w/w in the final composition is administered to the subject. Such administration of a composition comprising endoxifen free base or a salt thereof maintains the plasma endoxifen in the subject at steady state levels ≤ 30 nM.

Also provided herein, are compositions according to the claims for use in methods of treating a tamoxifen-refractory subject, the method comprising: (a) determining or having determined plasma endoxifen level in a test sample obtained from the subject; (b) comparing or having compared or having determined the level of plasma endoxifen in the test sample with a reference plasma endoxifen level; (c) determining or having determined a reduced level of plasma endoxifen in the test sample as compared to the reference plasma endoxifen level; and (d) administering a composition comprising endoxifen free base or a salt thereof to the subject, wherein the composition is administered topically, transdermally, transpapillarily or intraductally. Such administration of a composition comprising endoxifen free base or a salt thereof maintains the plasma endoxifen in the subject at steady state levels ≤ 30 nM.

Provided herein are compositions according to the claims for use in methods of treating a subject having or at risk of having a hormonal dependent breast disorder or a hormonal dependent reproductive tract disorder, the method comprising: (a) administering to the subject a first composition comprising tamoxifen; (b) determining or having determined the level of plasma endoxifen in a test sample obtained from the subject; (c) determining or having determined if the plasma endoxifen in test sample is reduced as compared to a reference level of plasma endoxifen; and (d) administering a composition disclosed herein to the subject, wherein the composition is administered topically, transdermally, transpapillarily or intraductally. The subject may be administered with the first composition comprising tamoxifen daily for at least 1 day, 2 days, 3 days, 15 days, 1 week, 2 weeks, 4 weeks, 1 month, 2 months, 3 months, 4 months, 5 months, 6 months, 1 year, 2 years, 3 years, 4 years, 5 years or 10 years. In some embodiments, administration of the composition comprising endoxifen free base or a salt thereof maintains the subject's plasma endoxifen at steady state levels ≤ 30 nM, ≤ 15 nM, ≤10 nM, ≤5 nM and ≤1 nM. In some embodiments, the subjects are administered a composition comprising (Z)-endoxifen D-gluconate, (Z)-endoxifen L-gluconate, (E)-endoxifen D-gluconate, (E)-endoxifen L-gluconate, or a combination thereof, wherein the composition is administered topically, including transdermally, transpapillarily or intraductally. In other embodiments, the composition comprising endoxifen salt is endoxifen HCl or endoxifen citrate, wherein the composition is administered topically, transdermally, transpapillarily or intraductally.

Provided herein are compositions according to the claims for use in methods of treating a subject with a hormone-dependent breast disorder or hormone-dependent reproductive tract disorder, the method comprising: (a) dosing the subject with a first composition comprising tamoxifen; (b) determining or having determined the subject's tamoxifen-metabolites profile in a test sample obtained from the subject; (c) determining if the subject's plasma endoxifen is reduced based on the subject's tamoxifen-metabolites profile as compared to a level of reference plasma endoxifen in a reference tamoxifen-metabolites profile; and (d) administering a topical composition disclosed herein (i.e. a topical composition comprising comprising endoxifen free base or a salt or a solvate thereof) to the subject, wherein the composition is administered topically, transdermally, transpapillarily and/or intraductally. In some embodiments, the subject is administered a topical composition comprising 0.01% to 10% (Z)-endoxifen w/w with respect to the final composition as disclosed herein. In certain embodiments, the composition comprising endoxifen is endoxifen gluconate, endoxifen HCl, or endoxifen citrate, wherein the composition is administered topically, including transdermally, transpapillarily or intraductally.

In an aspect, the present disclosure contemplates compositions according to the claims for use in a method of treating a subject having or at risk of having a hormone-dependent breast disorder or a hormone-dependent reproductive tract disorder or both, the method comprising resection of breast tissue of the subject or administering radiotherapy to the subject and administering a topical composition comprising endoxifen or a salt thereof as disclosed herein, wherein the composition is administered topically, transdermally, transpapillarily and/or intraductally. In another aspect, the present disclosure embraces compositions according to the claims for use in a method of treating a subject having or at risk of having a hormone-dependent breast disorder or a hormone-dependent reproductive tract disorder or both, the method comprising administering a topical composition disclosed herein prior to resection of resection of breast tissue of the subject or administering radiotherapy to the subject, wherein the composition is administered topically, transdermally, transpapillarily or intraductally.

In an aspect of the present disclosure, a subject may be administered a topical composition disclosed herein during the treatment via more than one route or mode of delivery, such as topically and intraductally, topically and transpapillarily, transpapillarily and transdermally, etc.

Dosage to be administered to a subject will be usually in a unit dosage form. Examples of ranges for the at least one active agent disclosed herein, i.e. endoxifen free base and salts and solvates thereof, in each unit dosage form range from 0.01 mg to 200 mg per unit dose.

In some embodiments, the compositions comprising endoxifen free base and salts and solvates thereof are administered to subjects at a dose of 0.01 mg to 200.0 mg. In other embodiments, the topical, transdermal, transpapillary and intraductal compositions comprising endoxifen free base and endoxifen salts are administered to the subject at a dose of 1 mg to 200 mg. In some embodiments, the compositions comprising endoxifen free base and salts and solvates thereof are administered to the subject at a dose of 0.01 mg, 0.05 mg, 0.1 mg, 0.25 mg, 0.5 mg, 0.75 mg, 1.0 mg, 1.5 mg, 2.0 mg, 3 mg, 4.0 mg, 5 mg, 6 mg, 7 mg, 8 mg, 9 mg, 10 mg, 15 mg, 20 mg, 25 mg, 40 mg, 50, and 100, and 200 mg per breast.

In some embodiments, the compositions comprising endoxifen free base and salts and solvates thereof are administered to the subject a unit dose of 0.01 mg, 0.05 mg, 0.1 mg, 0.25 mg, 0.5 mg, 0.75 mg, 1 mg, 1.5 mg, 2 mg, 3 mg, 4 mg, 5 mg, 6 mg, 7 mg, 8 mg, 9 mg, 10 mg, 15 mg, 20 mg, 25 mg, 40 mg, 50 mg, 75 mg, 100 mg, and 200 mg.

In some embodiments, the compositions comprising (Z)-endoxifen free base and salts and solvates thereof are administered to the subject a unit dose of 0.01 mg, 0.05 mg, 0.1 mg, 0.25 mg, 0.5 mg, 0.75 mg, 1.0 mg, 1.5 mg, 2.0 mg, 3 mg, 4.0 mg, 5 mg, 6 mg, 7 mg, 8 mg, 9 mg, 10 mg, 15 mg, 20 mg, 25 mg, 40 mg, 50 mg, 75 mg, and 100 mg.

In certain embodiments, the compositions comprising at least 40% (Z)-endoxifen (w/w) of endoxifen are administered a unit dose of 1 mg, 2 mg, 3 mg, 4 mg, 5 mg, 6 mg, 7 mg, 8 mg, 9 mg, 10 mg, 15 mg, 20 mg, 25 mg, 40 mg, 50 mg, 75 mg, and 100 mg.

In some embodiments, the compositions comprising endoxifen salts or solvates thereof such as endoxifen gluconate are administered at a dose ranging from 0.01 to 200 mg. In some embodiments, a composition comprising (Z)-endoxifen D-gluconate or (Z)-endoxifen L-gluconate is administered 0.5 mg, 1 mg, 2 mg, 3 mg, 4.0 mg, 5 mg, 6 mg, 7 mg, 8 mg, 9 mg, 10 mg, 15 mg, 20 mg, 25 mg, 40 mg, 50, and 100 mg per unit dose. In other embodiments, a composition comprising 0.5 mg to 100 mg of (Z)-endoxifen D-gluconate is administered. In yet other embodiments, a composition comprising 0.5 mg to 100 mg of (Z)-endoxifen L-gluconate is administered. In still other embodiments, a composition comprising 0.5 mg to 200 mg of (Z)-endoxifen D-gluconate and (E)-endoxifen D-gluconate is administered. In further embodiments, a composition comprising 0.5 mg to 100 mg of (Z)-endoxifen D-gluconate and (Z)-endoxifen L-gluconate is administered.

It will be appreciated by one of ordinary skill in the art that the treatment regimen for treating a breast disorder or an estrogen-related disorder with a topical composition disclosed herein may depend on a variety of factors, including the type, age, weight, sex, diet, and the medical condition of the subject, and pharmacological considerations such as activity, efficacy, pharmacokinetic and toxicology profiles of the particular active agent employed. Thus, the treatment regimen actually employed may vary widely from subject to subject. The present disclosure is also not limited to doses described herein. One of skill in the art will recognize that the dosing regimens that are encompassed by the present disclosure include dosing the subject on a once a day, twice a day, thrice a day, weekly, biweekly, semimonthly, monthly, every 2 months, quarterly, every 6 months, and annual basis or any regimen a physician or healthcare professional may deem suitable. Doses may be administered in a single dose or split dose.

It is an aspect of the present disclosure that the compositions of the disclosure can be used alone or in combination with one or more therapeutic agents as part of primary therapy, neoadjuvant therapy, or an adjuvant therapy. Combinations of the compositions may act to improve the efficacy of the therapeutic agents, and therefore can be used to improve standard cancer therapies. For example, when a subject has prostate cancer and is on bicalutamide or enzalutamide therapy for the treatment of prostate cancer, the subject is likely to develop gynecomastia as a result of the therapy. The compositions disclosed herein can be administered to the subject having prostate cancer in order to prevent and/or treat gynecomastia.

As another example, a subject with ER+/Her2+ positive breast cancer would be on a combination therapy with trastuzumab or other oncology drugs such as anti-neoplastics, immunotherapy, and a composition disclosed herein can be used to treat such a subject with ER+/Her2+ positive breast cancer.

Accordingly, in some embodiments, the compositions further comprise bicalutamide, enzalutamide, abiraterone acetate, an oncology drug such as antineoplastics such as capecitabine (Xeloda), carboplatin (Paraplatin), cisplatin (Platinol), cyclophosphamide (Neosar), docetaxel (Docefrez, Taxotere), doxorubicin (Adriamycin), pegylated liposomal doxorubicin (Doxil), epirubicin (Ellence), fluorouracil (5-FU, Adrucil), gemcitabine (Gemzar), methotrexate (multiple brand names), paclitaxel (Taxol), protein-bound paclitaxel (Abraxane), vinorelbine (Navelbine), eribulin (Halaven), ixabepilone (Ixempra), goserelin acetate, trastuzumab, ado-trastuzumab, bevacizumab, everolimus, check point inhibitors (such as pembrolizumab (Keytruda^{™}), nivolumab (Opdivo^{™}), atezolizumab (Tecentriq^{™}), durvalumab (Imfinzi^{™}), and avelumab (Bavencio^{™}).

In another aspect, a composition disclosed herein may comprise therapeutic agents that increase bioavailability of endoxifen in a subject. P-glycoprotein (P-gp, ABCB1) is a highly efficient drug efflux pump expressed in brain, liver, and small intestine, but also in cancer cells, that affects pharmacokinetics and confers therapy resistance for many anticancer drugs. Accordingly, in some embodiments, the compositions further comprise inhibitors of ATP-binding cassette (ABC family) transporters, such as inhibitors of breast cancer resistance protein (BCRP protein) and P-gp. Several inhibitors of BCRP protein and P-Gp are known in the art. For example, inhibitors of BCRP protein include cyclosporine, omeprazole, pantoprazole, saquinavir, and tacrolimus.

Non-limiting examples of P-gp inhibitors include first generation inhibitors such as Verapamil, cyclosporin A, reserpine, quinidine, yohimbine, tamoxifen and toremifene, second generation inhibitors such as Dexverapamil, dexniguldipine, valspodar (PSC 833), and Dofequidar fumarate (MS-209), third generation P-gp inhibitors such as Cyclopropyldibenzosuberane zosuquidar (LY335979), laniquidar (R101933), mitotane (NSC-38721), biricodar (VX-710), elacridar (GF120918/GG918), ONT-093, tariquidar (XR9576), and HM30181 and anti-P-gp monoclonal antibodies such as MRK-16).

The present disclosure additionally provides for therapeutic kits containing one or more of the compositions for use in the treatment of a subject having or at risk of having a hormone-dependent breast disorder or a hormone-dependent reproductive tract disorder. The kits of the present disclosure include the compositions disclosed herein, a sealed container for housing the composition, and instructions for use of the composition. In certain embodiments, the contents of the kits can be lyophilized and the kit can additionally contain a suitable solvent for reconstitution of the lyophilized components. Individual components of the kit would be packaged in separate containers and associated with such containers would be a notice in the form prescribed by a governmental agency regulating the manufacture, use, or sale of the pharmaceutical products, which notice reflects the approval by the agency of manufacture for use or sale for subject's administration.

In some embodiments, the disclosure provides a dose, a unit dose, or multiple dose of the pharmaceutical dose package. In some embodiments, the packaging reflects a dosing regimen or schedule of application, such as twice daily, daily, weekly, twice weekly, biweekly, monthly, quarterly, 6 monthly, or yearly application. Advantageously, such packaging of the pharmaceutical composition facilitates accurate application of an amount of the composition such as a therapeutically effective amount and will include a single dose applicator or device or metered dose applicator or device.

When the components of the kit are provided in one or more liquid solutions or gels, the liquid solution can be a topical composition disclosed herein, for example, a sterile solution comprising at least one active agent (i.e. endoxifen free base and salts and solvates thereof), the first compound and the second compound according to the claims.

For intraductal administration, the compositions may be formulated into a pharmaceutically acceptable syringeable composition. The container means may itself be a syringe, a catheter, a microcatheter a probe suitable for intraductal administration. The container may also be a pipette, a dropper, an applicator, deformable tubes, vials, bottles, metered pumps or dispensers, sachets, blister packs, ampules, pouch, a blow-fill-seal tube, drum, jar, glass or plastic bottle with pumps for metered dosing, collapsible tube, aerosol spray or such like apparatus, from which the formulation may be applied to an affected area of the subject. The container may any size suitable for single unit dose or multiple unit dose. For example, a blow-fill-seal unit dose may be of size ranging from 0.5 to 20 ml. The container may be a single use container or multi-use container such as a bottle with pumps providing a metered dose. The kits of the present disclosure may include a device (such as an applicator for applying the composition to the surface of the skin of the breast or a syringe or catheter for intraductal administration of the composition) or a transdermal drug delivery system for administering the compositions (such as a patch, a tape, a bandage, etc.), and instructions for use of the device or transdermal drug delivery system.

Pharmaceutical kits or packs comprising one or more of the compositions disclosed herein in combination with one or more therapeutic agent for e.g. bicalutamide, enzalutamide, abiraterone acetate, an oncology drug such as antineoplastics such as capecitabine (Xeloda), carboplatin (Paraplatin), cisplatin (Platinol), cyclophosphamide (Neosar), docetaxel (Docefrez, Taxotere), doxorubicin (Adriamycin), pegylated liposomal doxorubicin (Doxil), epirubicin (Ellence), fluorouracil (5-FU, Adrucil), gemcitabine (Gemzar), methotrexate (multiple brand names), paclitaxel (Taxol), protein-bound paclitaxel (Abraxane), vinorelbine (Navelbine), eribulin (Halaven), ixabepilone (Ixempra), goserelin acetate, trastuzumab, ado-trastuzumab, bevacizumab, everolimus, check point inhibitors (such as pembrolizumab (Keytruda^{™}), nivolumab (Opdivo^{™}), atezolizumab (Tecentriq^{™}), durvalumab (Imfinzi^{™}), and avelumab (Bavencio^{™}) and inhibitors of ABC-binding cassette reporters such as BCRP inhibitors and P-gp inhibitors as disclosed herein for combination therapy are also contemplated by the present disclosure. Other therapeutic agents known in the art may also be included in such kits and packs.

In yet other embodiments, the present disclosure relates to compositions according to the claims for use in a method for treating a subject having or at risk of having a hormone-dependent breast disorder, a hormone-dependent reproductive tract disorder, or both, the method comprising administering a topical composition comprising: (a) 0.01% to 10% (w/w) (Z)-endoxifen free base or a salt or solvate thereof; (b) a first compound comprising diethyleneglycol monoethyl ether; and (c) a second compound; wherein the second compound comprises a penetration enhancer selected from the group consisting of DMSO, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid.

In yet other embodiments, the present disclosure relates to compositions according to the claims for use in a method for treating a subject having or at risk of having a hormone-dependent breast disorder, a hormone-dependent reproductive tract disorder, or both, the method comprising administering a topical composition comprising: (a) 0.01% to 10% (w/w) (Z)-endoxifen free base or a salt or solvate thereof; (b) a first compound comprising DMSO; and (c) a second compound; wherein the second compound comprises a penetration enhancer selected from the group consisting of diethyleneglycol monoethyl ether, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid.

In yet other embodiments, the present disclosure relates to compositions according to the claims for use in a method for treating a subject having or at risk of having a hormone-dependent breast disorder, a hormone-dependent reproductive tract disorder, or both, the method comprising administering a topical composition comprising:
0.01% to 10% (w/w) (Z)-endoxifen free base or salts or solvates thereof; a first compound comprising diethyleneglycol monoethyl ether; and a second compound comprising isopropanol; and mineral oil;
0.01% to 10% (w/w) (Z)-endoxifen free base or salts or solvates thereof; a first compound comprising diethyleneglycol monoethyl ether; and a second compound comprising isopropanol; and caprylic/capric glycerides;
0.01% to 10% (w/w) (Z)-endoxifen free base or salts or solvates thereof; a first compound comprising diethyleneglycol monoethyl ether; and a second compound comprising isopropanol; mineral oil and caprylic/capric glycerides;
0.01% to 10% (w/w) (Z)-endoxifen free base or salts or solvates thereof; a first compound comprising 15% to 45% (w/w) diethyleneglycol monoethyl ether; and a second compound comprising 5% to 30% (w/w) isopropanol; 25% to 45% (w/w) caprylic/capric trigycerides; and q.s. 100% (w/w) with mineral oil;
0.01% to 10% (w/w) of (Z)-endoxifen or a salt thereof; a first compound comprising 25% to 65% (w/w) DMSO; and a second compound comprising 10% to 30% (w/w) of diethyl sebacate; 5% to 20% (w/w) of dipropylene glycol; 5% to 20% (w/w) of diisopropyl adipate; and q.s 100% (w/w) of polyethylene glycols such as PEG300;
0.01% to 10% (w/w) of (Z)-endoxifen or a salt thereof; a first compound comprising 25% to 55% (w/w) diethylene glycol monoethyl ether; and a second compound comprising 5% to 20% (w/w) of isopropanol; 20% to 40% (w/w) of capric triglyceride; and q.s. 100% (w/w) soybean oil;
0.01% to 10% (w/w) of (Z)-endoxifen or a salt thereof; a first compound comprising 25% to 65% (w/w) diethylene glycol monoethyl ether; and a second compound comprising 10% to 30% (w/w) of diethyl sebacate; 5% to 20% (w/w) of diisopropyl adipate; and q.s 100% (w/w) of polyethylene glycols such as PEG300;
0.01% to 10% (w/w) of (Z)-endoxifen or a salt thereof; a first compound comprising 25% to 65% (w/w) DMSO; and a second compound comprising 5% to 15% (w/w) of dipropylene glycol; 5% to 20% (w/w) of isopropanol; 0.01% to 10% surfactant such as polyethylene glycol dodecyl ether (Brij L4), polysorbates such as polysorbate 20 (Tween 20), polysorbate 40, polysorbate 60, polysorbate 80, polysorbate 85, and the like; and q.s. 100% (w/w) polypropylene glycols such as PEG 300;
0.01% to 10% (w/w) of (Z)-endoxifen or a salt thereof; a first compound comprising15% to 45% (w/w) DMSO; and a second compound comprising15% to 45% (w/w) diethylene glycol monoethyl ether; 5% to 15% (w/w) of dipropylene glycol; 5% to 20% (w/w) of diisopropyl adipate; 15% to 40% (w/w) of diethyl sebacate; and q.s. 100% (w/w) mineral oil;
0.01% to 10% (w/w) of (Z)-endoxifen or a salt thereof; a first compound comprising 10% to 30% (w/w) DMSO; and a second compound comprising 20% to 60% (w/w) of caprylic/capric triglyceride; 5% to 20% (w/w) of isopropanol; and q.s. 100% (w/w) soybean oil;
0.01% to 10% (w/w) of (Z)-endoxifen or a salt thereof; a first compound comprising 10% to 40% (w/w) diethylene glycol monoethyl ether; and a second compound comprising 20% to 60% (w/w) of caprylic/capric triglyceride; 5% to 20% (w/w) of isopropanol; and q.s. 100% (w/w) mineral oil;
0.01% to 10% (w/w) of (Z)-endoxifen or a salt thereof; a first compound comprising10% to 40% (w/w) diethylene glycol monoethyl ether; a second compound comprising 20% to 60% (w/w) of caprylic/capric triglyceride; 5% to 20% (w/w) of isopropanol; 0.01% to 20% (w/w) cetyl alcohol; and q.s. 100% (w/w) mineral oil;
0.01% to 10% (w/w) of (Z)-endoxifen or a salt thereof; a first compound comprising 10% to 40% (w/w) diethylene glycol monoethyl ether; and a second compound comprising 20% to 60% (w/w) of caprylic/capric triglyceride; 5% to 20% (w/w) of isopropanol; 0.01% to 20% (w/w) cetyl alcohol; 0.01% to 20% (w/w) stearic acid; and q.s. 100% (w/w) mineral oil;
0.01% to 10% (w/w) of (Z)-endoxifen or a salt thereof; a first compound comprising10% to 40% (w/w) diethylene glycol monoethyl ether; and a second compound comprising 20% to 60% (w/w) of caprylic/capric triglyceride; 1% to 20% (w/w) of dimethyl sebacate; 5% to 20% (w/w) of isopropanol; and q.s. 100% (w/w) mineral oil;
a thickening agent, a penetration enhancer, an emollient, a surfactant, an antioxidant, an antimicrobial, a controlled-release agent, a skin care active, or a combination thereof;
a second therapeutic agent;
a second therapeutic agent selected from the group consisting of bicalutamide, enzalutamide, abiraterone acetate, an oncology drug such as antineoplastics such as capecitabine (Xeloda), carboplatin (Paraplatin), cisplatin (Platinol), cyclophosphamide (Neosar), docetaxel (Docefrez, Taxotere), doxorubicin (Adriamycin), pegylated liposomal doxorubicin (Doxil), epirubicin (Ellence), fluorouracil (5-FU, Adrucil), gemcitabine (Gemzar), methotrexate (multiple brand names), paclitaxel (Taxol), protein-bound paclitaxel (Abraxane), vinorelbine (Navelbine), eribulin (Halaven), ixabepilone (Ixempra), goserelin acetate, trastuzumab, ado-trastuzumab, bevacizumab, everolimus, check point inhibitors (such as pembrolizumab (Keytruda^{™}), nivolumab (Opdivo^{™}), atezolizumab (Tecentriq^{™}), durvalumab (Imfinzi^{™}), and avelumab (Bavencio^{™}) and inhibitors of ABC-binding cassette reporters such as BCRP inhibitors and P-gp inhibitors;
the topical composition has a water content of less than 1%; or wherein the composition has a dielectric constant of less than 50, or both;
the (Z)-endoxifen or a salt or a solvate thereof is stable at ambient temperature for at least 3 months, at least 6 months, at least 9 months, at least 12 months, at least 15 months, and at least 18 months;
the topical composition is stable at ambient temperature for at least 18 months.

Embodiments of the disclosure will be described with reference to the following Examples which are provided for illustrative purposes only and should not be used to limit the scope of or construe the disclosure.

### EXAMPLES

### REFERENCE EXAMPLE 1.

### Synthesis of (Z)-endoxifen and (E)/(Z)-endoxifen mixtures

### Step 1. Demethylation of [4-[2-dimethylamino)ethoxy]phenyl] (4-hydroxyphenyl)methanone

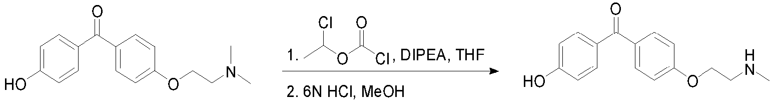

A suitable 10L reactor was charged with starting material [4-[2-(dimethylamino)ethoxy]phenyl](4-hydroxyphenyl)methanone, Compound of Formula (I) (0.5 Kg, 1.0 equiv.), DIPEA (1.5 Kg, 6.65 equiv., 3.0 wt./wt.) and Tetrahydrofuran (5 L, 10 vol./wt., 8.9 wt./wt.) under N₂ atmosphere. 1-Chloroethyl chloroformate (1.7 Kg, 6.65 equiv., 3.3 wt./wt.) was added slowly while maintaining the internal temperature at NMT 20°C. The mixture was heated to reflux and stirred at reflux for NLT 12 hr. The mixture was evaporated under reduced pressure at NMT 75°C until the volume reached the lowest agitateable volume. Methanol (2.5 L, 5 vol./wt., 4.0 wt./wt.) was added slowly and the mixture was distilled under reduced pressure at NMT 75°C until the volume reached the lowest agitateable volume. Methanol (2.5 L, 5 vol./wt., 4.0 wt./wt.) was added and the mixture was distilled under reduced pressure at NMT 75°C until the volume reached the lowest agitateable volume. Methanol (2.5 L, 5 vol./wt., 4.0 wt./wt.) was once again added and the mixture was further distilled under reduced pressure at NMT 75°C until the volume once again reached the lowest agitateable volume. Methanol (2 L, 4 vol./wt., 3.2 wt./wt.) and 6N HCl (2 L, 4 vol./wt., 4.0 wt./wt.) were added to the mixture and the mixture was heated to reflux. The mixture was stirred at reflux for NLT 12 hr. After reaction completion, the mixture was evaporated under reduced pressure at NMT 75°C until most of MeOH was removed. The mixture was cooled to 25±5°C and 8N NaOH (2.5 L, 5 vol./wt., 5.0 wt./wt.) was added slowly until the pH of the mixture was 11-12. The mixture was stirred at 0-5°C for NLT 2 hr. The mixture was filtered and washed with H₂O (1 L, 2 vol./wt.) and Ethyl acetate (1 L, 2 vol./wt., 1.8 wt./wt.). The wet cake was dried at NMT 50°C under reduced pressure to afford (4-hydroxyphenyl)(4-(2-(methylamino)ethoxy)phenyl)methanone, **Compound of Formula (II).** Yield: 297 gm, 63%; Purity: 100% *(Expected Yield - 60 - 90%)*

### Step 2. McMurry Reaction

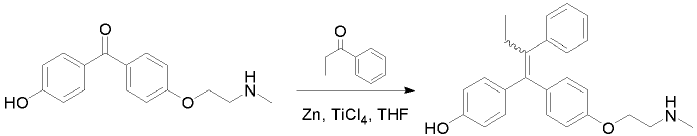

A suitable 10 L reactor was charged with Zn powder (0.27 Kg, 4.0 equiv., 0.9 wt./wt.) and Tetrahydrofuran (1.5 L, 5 vol./wt., 4.4 wt./wt.) under N2 atmosphere. TiCl4 (0.42 Kg, 2.0 equiv., 1.4 wt./wt.) was added slowly while maintaining the internal temperature at NMT 15°C. The reaction was heated to reflux and stirred at reflux for NLT 2 hr. A suspension of (4-hydroxyphenyl)(4-(2-(methylamino)ethoxy)phenyl)methanone, Compound of Formula (II) obtained from Step 1, (0.297 Kg, 1.0 equiv.) and propiophenone (0.21 Kg, 1.5 equiv., 0.7 wt./wt.) in Tetrahydrofuran (3.0 L, 10 vol./wt., 8.9 wt./wt.) was added and the reflux was continued for NLT 8 hr. The mixture was cooled to 20-30°C and added into 25% Ammonium chloride (5.9 L, 20 vol./wt., 20 wt./wt.)/silica (diatomaceous earth/silicon dioxide; tradename Celite^{®}S) available from Sigma Aldrich (0.3 Kg, 1.0 wt./wt.) mixture. The mixture was filtered and washed with Tetrahydrofuran (0.9 L, 3 vol./wt., 2.7 wt./wt.).

The mixture was settled for phase separation. The organic layer was collected and the aqueous layer was washed with Tetrahydrofuran (0.9 L, 3 vol./wt., 2.7 wt./wt.). The organic layer was again collected and combined with the first organic layer. The combined organic layer was washed with 40% K2CO3 (1.2 L, 4 vol./wt., 5.6 wt./wt.). The organic layer was concentrated at NMT 75°C under reduced pressure until the volume reached the lowest agitateable volume. Ethyl acetate (1.5 L, 5 vol./wt., 4.5 wt./wt.) was added and the mixture was distilled under reduced pressure at NMT 75°C until the volume reached the lowest agitateable volume. Ethyl acetate (1.5 L, 5 vol./wt., 4.5 wt./wt.) was added and the mixture was distilled under reduced pressure at NMT 75°C until the volume reached 5 vol. (1.5 L). The mixture was heated to dissolution and n-Heptane (3.0 L, 10 vol./wt., 6.8 wt./wt.) was added. The mixture was cooled to 0±5°C and stirred at 0±5°C for NLT 2 hr. The mixture was filtered and the residue was washed with Ethyl acetate /n-Heptane=1/2 (v/v, 1.5 L, 5 vol./wt., 3.7 wt./wt.). The residue wet cake was dried under reduced pressure at NMT 60°C to afford (E/Z)-4-[1-[4-[2-(Methylamino)ethoxy]phenyl]-2-phenyl-1-buten-1-yl]-phenol, a **mixture of (Z)-endoxifen and (E)-endoxifen, Compounds of Formula (III).** Yield: 176 g, 42%; Purity: 81.96%. E/Z ratio: 3.1:1 *(Expected yield - 40 - 60%).*

To a suitable 10 L reactor was charged with above (E/Z)-endoxifen (0.250 Kg, 1.0 wt.), isopropanol (1.25 L, 5 vol./wt., 3.9 wt./wt.) and Purified water (1.25 L, 5 vol./wt., 5.0 wt./wt.). The mixture was heated to 70±5°C and stirred at 70±5°C for NLT 2 hr. The mixture was cooled to 0±5°C and stirred at 0-5°C for NLT 2 hr. The mixture was filtered and washed with pre-cooled isopropanol/Purified water=1/1 (0.5 L, 2 vol./wt., 3.6 wt./wt.). The residue wet cake was dried at NMT 60°C to afford (E/Z)-4-[1-[4-[2-(Methylamino)ethoxy]phenyl]-2-phenyl-1-buten-1-yl]-phenol, a **mixture of (Z)-endoxifen and (E)-endoxifen, Compounds of Formula (III).** Yield: 105 g, 41%; Purity: 96.79%. E/Z ratio: **48.6:1** *(Expected yield - 40 - 60%).*

### Step 3. Enrichment and Purification of (Z)-endoxifen

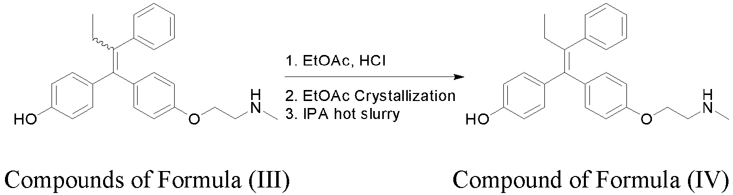

A suitable reactor was charged with the mixture of (Z)-endoxifen and (E)-endoxifen, Compounds of Formula III, (0.105 Kg, 1.0 wt.) and Ethyl acetate, (1.1 L, 10 vol./wt., 9.0 wt./wt.). The mixture was cooled to 0-5°C and 6N HCl (0.3 L, 3 vol./wt., 3.0 wt./wt.) was added slowly to the mixture. The mixture was heated to 60±5°C and stirred at 60±5°C for NLT 6 hr. The mixture was cooled to 0±5°C and 8N NaOH (0.3 L, 3.0 vol./wt., 3.0 wt./wt.) was added slowly until the pH of the mixture was ≧ 12.

The mixture was settled for phase separation, organic layer was collected and the aqueous layer was washed with Ethyl acetate (0.5 L, 5 vol./wt., 4.5 wt./wt.). The organic layer was collected. The combined organic layers were washed with 20% NaCl (0.3 L, 3 vol./wt., 3.0 wt./wt.). The organic layer was treated with activated carbon (charcoal) (0.005 Kg, 0.05 wt.) and stirred at 50±5°C for NLT 1 hr. The mixture was filtered through diatomeaceous earth/silica (Celite^{®}S) bed and washed with Ethyl acetate (0.5 L, 5 vol./wt., 4.5 wt./wt.). The filtrate was concentrated at NMT 75°C until the volume reached 10 vol. (1.1 L). The mixture was cooled to 0-5°C and stirred at 0-5°C for NLT 2 hr. The mixture was filtered and the filtrate was collected. The filtrate was concentrated at NMT 75°C until the volume reached lowest agitateable volume.

Isopropanol, (1.1 L, 10 vol./wt., 7.9 wt./wt.) was added and the mixture was concentrated at NMT 75°C until the volume reached lowest agitateable volume. isopropanol (1.1 L, 10 vol./wt., 7.9 wt./wt.) was again added and the mixture was concentrated at NMT 75°C until the volume reached lowest agitateable volume. isopropanol (1.1 L, 10 vol./wt., 7.9 wt./wt.) was added and the mixture was concentrated at NMT 75°C until the volume reached 5 vol. (0.5 L). The suspension was stirred at 50±5 °C for NLT 6 hr. The mixture was cooled to 20±5°C and stirred at 20±5°C for NLT 4 hr. The mixture was filtered and washed with isopropanol (0.2 L, 2 vol./wt., 1.6 wt./wt.). The wet cake was dried at NMT 60°C to afford **purified (Z-Endoxifen)** solid **Compound of Formula (IV)** as an off-white solid. Yield: 39.5 g, 36%, (E)/(Z) ratio: 1/12.2; Purity (Z): 89.59% by HPLC. (*Expected Yield - 20-40%*)

Next, a suitable 1 L reactor was charged with above Z-Endoxifen (39 g) solid Compound of Formula (IV) and isopropanol (390 mL, 10 vol./wt.). The suspension was stirred at 50±5°C for NLT 6 hr. The mixture was cooled to 20±5°C and stirred at 20±5°C for NLT 4 hr. The mixture was filtered and washed with isopropanol (78 mL, 2 vol./wt.). The wet cake was dried at NMT 60°C to afford **purified (Z-Endoxifen)** solid **Compound of Formula (IV)** as an off-white solid. Yield: 25 g, 61%, (E)/(Z) ratio: 1/55.1; Purity (Z): ***95.81%;** (Expected Yield - 50 - 70%).*

Endoxifen free base prepared by the procedure above is essentially free of endoxifen salts. Impurities were less than 1%.

### REFERENCE EXAMPLE 2

### Relative Solubility of Endoxifen Free Base

(Z)-endoxifen free base was synthesized as described above in **Example 1.** (Z)-endoxifen was solubilized in various solvents as provided in **Table 1,** and tested for physical and chemical stability in solution. The solutions were prepared in ~1 ml batches typically for solubility and stability testing. An excess amount of endoxifen was added into solvents listed in Table 1. The mixture was stirred overnight on a rotisserie at room temperature. The next, day, the suspension was filtered using a syringe filter (0.2 um pore size GHP membrane). The filtrated solution was then analyzed for the concentration of (Z)-endoxifen and (E)-endoxifen as using a validated HPLC-UV method as described below.

**Table 1. Solubility of (Z) and (E)-endoxifen in polar organic solvents**

| | | | | **Solubility Limit** | | |
|---|---|---|---|---|---|---|
| **Solvent No.** | **Solvents/Carriers** | **Z/E Ratio** | **Z:E (wt%/wt%)** | **(E)-endoxifen free base** | **(Z)-endoxifen free base** | **Total Endoxifen free base** |
| 1 | Dimethyl Sulfoxide | 1.87 | 65:35 | ++ | ++++ | +++++ |
| 2 | Diethyl Sebacate | 1.7 | 63:37 | + | + | ++ |
| 3 | Diisopropyl Adipate | 1.87 | 65:35 | + | + | + |
| 4 | Dimethyl Isosorbide | 1.82 | 65:35 | + | ++ | +++ |
| 5 | Dipropylene Glycol | 1.82 | 65:35 | + | + | ++ |
| 6 | Ethanol | 1.72 | 63:37 | ++ | ++++ | +++++ |
| 7 | Isopropanol | 1.88 | 65:35 | + | + | + |
| 8 | PEG300 | 1.77 | 64:36 | + | + | ++ |
| 9 | Propylene Glycol | 1.60 | 62:38 | + | + | + |
| 10 | Diethylene glycol monoethyl ether | 1.89 | 65:35 | ++ | +++ | ++++ |
| 11 | PEG 400 | 1.78 | 64:36 | + | + | + |
| 12 | Isopropyl Myristate | 1.09 | 52:48 | + | + | + |
| 13 | Soybean Oil | 0.76 | 43:57 | + | + | + |
| 14 | Oleic Acid | 1.02 | 50:50 | + | + | ++ |
| 15 | Capric Trigylceride | 0.63 | 38:62 | + | + | + |

HPLC-UV. Briefly, (E)- and (Z)-endoxifen were analyzed by High Performance Liquid Chromatography, HPLC (instrument: Agilent 1100) using a UV detector (Agilent). Chromatographic resolution was obtained using a Luna Phenyl Hexyl Column (3.0 um x 4.6 um x 150 mm) at a column temperature of 40°C. Samples were injected in an injection volume of 10 ul and run at a flow rate of 1.0 ml/min. The mobile phase system used consisted of: A) water with 10 mM ammonium formate (HCOONH4) with 0.03% formic acid and B) Methanol with 10 mM ammonium formate (HCOONH4). The mobile phase was filtered through a ZapCap CR 0.2µm membrane filter and run at a gradient as shown in **Table 2.**

**Table 2. Gradient**

| Time (minutes) | %B |
|---|---|
| 0 | 50 |
| 15 | 70 |
| 19 | 85 |

UV detection of (E)-endoxifen and (Z)-endoxifen isomers was performed at 243 nm with a run time of 30 minutes. Retention times for (E)-endoxifen and (Z)-endoxifen were 15.14 m and 16.25 m respectively. Results show that (Z)-endoxifen is soluble in the solvents studied above. The solubility of total endoxifen ranged from about 2 mg/g of solution to over 50 mg/g of solution. The solubility of (Z)-endoxifen ranged from about 1 mg/g of solution to over 30 mg/g of solution.

Endoxifen was significantly soluble in polar organic solvents/MPEs such as DMSO, ethanol, diethylene monoethyl ether, and oleic acid. The (Z)-endoxifen is known to be rapidly converted to its (E)-isomer in solution. Surprisingly however, the above data shows that (Z)-isomer conversion to its inactive form (E)-isomer is lower than expected per published data by Elkins et al., and the ratio of (Z)-isomer to (E)-isomer (Z:E) in the solvents ranged from 87:13 to 38:62 in each sample as shown above in **Table 1.** The Z/E ratio ranged from 0.63 to 1.89. (Z)-endoxifen is surprisingly stable in the above disclosed polar organic solvents. The polar solvents of **Table 1** are also penetration enhancers/MPEs suitable for multiplexing for preparation of stable topical compositions.

### REFERENCE EXAMPLE 3

### Stability of (Z)-Endoxifen Free Base in Polar Organic Solvents

The stability studies of endoxifen free base in various pure polar organic solvents/MPEs were performed as described below. Accelerated stability studies of (Z)-endoxifen free base in solvents/MPEs were performed over a course of 2 days at 45°C in both the presence and the absence of human cadaver skin to assess the degree to which the (Z)-endoxifen conversion to (E)-endoxifen by isomerization and or chemical degradation of the endoxifen is accelerated upon contact with skin and skin components. (Z)-Endoxifen solutions at ~80% of saturation concentrations were prepared in 1 ml batches in solvents as listed below in the **Table 3.** The potencies of (Z)-endoxifen and (E)-endoxifen were determined by HPLC-UV analysis as described above. Potency results are shown compared with potencies measured when the sample were stored at the initiation of stability study.

**Table 3 - Stability of endoxifen free base in polar organic solvents in the absence and presence of skin**

| **Solvent** | **Condition** | **%Potency (E)-endoxifen** | **%Potency (Z)-endoxifen** |
|---|---|---|---|
| Dimethyl Sulfoxide | 45°C | 101 | 102 |
| | 45°C + skin | 101 | 101 |
| Diethyl Sebacate | 45°C | 101 | 100 |
| | 45°C + skin | 99 | 94 |
| Diisopropyl Adipate | 45°C | 100 | 99 |
| | 45°C + skin | 101 | 99 |
| Dimethyl Isosorbide | 45°C | 15 | 16 |
| | 45°C + skin | 16 | 17 |
| Dipropylene Glycol | 45°C | 95 | 93 |
| | 45°C + skin | 95 | 94 |
| Isopropanol | 45°C | 106 | 105 |
| | 45°C + skin | 106 | 105 |
| PEG 300 | 45°C | 91 | 91 |
| | 45°C + skin | 92 | 92 |
| Diethylene glycol monoethyl ether | 45°C | 100 | 100 |
| | 45°C + skin | 98 | 98 |
| Isopropyl Myristate | 45°C | 67 | 65 |
| | 45°C + skin | 68 | 63 |

Results show that both (E)- and (Z)-endoxifen free base maintained potency and stability in the solvents in the presence of skin as compared with potency in absence of skin after 2 days. Results of stability studies with polar organic solvents/MPEs such as DMSO, dimethyl sebacate, diisopropyl adipate, isopropanol, and diethylene glycol monoethyl ether show that they are useful for the preparation of surprisingly chemically and physically stable topical formulations comprising endoxifen. Unexpectedly, endoxifen dissolved in dimethyl isosorbide and isopropyl myristate showed poor stability. Formulations of the compositions disclosed herein can thus include one or more of the polar organic solvents/MPEs of this **Table 3.**

### EXAMPLE 4

### Preparation of Topical Endoxifen Free Base Formulations

(Z)-endoxifen free base was synthesized as described above in **Example 1.** Topical formulations for delivering (Z)-endoxifen to the skin were prepared and tested for physical and chemical stability. The formulations were typically prepared in ~1 ml batches. The applicants have discovered that surprisingly advantageous combinations of the following components can be used in the preparation of stable topical compositions of the present disclosure. All the ingredients such as endoxifen (with a Z/E ratio of 1.8:1), diethylene glycol monoethyl ether (transcutol^{®}), capric triglyceride, isopropanol, mineral oil, polyethylene glycol dodecyl ether (Brij L4), PEG 400, diethyl sebacate etc. as shown in **Table 4,** were mixed together in a glass vial and the mixtures were stirred vigorously overnight to prepare clear homogenous solution. Endoxifen free base was not enclosed in lipid-complex in the compositions.

**Table 4 - Endoxifen Free Base Solution Formulations**

| Sample No. | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Ingredient (wt%/wt%) | | | | | | | | | | | | | | |
| Endoxifen | 1 | 1 | 1 | 1 | 1 | 1 | 1 | 1 | 1 | 1 | 1 | 1 | 1 | 1 |
| Diethyl sebacate | 20 | | 20 | | 20 | 20 | | | | | | | | 10 |
| Dimethyl sulfoxide | 39 | | | 45 | 30 | | 20 | | | | | | | |
| Diisopropyl adipate | 10 | | 10 | | 9 | 10 | | | | | | | | |
| Dipropylene glycol | 10 | | | 10 | 10 | | | | | | | | | |
| PEG 300 | 20 | | 29 | 29 | | | | | | | | | | |
| Diethylene glycol monoethyl ether | | 40 | 40 | | 30 | 19 | | 19 | | 19 | 30 | 29 | 19 | 19 |
| Capric triglyceride | | 30 | | | | 40 | 40 | 40 | 40 | | 30 | 30 | 40 | 40 |
| Soybean Oil | | 19 | | | | | 29 | | | | | | | |
| Isopropanol | | 10 | | 10 | | 10 | 10 | 10 | 10 | 10 | 10 | 10 | 10 | 10 |
| Brij L4 | | | | 5 | | | | | | | | | | |
| Mineral Oil | | | | | | | | 30 | | 40 | 25 | 20 | 20 | 10 |
| Dimethyl isosorbide | | | | | | | | | 19 | 19 | | | | |
| Cedar Leaf Oil | | | | | | | | | | 30 | | | | |
| Cetyl Alcohol | | | | | | | | | | | 4 | 5 | | |
| Stearic Acid | | | | | | | | | | | | 5 | | |

Formulation with endoxifen free base prepared by the procedure above is essentially free of endoxifen salts. Impurities were less than 1%.

### EXAMPLE 5

### Stability of (Z)-Endoxifen Free Base Formulations

For the formulation stability studies, the formulations Samples 1 to 10 **(Table 1)** were first prepared in 1 ml batches. A 10-day accelerated stability study was performed. A sample aliquot (~20 ul) from the formulations was taken and analyzed for initial (Day 0) concentrations of (Z)-endoxifen and (E)-endoxifen in the samples by HPLC-UV. The remaining amounts of formulations were then stored in glass vials at 40°C under dry nitrogen (inert) for 10 days. After incubation for 10 days, 20 ul aliquots of the formulations were collected in triplicates and analyzed for (Z)-endoxifen and (E)-endoxifen concentrations by HPLC-UV and the data is shown in **Table 5.** Certain sample formulations were stored for longer periods, and additional aliquots of these formulations were collected at 5 to 8 weeks. Samples were analyzed in triplicates for (Z)-endoxifen and (E)-endoxifen potencies by HPLC-UV and the data is shown in **Table 6.** The potencies of each of (E)-endoxifen and (Z)-endoxifen are shown as percentage potency on samples relative to potencies in the same samples on Day 0. The results shown are average of triplicate measurements.

**Table 5. Accelerated 10 Day Stabilities of endoxifen isoforms relative to Day 0**

| Sample ID. | Av. % wt E | Av. % wt Z | (Z):(E) % | Z/E ratio | % Potency E | % Potency Z |
|---|---|---|---|---|---|---|
| 1 | 0.338 | 0.619 | 64:36 | 1.831 | 97.7 | 97.1 |
| 2 | 0.368 | 0.665 | 64:36 | 1.804 | 97.2 | 96.9 |
| 3 | 0.335 | 0.593 | 64:36 | 1.772 | 93.6 | 90.8 |
| 4 | 0.331 | 0.592 | 64:36 | 1.790 | 93.0 | 90.2 |
| 5 | 0.362 | 0.638 | 64:36 | 1.763 | 99.2 | 95.3 |
| 6 | 0.368 | 0.651 | 64:36 | 1.771 | 99.0 | 95.3 |
| 7 | 0.316 | 0.543 | 63:37 | 1.717 | 83.5 | 80.6 |
| 8 | 0.371 | 0.635 | 63:37 | 1.713 | 98.2 | 98.0 |
| 9 | 0.080 | 0.143 | 64:36 | 1.791 | 23.9 | 24.4 |
| 10 | 0.089 | 0.157 | 64:36 | 1.768 | 26.6 | 27.1 |

**Table 6. Stabilities of Endoxifen Topical Formulations After 7-15 Weeks Exposure**

| Sample ID. | Stability Period (weeks) | Av. % wt E | Av. % wt Z | Z/E ratio | (Z):(E) %wt:%wt | % Potency E | % Potency Z |
|---|---|---|---|---|---|---|---|
| 1 | 8 | 0.076 | 0.128 | 1.701 | 63:37 | 70.8 | 65.4 |
| 5 | 8 | 0.077 | 0.115 | 1.498 | 60:40 | 81.5 | 66.2 |
| 6 | 7 | 0.368 | 0.611 | 1.659 | 62:38 | 101.0 | 91.3 |
| 8 | 12.5 | 0.364 | 0.636 | 1.750 | 64:36 | 96.4 | 95.9 |
| 8 | 15 | 0.364 | 0.617 | 1.694 | 62:36 | 96.5 | 93.5 |

Results show that topical formulations of the disclosure are surprising stable at 10 days and at 7 to 15 weeks storage at 40°C under inert conditions. In contrast to collective expectations based on literature, interconversion of (Z)-endoxifen to (E)-endoxifen is surprising low as shown by the studies herein (Elkins et al. provide t₉₀ of 149 days at room temperature and t₉₀ of 9 days at 45°C and observed degradation of (Z)-endoxifen HCl in aqueous medium from 98% to 75% at 45°C on day 15).

Except for Samples 9 and 10, the relative potencies of (Z)-endoxifen in the topical formulations ranged from 80% to 98% and (Z):(E) ratio remained above 60:40 after 10 days. Samples 6 and 8 remained at 88.5% to 91.3% and (Z):(E) ratio remained above 60:40 even after 7 to 15 weeks when stored at 40°C. These accelerated stability studies are predictive of long term (~18 months) ambient temperature stability.

### EXAMPLE 6

### Skin Permeation Studies

Franz diffusion cell experiments were used to analyze the flux rates of varying endoxifen topical formulations across a substrate membrane. Franz diffusion cells are a common and well known method for measuring transdermal flux rates (Lee et al., Breast Cancer (Dove Med Press) 2011, 3:61-70; Mah et al. Lee et al. Cancer Chemother Pharmacol 2015, 76:1235-1246; Int J Pharm 2013, 441:433-440). The general Franz cell procedure is described in Franz, T. J., Percutaneous absorption: on the relevance of in vitro data. J Invest Derm, 64:190-195 (1975). The following was the methodology used in the present Example.

Skin delivery and permeation studies on topical formulations disclosed herein and a control using human cadaver skin in Franz Diffusion Cells (FDCs) were performed. Skin from a single donor was shipped and stored frozen at -20°C until needed. In order to conduct permeation studies, the skin was removed from the freezer, allowed to equilibrate to room temperature, and then cut to ~2 cm x 2 cm pieces before testing. FDCs with a 3.3 ml receiver volume and 0.55 cm² diffusional were employed. Receptor wells were filled with receptor fluid (1x PBS buffer, pH7.4 with wt% 0.01% NaN3 4% hydroxypropyl beta-cyclodextrin (HBCD). Pieces of skin were mounted on the receptor cells. Next, the donor wells were applied to the receptor wells and the assembly was clamped together with uniform pressure using a pinch clamp. After assembly of the FDC, the skin was allowed to hydrate for 20 minutes in contact with the receptor fluid. Any FDC that evidenced any leakage during this period was discarded.

The integrity and quality of the skin was tested prior to application of the test formulations through measurement of the transdermal flux of tritiated water. Skin pieces evidencing an excessively high tritiated water flux were discarded and the tritiated water fluxes of accepted skin pieces were used to guide the distribution of formulation samples over the skin piece set. After removal of the tritiated water samples from the donor wells, the clamp and donor wells were removed. The skin was tapped dry with a KimWipe and the receptor well solutions were replenished with fresh receptor well medium.

Six (6) replicates of each formulation (Samples 1, 2, 5, 6 and 8 from **Table 4**) were examined in a batch of 36 FDCs. Each test formulation was applied at a finite dose of 5µL/0.55 cm⁻² (corresponding to some 9 mg cm⁻²) on skin maintained at 32°C throughout the experiments. The fluids in the receptor wells were stirred with a magnetic stir bar throughout.

A sample aliquot was abstracted from each receptor well at each of 4h, 8h, 24h and 48h, the receptor well replenished with fresh Receptor Fluid. At 48 hours, the skin was removed and washed with a water/ethanol solution and the skin tapped dry with a Kimwipe to remove residual formulation. The washed skin was then tapestripped three times, namely, cellophane tape was applied to the skin with uniform pressure and peeled off to systematically remove the outermost layers of the stratum corneum. These tape strips were then discarded. The remaining skin was split into epidermal and dermal compartments using a pair of spatulas. The endoxifen in the epidermal and dermal compartments of each skin sample was extracted by placing the skin pieces into a glass vial, adding 3 ml of DMSO to the vial and incubating at 40°C for 24 hours. At 24 hours, a 20 ul sample aliquot of DMSO was collected. The concentrations of API ((Z)-endoxifen) and of (E)-endoxifen in each receptor well sample (reflecting the amount of endoxifen diffusing through the skin into the wells below) and the DMSO sample from epidermis and dermis were assayed by the HPLC analytical method described above.

Results from skin permeation studies using FDC show that topical formulations of the present disclosure are able to cross the skin barrier in a time dependent manner accumulating increasingly over the 2-day period. Epidermis and dermis also show the accumulation of the formulation over the 2-day period **(****Figure 2A, 2B****,** **2C and 2D****).**

### EXAMPLE 7

### In Vitro Release Testing

Topical and transdermal formulations of (Z)-endoxifen. Examples of extended-release dosage forms include controlled-release, sustained-release, and long-acting drug products. Samples will be tested by USP 725: Topical And Transdermal Drug Products-Product Performance Tests methodology. Topical and transdermal formulation may be extended release formulations, including for example, Sample Nos 5 and 8 from **Table 4** can be tested for extended release properties by using Transdermal Reciprocating disk (Apparatus 7) at Stroke depth: 2-3 cm; 30-60 cycles per minute in Distilled Water in 25 × 150 mm test-tubes containing 20 mL for 1, 2, 4, 6, 12, 18, 24, 36, 48, and 72 hours. Transdermal formulations may be tested for extended release properties also by using Paddle over Disk (Apparatus 5) at 50 RPM in 0.1 M Phosphate buffer, monobasic, pH 5 at 32°Cin 500 mL for 1, 2, 4, 8, 12, 16, 20 and 24 hours or using Rotating Cylinder (Apparatus 6) at 50 rpm in 0.1 M Phosphate buffer, monobasic, pH 5 at 32°C in a 1000 mL volume for 1, 2, 4, 8, 12, 16, 20 and 24 hours.

### REFERENCE EXAMPLE 8

### Preparation of (E/Z)-endoxifen Salt

(E/Z)-Endoxifen D-gluconate salt is prepared by mixing an ethanolic slurry of (E/Z)-endoxifen free base mixture with an aqueous solution of D-gluconic acid, followed by hydrolyzing a 20% w/v solution of D-gluconolactone in water and heating at 70°C for 15 to 30 min. Minimum volume of ethanol is used and 5 ml of the aqueous D-gluconic acid solution is adder per 1 g of endoxifen free base. Stirring is then continued until a clear solution is obtained. The required volume of this solution is then added to other excipient to produce the gel formulations in which "active ingredient" is endoxifen D-gluconate salt.

**Table 8 - (E/Z)-endoxifen D-gluconate Gel**

| **Ingredient** | **Percentage (%)** |
|---|---|
| E/Z-endoxifen D-gluconate | 0.01 to 10 |
| HPMC | 5 |
| Dow Corning Q7-9180 silicone fluid | q.s. 100 |

### EXAMPLE 9

### Placebo-Controlled Study of Topical Endoxifen

Healthy female subjects, ages 18 to 60, were randomized into 3 cohorts of 8 subjects. Six subjects in each cohort received a daily dose of 2 mg (1 mg/breast), 6 mg (3 mg/breast), or 10 mg (5 mg/breast) topical endoxifen and 2 subjects received placebo. Dosing was based on the amount of (Z)-endoxifen in the topical endoxifen comprising E/Z-endoxifen mixture. Subjects were engaged in the study for a period of 63 days (including 28 day screening, 28 day on-study and a 7 day post treatment period). Following the 28 day screening period, the subjects were dosed daily on days 1 to 28. On day 14, a safety data review was conducted. Additional post-treatment safety review was conducted after termination on day 28, on days 29, 31, and 33 and end of study visit on day 35.

Each morning, the skin on both breasts of the subject was cleaned, dried and ensured that it is not broken. Topical endoxifen was provided to the subject in 2 pre-sealed sachets. Each sachet was opened individually. The content of a single sachet was applied on one breast. Sachets were cut or torn at the notches indicated near the top edge of the sachet. Starting with the left breast the contents of the sachet was applied to the top of the breast ensuring the entire contents is used. Using one or both hands topical endoxifen was rubbed gently by the participant on to the left breast area. Topical endoxifen was applied evenly and rubbed into the breast continually for 3 minutes or until the topical endoxifen was thoroughly absorbed. The process was then repeated for the right breast. Consecutive doses were administered 24 hrs (±2hrs) apart.

On day 1 of the study dosing, prior to dosing with either placebo or topical endoxifen vital signs (systolic/diastolic blood pressure, pulse, temperature, respiratory rate) and hot flashes occurrence before dosing and at the following time points: 1, 2, 4, 8 and 12 hours following dose administration were checked. A 12-lead ECG before dosing and at 4 hours following administration of the first dose were performed. Blood samples for biomarker and pharmacokinetic (PK) analysis were collected 10 ±1 minutes prior to dose administration to establish the baseline.

On days 4, 7, 14, 21 and the end of the study, vital signs (systolic/diastolic blood pressure, pulse, temperature, respiratory rate) and hot flashes occurrence were checked. ECG, within 60 minutes prior to dose administration were also be performed, hematology, serum chemistry, coagulation and urinalysis and blood samples for PK analysis were collected 10 ±1 minutes prior to administration of the study drug.

Safety endpoints were summarized by dose cohorts, with placebos pooled across cohorts. Treatment-Emergent Adverse Events (TEAEs) were coded using the latest version of MedDRA by System Organ Class (SOC) and Preferred Term, classified from verbatim terms. The incidence and frequency of TEAEs, and Serious Adverse Effects (SAEs), were summarized by cohort according to SOC and Preferred Terms, and by severity and relationship. The duration of AEs was determined and included in listings, along with the action taken and outcome. Vital signs, ECG and safety laboratory parameters were summarized at each scheduled time point using descriptive statistics. Post-dose assessments were compared with baseline measurements. The incidence of laboratory abnormalities was summarized. Physical examination findings were presented in listings.

Mean and individual (Z)-Endoxifen serum concentration-time curves were tabulated for each dose cohort. Pharmacokinetic parameters, was determined for each participant and summarized by cohort using descriptive statistics (arithmetic means, standard deviations, coefficients of variation, sample size, minimum, maximum and median). In addition, geometric means was calculated for AUC and Cmax. Analyses using linear models was performed to assess dose proportionality, time dependence and accumulation, and attainment of steady state (multiple dose).

### Results.

*Pharmacokinetics - Plasma Endoxifen Levels.* Plasma endoxifen levels of the subjects was determined by validated tandem LC-MS/MS tamoxifen metabolite assay as a measure of topical endoxifen permeation into the breast tissue. The sensitivity of endoxifen analyte detection by tandem LC-MS/MS tamoxifen metabolite assay was 0.15 ng/mL. An analysis was conducted to determine if there was a dose dependent relationship with plasma endoxifen. The total plasma endoxifen from all 12 samples was determined in each subject in each dosage group. A total of 310 samples were analyzed.

71% (165) of the total 232 blood samples from the active drug subjects had detectable endoxifen levels. >80% had plasma endoxifen levels of ≤ 1 ng/mL (2.68 nM). Approximately 56% samples from subjects dosed with active endoxifen showed plasma endoxifen levels ranging from >0.93 ng/mL to 5 ng/mL (13.4 nM). Results suggests a dose dependent increase in plasma endoxifen levels in the various dosage groups *(See* **Figure 3**) proving the topical endoxifen permeates into the breast tissue and achieves optimal systemic exposure at reliably low but detectable levels.

*Serious Adverse Effects (SAEs).* There were no SAEs in any subject at any time during the 672 Subject-Days of the study. There were no dose limiting toxicities or side effects observed.

*Treatment-Related Side Effects.* When asked if the subject was bothered by side effects of treatment on FACT-ES Validated Questionnaire appropriately modified to reflect this study during their weekly visit, 96% of subjects responded "not-at all," and 3% subjects responded "a little bit" and 1% subjects responded "somewhat." There were no reports of "quite a bit" or "very much." There was no dose-relationship with responses.

*Tolerability.* Treatment-related adverse events observed were generally mild and self-limiting. Seven study subjects (1 placebo and 2 subjects from each cohort) reported having hot flashes (33% subjects dosed with topical endoxifen as compared with the literature reported 46% subjects who experience hot flashes when taking oral tamoxifen). Further, four of the seven subjects reporting hot flashes had them at baseline before being given the test drug. These four subjects reported 18 of the 21 reports of hot flashes during the study. Only three subjects who reported no hot flashes pre-dosing reported one episode each of hot flashes during the study and no subject reported more than one hot flash that was treatment emergent. No discernable relationship was noted between plasma endoxifen levels and the incidence and/or severity of hot flashes, or the dose level to which the participant was assigned.

*Coagulation Parameters.* Rare but serious oral tamoxifen toxicities include strokes and pulmonary emboli. For this study, International Normalized Ratio (INR) and activated partial thromboplastin time (aPTT) were measured at baseline and during the study. There were no clinically significant changes in either parameter in any subject in the study.

*Serum Chemistry Parameters.* Oral tamoxifen is associated with elevated liver function tests, especially SGOT/AST, in about 5% of patients. In this study, 23 serum chemistry parameters were measured at baseline and then at four intervals during the study in all 24 subjects. An analysis of all 2,760 tests showed no change in a clinically significant manner.

*Hematology Parameters.* 11 hematology parameters were measured at baseline and at four intervals during the study in all 24 subjects. An analysis of all 1,320 test scores showed no change in a clinically significant manner.

*Urinalysis.* 10 urinalysis parameters were measured at baseline and then at four intervals during the study in all 24 subjects. An analysis of all 1,200 tests showed no change in a clinically significant manner.

*Vital signs.* Vital signs were measured at baseline and then 22-times during the study in all 24 subjects. An analysis of all vital sign measurements showed no change in a clinically significant manner in any of the subjects in the study.

*Electrocardiograms (ECG).* In this study, ECGs were measured at baseline and then 9-times during the study in all 24 subjects. An analysis of all ECGs showed none were changed in a clinically significant manner.

*Local Tolerance (Self-Assessment).* Greater than 97% subjects indicate reports no redness, burning, itching, irritation and pain. 3360 assessments were performed. 1.8% mild and 0.2% reported experience of redness, burning, itching, irritation and pain.

**Table 9. Summary of Treatment Emergent Adverse Events of at Least Moderate Intensity and Related to Study Treatment**

| | Topical Endoxifen | | | | | |
|---|---|---|---|---|---|---|
| System Organ Class Preferred Term | Cohort 1 2 mg (n=6) | Cohort 2 6 mg (n=6) | Cohort 3 10 mg (n=6) | All Actives (n=18) | Placebo 0 mg (n=7) | All Subjects (n=25) |
| Subjects with at least 1 TEAE No. of TEAEs | | 1 (17%) (3) | | 1 (6%) (3) | 1 (14%) (1) | 2 (8%) (4) |
| Nervous System Disorders | | 1 (17%) (2) | | 1 (6%) (2) | 1 (14%) (1) | 2 (8%) (3) |
| Headaches | | 1 (17%) (2) | | 1 (6%) (2) | 1 (14%) (1) | 2 (8%) (3) |
| Gastrointestinal Disorders | | 1 (17%) (1) | | 1 (6%) (1) | | 1 (4%) (1) |
| Nausea | | 1 (17%) (1) | | 1 (6%) (1) | | 1 (4%) (1) |

Results suggest that topical endoxifen successfully permeates across subjects' skin barrier to enter breast tissue while keeping the systemic exposure to the drug low (≤ 30 nM). Topical endoxifen does not produce clinically significant changes in coagulation, serum chemistry, hematology, and urinalysis parameters, vital signs, ECGs. Topical endoxifen is better tolerated by subjects treated with topical endoxifen at all doses and is likely to have low potential for patient compliance failure as compared with oral tamoxifen.

### EXAMPLE 10

### Double-blinded, Placebo Controlled, Dose Escalation Study of Topical Endoxifen in Males

Healthy male subjects, ages 18 to 65 with BMI ranging from 18 to 32 and having no acute or chronic disease, were randomized into 3 cohorts of 8 subjects. Six subjects in each cohort received a daily dose of 2 mg (1 mg/breast), 6 mg (3 mg/breast), or 10 mg (5 mg/breast) topical endoxifen and 2 subjects received placebo. Dosing was based on the amount of (Z)-endoxifen in the topical endoxifen comprising E/Z-endoxifen mixture. Subjects were engaged in the study for a period of 63 days (including 28-day screening; 28-days of study drug administration; and a 7-day post treatment period). Following the 28-day screening period, the subjects self-administered daily doses of the study drug on days 1 to 28. On day 14, a safety data review was conducted. Additional post-treatment safety review was conducted after termination on day 28, on days 29, 31, and 33 and end of study visit on day 35.

Each morning, the skin on both breasts of the subject was cleaned, dried and ensured that it is not broken. The study drug (topical endoxifen or placebo) was supplied as a single dose sachet per breast pre-sealed sachets. Each sachet was opened individually at the time of use. The content of a single sachet was applied on one breast. Sachets were cut or torn at the notches indicated near the top edge of the sachet. Starting with the left breast the contents of the sachet was applied to the top of the breast ensuring the entire contents is used. Using one or both hands topical endoxifen was rubbed gently by the participant on to the left breast area. The study drug was applied evenly and rubbed into the breast continually for 3 minutes or until it was thoroughly absorbed. The process was then repeated for the right breast. Consecutive doses were administered 24 hours (±2hrs) apart.

On day 1 of the study dosing, and prior to dosing with either the study drug (placebo or topical endoxifen), vital signs (systolic/diastolic blood pressure, pulse, temperature, respiratory rate) and the occurrence of vasoinotor symptoms (e.g., hot flashes, etc.) were obtained and also at the following time points: 1, 2, 4, 8 and 12 hours following dose administration. A 12-lead ECG before dosing and at 4 hours following administration of the first dose was performed. Blood samples for biomarker and pharmacokinetic (PK) analysis were collected 10 ± 1 minutes prior to dose administration to establish the baseline.

On days 4, 7, 14, 21 and the end of the study, vital signs (systolic/diastolic blood pressure, pulse, temperature, respiratory rate) and the occurrence of vasomotor symptoms (e.g., hot flashes, etc.) were obtained. ECG, within 60 minutes prior to dose administration was also performed, as well as hematology, serum chemistry, coagulation and urinalysis and blood samples for PK analysis were collected 10 ±1 minutes prior to administration of the study drug.

Safety endpoints were summarized by dose cohorts, with placebos pooled across cohorts. Treatment-Emergent Adverse Events (TEAEs) were coded using the latest version of MedDRA by System Organ Class (SOC) and Preferred Term, classified from verbatim terms. The incidence and frequency of TEAEs, and Serious Adverse Effects (SAEs), were summarized by cohort according to SOC and Preferred Terms, and by severity and relationship. The duration of AEs was determined and included in listings, along with any action taken, and outcome. Vital signs, ECG and safety laboratory parameters were summarized at each scheduled time point using descriptive statistics. Post-dose assessments were compared with baseline measurements. The incidence of laboratory abnormalities was summarized. Physical examination findings were presented in listings.

Mean and individual Endoxifen serum concentration-time curves were tabulated for each dose cohort. Pharmacokinetic parameters, was determined for each participant and summarized by cohort using descriptive statistics (arithmetic means, standard deviations, coefficients of variation, sample size, minimum, maximum and median). In addition, geometric means was calculated for AUC and Cmax. Analyses using linear models was performed to assess dose proportionality, time dependence and accumulation, and attainment of steady state (multiple dose).

### Results.

*Safety Signals.* No clinically significant safety signals observed in weekly assessments of blood chemistry, coagulation parameters, hematology parameters, urinalysis, vital signs, heart, and physical examinations.

*Serious Adverse Events*/*Treatment-emergent Adverse Events.* There were no Serious Adverse Events or clinically significant treatment-emergent Adverse Events related to study drug

*Tolerance.* A daily self-assessment of local tolerance was performed for 24 subjects for 28 days for a total of 672 daily assessments. Each participant was interviewed every seven days for side-effect information. Redness, burning, pain, itching and irritation were assessed each day. Subject were scored as None, Mild, Moderate or Severe for all five parameters. Greater than 97% scored None in the tolerability score and 1.8% scored mild in the tolerability score.

*Pharmacokinetics - Serum Endoxifen Levels.* Serum endoxifen levels of the subjects were determined by validated tandem LC-MS/MS tamoxifen metabolite assay as a measure of systemic endoxifen distribution from the breast tissue. The sensitivity of endoxifen analyte detection by tandem LC-MS/MS tamoxifen metabolite assay was 0.15 ng/mL. No discernable levels of topical endoxifen were detected in the serum of the subjects.

Based on the results obtained thus far, topical endoxifen appears to be well tolerated at each dose level and for the dosing duration utilized in the study with no detectable levels of topical endoxifen in the blood stream, no clinically significant safety signals and no clinically significant adverse events in the subjects receiving topical endoxifen.

### EXAMPLE 11

### Therapeutic Treatment of Tamoxifen-Refractory Breast Cancer by Topical Administration of Z-Endoxifen Free Base

Objective of the study is to demonstrate that stable and therapeutic (Z)-endoxifen levels can be achieved in tamoxifen-refractory patients by supplementing tamoxifen with (Z)-endoxifen. Plasma endoxifen levels will be used as a surrogate endpoint to predict clinical benefit as well as the rate of recurrence in this population and to confirm the safety and tolerability of (Z)-endoxifen administration when compared with tamoxifen.

Generally healthy subjects with early stage estrogen receptor positive breast cancer who are on adjuvant therapy of tamoxifen for at least 30 days will be enrolled for the study for up to 6 months. At least seventy-five tamoxifen-refractory breast cancer subjects will be enrolled in the study to ensure an evaluable population consists of at least 25 tamoxifen and 25 (Z)-endoxifen subjects is achieved. Additional subjects may be enrolled to achieve the desired population of tamoxifen-refractory subjects. These subjects will have been diagnosed with breast cancer and undergone mastectomy or lumpectomy.

After at least 30 days of oral tamoxifen, endoxifen plasma levels will be measured. If the endoxifen level is below 30 or 40 nM, then 1 mg of topical (Z)-endoxifen will be added to the oral tamoxifen adjuvant regimen. Additional (Z)-endoxifen doses will be added until a stable endoxifen of ≤ 30 nM is achieved. The endpoint is to establish a therapeutically stable endoxifen level in the tamoxifen+endoxifen group for at least 6 months.

### EXAMPLE 12

### Pre-surgical Treatment of Estrogen Receptor Positive Breast Cancer by Topical Administration of Z-Endoxifen-D-gluconate

The objective is to determine if topical Z-Endoxifen-D-gluconate reduces tumor activity in pre-surgical estrogen receptor positive breast cancer patients. Upon the initial diagnosis of ER+ breast cancer, 8 patients will be assigned to one of 3 groups, where each patient will receive 1, 2, or 5 mg of topical (Z)-endoxifen-D-gluconate per breast for 21 days prior to surgery.

The biomarker KI-67 levels of the tumor will be compared from the time of initial biopsy and the surgical sample will be compared to determine if one of the 3 doses results in a decrease in tumor activity.

### EXAMPLE 13

### Therapeutic Treatment of Breast Cancer by Topical Administration of (Z)-Endoxifen D-Gluconate

Objective of the study is to demonstrate that therapeutic (Z)-endoxifen levels in the subject's plasma can be achieved in tamoxifen-refractory patients initiating adjuvant breast cancer therapy. Plasma endoxifen levels will be used as a surrogate endpoint to predict clinical benefit as well as the rate of recurrence in this population and to confirm the safety and tolerability of oral (Z)-endoxifen D-gluconate administration when compared with tamoxifen.

Generally healthy subjects with estrogen receptor positive breast cancer for whom tamoxifen is indicated as part of their complete treatment regimen will be enrolled for the study for up to 6 months. At least seventy five (75) tamoxifen-refractory breast cancer subjects will be enrolled in the study to ensure an evaluable population consists of at least 25 tamoxifen and 25 (Z)-endoxifen subjects is achieved. Additional subjects may be enrolled to achieve the desired population of tamoxifen-refractory subjects. These subjects will have been diagnosed with breast cancer and undergone mastectomy or lumpectomy.

Immediately after mastectomy or lumpectomy, 25 subjects will be assigned to the tamoxifen group and administered daily with oral 20 mg Tamoxifen for a period of 3 months. Additional 25 subjects will be assigned to the (Z)-endoxifen group and administered daily with oral 10 mg tablet of (Z)-endoxifen D-gluconate for a period of 3 months. Blood will be drawn from each subject at day 0 (baseline), and on days 7, 14, 21, 28, 60 and 90. Plasma (Z)-endoxifen levels, hematology, chemistry, and coagulation parameters will be taken for both treatment sets on day 0 (baseline measurement), as wells as on days 7, 14, 21, 28, 60 and 90.

The plasma (Z)-endoxifen levels of the two treatment sets will be compared to each other. If the plasma endoxifen levels of the tamoxifen treated subjects are less than 30 nM, then those subjects will be transferred to the (Z)-endoxifen therapy group and the treatment will continue for additional 6 months. If the plasma endoxifen levels of the tamoxifen treated subjects are above 30 nM, they will continue to be treated with tamoxifen. Bloods samples will continue to be drawn weekly until the end of the study to monitor the subjects' plasma (Z)-endoxifen levels, hematology, chemistry, and coagulation parameters. Recurrence of cancer in the subjects will be monitored and compared between the treatment groups. Safety and efficacy will be evaluated every 3 months until the end of the study.

### EXAMPLE 14

### A Double-blinded, Placebo-controlled, Efficacy, Tolerability and Safety Study of Topical Endoxifen to Reduce Mammographic Breast Density

The objective of the study is to determine if topical endoxifen administration for up to twelve (12) months reduces mammographic breast density compared to a placebo. Two hundred and forty (240) healthy women of age 40 to 74 with a history of measurable mammographic breast density classified as BIRADS B, C and D and having ≥ 4.5 % density (volumetric) as measured by Volpara mammography will participate in this study.

The patient population will be randomly assigned into 2 groups (1:1). Placebo Control (Group 1) will include 120 subjects receiving topical placebo (0 mg topical endoxifen, i.e., no actives). Group 2 population will include 120 subjects receiving 10 mg topical endoxifen once daily (5 mg/breast/day). Superiority of topical endoxifen to the placebo and safety and efficacy of the subjects dosed with 10 mg topical endoxifen will be tracked as compared to the Group 1 population (Placebo Control). Each morning topical endoxifen at doses of 0 mg (Placebo) and 10 mg per day topical endoxifen will be applied to clean healthy intact skin of each of the breasts of subjects as described above. Treatment period will be 12 m. Topical endoxifen compositions that will be used for this study are disclosed herein. Topical endoxifen formulations listed in Tables 1 and 4 are useful for this study. Topical endoxifen will be packaged in sachets. At the time of dosing, the subject can tear open the sachet and apply the topical endoxifen directly to the skin of a breast.

Breast density will be evaluated by Volpara mammography at 6 and 12 m. Subjects may be evaluated by mammography for additional 12 months to determine the durability of mammographic density change.

The safety profile and tolerability of each dose group will be determined by clinical assessment, subject reports and active follow-up throughout the study period. All adverse events, regardless of severity, will be recorded and evaluated.

Breast density in the two groups will be compared (Group 1 vs Group 2). The density for women in the placebo control arm is only affected by the natural reduction in density that typically comes with increased age. Efficacy will be determined by change in mammographic density from baseline to 6 and 12 months and from 6 to 12 months of topical endoxifen administration. Comparison between topical endoxifen treatment and control arm is done using a Wilcoxon test (Alpha=0.05 two sided).

### EXAMPLE 15

### A Double-blinded, Randomized, Placebo-Controlled, Study of Topical Endoxifen for Gynecomastia in Prostate Cancer Patients

It is the objective of the study to determine if topical endoxifen administration for up to six months can prevent the onset of, or mitigate gynecomastia, as determined by breast ultrasound and the number of breast events. Adult men with prostate cancer diagnosis and initiating bicalutamide as part of androgen therapy will be included for the study of topical endoxifen safety and efficacy in preventing or mitigating gynecomastia. At least 100 subjects will be randomly chosen and enrolled in the trial to ensure a statistically evaluable population of at least 75 subjects.

Patient population initiating and receiving bicalutamide (50 mg capsules administered orally once daily) will be divided into 2 dose groups. Placebo Control (Group 1) will include 25 subjects receiving topical placebo (0 mg topical endoxifen, i.e., no actives) in addition to the 50 mg of bicalutamide orally, Group 2 population will include 50 subjects receiving 10 mg topical endoxifen once daily in addition to 50 mg bicalutamide orally. Superiority of topical endoxifen to the placebo and safety and efficacy of the subjects dosed with 10 mg topical endoxifen will be tracked as compared to the Group 1 population (Placebo Control).

Each morning topical endoxifen at doses of 0 mg (Placebo) and 10 mg per day topical endoxifen will be applied to clean healthy intact skin of each of the breasts of subjects as described above. Subjects will also be administered bicalutamide orally. Placebo Control subjects (Group 1) will be administered bicalutamide orally in the morning and they will not receive any active topical endoxifen but will receive a placebo. Treatment period will be 6 m.

Topical endoxifen compositions that will be used for this study are disclosed herein. Topical endoxifen formulations listed in Tables 1 and 4 are useful for this study. Topical endoxifen will be packaged in sachets. At the time of dosing, the subject can tear open the sachet and apply the topical endoxifen directly to the skin of a breast.

Gynecomastia in the subjects will be detected by breast ultrasound. Gynecomastia may also be graded using caliper measurements. Severity of gynecomastia will be scored on the basis of the largest diameter as follows: grade 1: 2 cm; grade 2: 2 to 4 cm; grade 3: 4 to 6 cm; and grade 4: > 6 cm. Assessment will be performed on day 0 prior to initiation of the topical endoxifen treatment regimen (baseline), and at 3, 6, 9 and 12 m for gynecomastia, breast pain, chemistry and coagulation parameters, and endoxifen levels. Monthly breast pain scores (none, slight moderate, and severe) will be used to determine if topical endoxifen will reduce breast pain the subjects as compared to Placebo Control. Safety will be determined by comparing any adverse blood safety signal difference. The safety and tolerability of topical endoxifen will be evaluated in subjects (Group 1 vs Group 2). Side effects such as local irritation and inflammation will be monitored. Quality of Life (QoL) will be assessed using a validated questionnaire, including specific questions relative to sexual interest and functioning. Questionnaires will be administered at baseline and at 3-month intervals during participation

Time spent without gynecomastia will be calculated with the Kaplan-Meier method and curves compared using the log-rank test (2 df P value; 1 df P value for pairwise comparisons) Group 1 vs Group 2.

It is intended that percentages (%) refer to amounts by weight based upon the total weight of the final composition (w/w) or (wt%/wt%). However, "endoxifen comprising at least 40%" (w/w) (Z)-endoxifen or salts or solvates thereof," "endoxifen comprising at least 60%" (w/w) (Z)-endoxifen or salts or solvates thereof, and the like refer to the percent weight of (Z)-endoxifen isomer as compared to the total weight of endoxifen in the final composition, whereas "Composition comprising 0.01% to 10% (Z)-endoxifen (w/w) refer to percent weight of (Z)-endoxifen isomer in a composition. The sum of the different components of the final composition adds up to 100% (w/w) of the total composition.

It is to be understood that compositions comprising endoxifen free base and salts thereof disclosed herein may be prepared with synthetically prepared endoxifen as well as isolated endoxifen. It is to be further understood that dosing of the subjects is based on amount of (Z)-endoxifen present in the composition.

## Claims

1. A topical composition comprising:
a. endoxifen or a salt or a solvate thereof;
b. a first compound; and
c. a second compound; and
wherein the first compound comprises:
i. diethyleneglycol monoethyl ether, and the second compound comprises a penetration enhancer selected from the group consisting of dimethyl sulfoxide (DMSO), diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof; or
ii. DMSO and the second compound comprises a penetration enhancer selected from the group consisting of diethyleneglycol monoethyl ether, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof.

2. The topical composition of claim 1, wherein:
a. the total concentration of the first compound and the second compound is up to about 95%, up to about 90%, up to about 85%, up to about 80%, up to about 75%, up to about 70%, up to about 65%, up to about 60%, up to about 55%, up to about 50%, up to about 45%, up to about 40%, up to about 35%, up to about 30%, up to about 25%, up to about 20%, up to about 15%, up to about 10%, or up to about 5% w/w of the topical composition;
b. the total concentration of the first compound and the second compound ranges from 10% to 90% w/w of the composition;
c. the total concentration of the first compound ranges from 10% to 90% (w/w) of the topical composition; and the total concentration of the second compound ranges from 5% to 80% (w/w) of the topical composition; and/or
d. the ratio of the first compound to second compound:
(i) ranges from 1:9 to 9:1;
(ii) ranges from 1:4 to 4:1;
(iii) ranges from 1:2 to 2:1; and/or
(iv) is about 1:1.

3. The topical composition of claim 1 or claim 2, wherein the endoxifen or a salt or a solvate thereof comprises: (a) at least 40% (Z)-endoxifen free base (w/w) with respect to total endoxifen in the final composition; or (b) (Z)-endoxifen and (E)-endoxifen at a Z:E ratio ranging from 99:1 to 1:99, from 90:10 to 10:90, from 85:15 to 15:85, from 80:20 to 20:80, from 75:25 to 25:75, from 70:30 to 30:70, from 65:35 to 35:65, from 60:40 to 40:60, from 55:45 to 45:55 and about 50:50.

4. The topical composition of any of the preceding claims, wherein the topical composition comprises:
a. 0.01% to 20% (w/w) of endoxifen free base or a salt or a solvate thereof; and/or
b. 0.01% to 10% (w/w) of (Z)-endoxifen free base or a salt or a solvate thereof.

5. The topical composition of any of the preceding claims, wherein the endoxifen salt is endoxifen gluconate, endoxifen HCl, or endoxifen citrate, or a solvate thereof.

6. The topical composition of any of the preceding claims, comprising:
(i) 0.01% to 10% (w/w) of (Z)-endoxifen free base or salts or solvates thereof;
(ii)
a. 0.01% to 10% (w/w) of (Z)-endoxifen free base or salts or solvates thereof;
b. a first compound comprising 10% to 90% (w/w) diethyleneglycol monoethyl ether;
c. a second compound comprising 5% to 80% (w/w) a penetration enhancer selected from the group consisting of DMSO, diethyl sebacate, diisopropryl adipate, dipropylene glycol, polyethylene glycols, isopropanol, t-butanol, polyethylene glycol dodecyl ether, cetyl alcohol, mineral oil, caprylic triglyceride, capric triglyceride, caprylic/capric triglycerides, and stearic acid, or a combination thereof;
(iii)
a. 0.01% to 10% (w/w) (Z)-endoxifen free base or salts or solvates thereof;
b. a first compound comprising diethyleneglycol monoethyl ether;
c. a second compound comprising isopropanol and mineral oil;
(iv)
a. 0.01% to 10% (w/w) of (Z)-endoxifen free base or salts or solvates thereof;
b. a first compound comprising diethyleneglycol monoethyl ether;
c. a second compound comprising isopropanol and caprylic/capric glycerides;
(v)
a. 0.01% to 10% (w/w) of (Z)-endoxifen free base or salts or solvates thereof;
b. a first compound comprising 10% to 90% (w/w) diethyleneglycol monoethyl ether;
c. a second compound comprising 5% to 30% (w/w) isopropanol; 10% to 80% (w/w) caprylic/capric trigycerides or mineral oil or both;
(vi)
a. 0.01% to 10% (w/w) of (Z)-endoxifen free base or salts or solvates thereof;
b. a first compound comprising 10% to 90% (w/w) diethyleneglycol monoethyl ether;
c. a second compound comprising 5% to 30% (w/w) isopropanol; 25% to 45% (w/w) caprylic/capric trigycerides; and q.s. 100% (w/w) with mineral oil; and/or
(vii)
a. 0.01% to 10% (w/w) of (Z)-endoxifen free base or salts or solvates thereof;
b. a first compound comprising 15% to 45% (w/w) diethyleneglycol monoethyl ether;
c. a second compound comprising 5% to 15% (w/w) isopropanol; 25% to 45% (w/w) caprylic/capric trigycerides; and q.s. 100% (w/w) with mineral oil.

7. The topical composition according to claim 1, wherein the topical composition comprises:
(i) 1% (w/w) endoxifen, 20% (w/w) diethyl sebacate, 39% (w/w) dimethyl sulfoxide, 10% (w/w) diisopropyl adipate, 10% (w/w) dipropylene glycol and 20% (w/w) PEG 300;
(ii) 1% (w/w) endoxifen, 40% (w/w) diethylene glycol monoethyl ether, 30% (w/w) capric triglyceride, 19% (w/w) soybean oil and 10% (w/w) isopropanol;
(iii) 1% (w/w) endoxifen, 20% (w/w) diethyl sebacate, 10% (w/w) diisopropyl adipate, 29% (w/w) PEG 300 and 40% (w/w) diethylene glycol monoethyl ether;
(iv) 1% (w/w) endoxifen, 45% (w/w) dimethyl sulfoxide, 10% (w/w) dipropylene glycol, 29% (w/w) PEG 300, 10% (w/w) isopropanol and 5% (w/w) polyethylene glycol dodecyl ether (Brij^{™} L4);
(v) 1% (w/w) endoxifen, 20% (w/w) diethyl sebacate, 30% (w/w) dimethyl sulfoxide, 9% (w/w) diisopropyl adipate, 10% (w/w) dipropylene glycol and 30% (w/w) diethylene glycol monoethyl ether;
(vi) 1% (w/w) endoxifen, 20% (w/w) diethyl sebacate, 10% (w/w) diisopropyl adipate, 19% (w/w) diethylene glycol monoethyl ether, 40% (w/w) capric triglyceride and 10% (w/w) isopropanol;
(vii) 1% (w/w) endoxifen, 20% (w/w) dimethyl sulfoxide, 40% (w/w) capric triglyceride, 29% (w/w) soybean oil and 10% (w/w) isopropanol;
(viii) 1% (w/w) endoxifen, 19% (w/w) diethylene glycol monoethyl ether, 40% (w/w) capric triglyceride, 10% (w/w) isopropanol and 30% (w/w) mineral oil;
(ix) 1% (w/w) endoxifen, 30% (w/w) diethylene glycol monoethyl ether, 30% (w/w) capric triglyceride, 10% (w/w) isopropanol, 25% (w/w) mineral oil and 4% (w/w) cetyl alcohol;
(x) 1% (w/w) endoxifen, 29% (w/w) diethylene glycol monoethyl ether, 30% (w/w) capric triglyceride, 10% (w/w) isopropanol, 20% (w/w) mineral oil, 5% (w/w) cetyl alcohol and 5% (w/w) stearic acid;
(xi) 1% (w/w) endoxifen, 19% (w/w) diethylene glycol monoethyl ether, 40% (w/w) capric triglyceride, 10% (w/w) isopropanol and 20% (w/w) mineral oil; or
(xii) 1% (w/w) endoxifen, 10% (w/w) diethyl sebacate, 19% (w/w) diethylene glycol monoethyl ether, 40% (w/w) capric triglyceride, 10% (w/w) isopropanol and 10% (w/w) mineral oil.

8. The topical composition of any of the preceding claims, further comprising:
a. a pharmaceutically acceptable excipient;
b. a thickening agent, a penetration enhancer, an emollient, a surfactant, an antioxidant, an antimicrobial, a skin care active, a controlled-release agent, or a combination thereof; and/or
c. a second therapeutic agent; optionally wherein the second therapeutic agent selected from the group consisting of bicalutamide, enzalutamide, abiraterone acetate, an oncology drug such as antineoplastics such as capecitabine (Xeloda), carboplatin (Paraplatin), cisplatin (Platinol), cyclophosphamide (Neosar), docetaxel (Docefrez, Taxotere), doxorubicin (Adriamycin), pegylated liposomal doxorubicin (Doxil), epirubicin (Ellence), fluorouracil (5-FU, Adrucil), gemcitabine (Gemzar), methotrexate (multiple brand names), paclitaxel (Taxol), protein-bound paclitaxel (Abraxane), vinorelbine (Navelbine), eribulin (Halaven), ixabepilone (Ixempra), goserelin acetate, trastuzumab, ado-trastuzumab, bevacizumab, everolimus, check point inhibitors (such as pembrolizumab (Keytruda^{™}), nivolumab (Opdivo^{™}), atezolizumab (Tecentriq^{™}), durvalumab (Imfinzi^{™}), and avelumab (Bavencio^{™}), and inhibitors of ABC-binding cassette reporters such as BCRP inhibitors and P-gp inhibitors.

9. The topical composition of any of the preceding claims, wherein the topical composition:
a. has a water content of less than 1% or wherein the composition has a dielectric constant of less than 50 or both; and/or
b. is stable at ambient temperature for at least 18 months.

10. The topical composition of any of the preceding claims, wherein the topical composition comprising endoxifen or a salt or a solvate thereof is formulated at a unit dose of endoxifen or a salt or a solvate thereof of 0.01 mg, 0.05 mg, 0.1 mg, 0.25 mg, 0.5 mg, 0.75 mg, 1 mg, 1.5 mg, 2 mg, 3 mg, 4 mg, 5 mg, 6 mg, 7 mg, 8 mg, 9 mg, 10 mg, 15 mg, 20 mg, 25 mg, 50 mg, 75 mg, 100 mg, and 200 mg.

11. The topical composition of any of claims 1 to 10 for use in a method of treating a subject having or at risk of having a hormone-dependent breast disorder, a hormone-dependent reproductive tract disorder, or both.

12. The topical composition for use according to claim 11, wherein:
a. the hormone-dependent breast disorder or the hormone-dependent reproductive tract disorder is a benign breast disorder, hyperplasia, atypia, atypical ductal hyperplasia, atypical lobular hyperplasia, increased breast density, gynecomastia, McCune-Albright Syndrome, precocious puberty, DCIS, LCIS, breast cancer, endometrial cancer, ovarian cancer, uterine cancer, cervical cancer, vaginal cancer, or vulvar cancer;
b. the subject has prostate cancer; and wherein the subject has initiated or is about to initiate chemotherapy;
c. the subject having or at risk of having the hormone-dependent breast disorder or hormone-dependent reproductive tract disorder is tamoxifen-refractory or tamoxifen-resistant; and/or
d. the subject displays a low plasma endoxifen level based on subject's tamoxifen-metabolites profile as compared to a level of plasma endoxifen in a reference tamoxifen-metabolites profile following prior administration of a composition comprising tamoxifen.

13. The topical composition for use according to claim 11 or claim 12, wherein:
a. the topical composition comprises endoxifen or a salt or a solvate thereof at a unit dose ranging from 0.01 mg to 200 mg;
b. the topical composition comprises (Z)-endoxifen or a salt or a solvate thereof at:
(i) a unit dose ranging from 0.01 mg to 100 mg;
(ii) a dose of 2.0 mg, 6 mg, or 10 mg;
(iii) a dose of 1.0 mg, 3.0 mg, or 5 mg per breast;
c. the topical composition is administered once a day, twice a day, thrice a day, four times a day, every other day, twice a week, weekly, fortnightly, twice a month, monthly, quarterly, once every six months, or annually; and/or
d. the method comprises administering the topical composition to maintain the subject's plasma endoxifen at a steady state level of ≤ 30 nM.

14. The topical compositions for use according to any of claims 11 to 13, wherein the method comprises administering the topical composition either alone or in combination with a second therapeutic agent; optionally wherein the second therapeutic agent is selected from the group consisting of bicalutamide, enzalutamide, abiraterone acetate, an oncology drug such as antineoplastics such as capecitabine (Xeloda), carboplatin (Paraplatin), cisplatin (Platinol), cyclophosphamide (Neosar), docetaxel (Docefrez, Taxotere), doxorubicin (Adriamycin), pegylated liposomal doxorubicin (Doxil), epirubicin (Ellence), fluorouracil (5-FU, Adrucil), gemcitabine (Gemzar), methotrexate (multiple brand names), paclitaxel (Taxol), protein-bound paclitaxel (Abraxane), vinorelbine (Navelbine), eribulin (Halaven), ixabepilone (Ixempra), goserelin acetate, trastuzumab, ado-trastuzumab, bevacizumab, everolimus, check point inhibitors (such as pembrolizumab (Keytruda^{™}), nivolumab (Opdivo^{™}), atezolizumab (Tecentriq^{™}), durvalumab (Imfinzi^{™}), avelumab (Bavencio^{™}), and inhibitors of ABC-binding cassette reporters such as BCRP inhibitors and P-gp inhibitors.

## Patentansprüche

1. Eine topische Zusammensetzung, die Folgendes umfasst:
a. Endoxifen oder ein Salz oder ein Solvat davon;
b. eine erste Verbindung; und
c. eine zweite Verbindung; und
wobei die erste Verbindung Folgendes umfasst:
i. Diethylenglycolmonoethylether, und die zweite Verbindung einen Penetrationsverbesserer umfasst, der aus der Gruppe ausgewählt ist, die aus Dimethylsulfoxid (DMSO), Diethylsebacat, Diisopropyladipat, Dipropylenglycol, Polyethylenglycolen, Isopropanol, t-Butanol, Polyethylenglycoldodecylether, Cetylalkohol, Mineralöl, Caprylsäuretriglycerid, Caprinsäuretriglycerid, Capryl-/Caprinsäuretriglyceriden und Stearinsäure oder einer Kombination davon besteht; oder
ii. DMSO, und die zweite Verbindung einen Penetrationsverbesserer umfasst, der aus der Gruppe ausgewählt ist, die aus Diethylenglycolmonoethylether, Diethylsebacat, Diisopropryladipat, Dipropylenglycol, Polyethylenglycolen, Isopropanol, t-Butanol, Polyethylenglycoldodecylether, Cetylalkohol, Mineralöl, Caprylsäuretryiglycerid, Caprinsäuretriglycerid, Capryl-/Caprinsäuretriglyceriden und Stearinsäure oder einer Kombination davon besteht.

2. Topische Zusammensetzung nach Anspruch 1, wobei:
a. die Gesamtkonzentration der ersten Verbindung und der zweiten Verbindung bis zu etwa 95%, bis zu etwa 90%, bis zu etwa 85%, bis zu etwa 80%, bis zu etwa 75%, bis zu etwa 70%, bis zu etwa 65%, bis zu etwa 60%, bis zu etwa 55%, bis zu etwa 50%, bis zu etwa 45%, bis zu etwa 40%, bis zu etwa 35%, bis zu etwa 30%, bis zu etwa 25%, bis zu etwa 20%, bis zu etwa 15%, bis zu etwa 10% oder bis zu etwa 5% Gew./Gew. der topischen Zusammensetzung beträgt;
b. die Gesamtkonzentration der ersten Verbindung und der zweiten Verbindung im Bereich von 10% bis 90% Gew./Gew. der Zusammensetzung liegt;
c. die Gesamtkonzentration der ersten Verbindung im Bereich von 10% bis 90% (Gew./Gew.) der topischen Zusammensetzung liegt; und die Gesamtkonzentration der zweiten Verbindung im Bereich von 5% bis 80% (Gew./Gew.) der topischen Zusammensetzung liegt; und/oder
d. das Verhältnis der ersten Verbindung zu der zweiten Verbindung:
(i) im Bereich von 1:9 bis 9:1 liegt;
(ii) im Bereich von 1:4 bis 4:1 liegt;
(iii) im Bereich von 1:2 bis 2:1 liegt; und/oder
(iv) etwa 1:1 beträgt.

3. Topische Zusammensetzung nach Anspruch 1 oder Anspruch 2, wobei das Endoxifen oder ein Salz oder ein Solvat davon Folgendes umfasst: (a) zu mindestens 40% (Z)-Endoxifen freie Base (Gew./Gew.), bezogen auf das gesamte Endoxifen in der endgültigen Zusammensetzung; oder (b) (Z)-Endoxifen und (E)-Endoxifen in einem Z:E-Verhältnis im Bereich von 99:1 bis 1:99, von 90:10 bis 10:90, von 85:15 bis 15:85, von 80:20 bis 20:80, von 75:25 bis 25:75, von 70:30 bis 30:70, von 65:35 bis 35:65, von 60:40 bis 40:60, von 55:45 bis 45:55 und etwa 50:50.

4. Topische Zusammensetzung nach einem der vorhergehenden Ansprüche, wobei die topische Zusammensetzung Folgendes umfasst:
a. zu 0,01% bis 20% (Gew./Gew.) Endoxifen freie Base oder ein Salz oder ein Solvat davon; und/oder
b. zu 0,01% bis 10% (Gew./Gew.) (Z)-Endoxifen freie Base oder ein Salz oder ein Solvat davon.

5. Topische Zusammensetzung nach einem der vorhergehenden Ansprüche, wobei das Endoxifensalz Endoxifengluconat, Endoxifen-HCl oder Endoxifencitrat oder ein Solvat davon ist.

6. Topische Zusammensetzung nach einem der vorhergehenden Ansprüche, die Folgendes umfasst:
(i)
zu 0,01% bis 10% (Gew./Gew.) (Z)-Endoxifen freie Base oder Salze oder Solvate davon;
(ii)
a. zu 0,01% bis 10% (Gew./Gew.) (Z)-Endoxifen freie Base oder Salze oder Solvate davon;
b. eine erste Verbindung, die zu 10% bis 90% (Gew./Gew.) Diethylenglycolmonoethylether umfasst;
c. eine zweite Verbindung, die zu 5% bis 80% (Gew./Gew.) einen Penetrationsverbesserer umfasst, der aus der Gruppe ausgewählt ist, die aus DMSO, Diethylsebacat, Diisopropyladipat, Dipropylenglycol, Polyethylenglycolen, Isopropanol, t-Butanol, Polyethylenglycoldodecylether, Cetylalkohol, Mineralöl, Caprylsäuretryiglycerid, Caprinsäuretriglycerid, Capryl-/Caprinsäuretriglyceriden und Stearinsäure oder einer Kombination davon besteht;
(iii)
a. zu 0,01% bis 10% (Gew./Gew.) (Z)-Endoxifen freie Base oder Salze oder Solvate davon;
b. eine erste Verbindung, die Diethylenglycolmonoethylether umfasst;
c. eine zweite Verbindung, die Isopropanol und Mineralöl umfasst;
(iv)
a. zu 0,01% bis 10% (Gew./Gew.) (Z)-Endoxifen freie Base oder Salze oder Solvate davon;
b. eine erste Verbindung, die Diethylenglycolmonoethylether umfasst;
c. eine zweite Verbindung, die Isopropanol und Caprylsäure-/Caprinsäureglyceride umfasst;
(v)
a. zu 0,01% bis 10% (Gew./Gew.) (Z)-Endoxifen freie Base oder Salze oder Solvate davon;
b. eine erste Verbindung, die zu 10% bis 90% (Gew./Gew.) Diethylenglycolmonoethylether umfasst;
c. eine zweite Verbindung, die Folgendes umfasst: zu 5% bis 30% (Gew./Gew.) Isopropanol; zu 10% bis 80% (Gew./Gew.) Caprylsäure-/Caprinsäuretriglyceride oder Mineralöl oder beides;
(vi)
a. zu 0,01% bis 10% (Gew./Gew.) (Z)-Endoxifen freie Base oder Salze oder Solvate davon;
b. eine erste Verbindung, die zu 10% bis 90% (Gew./Gew.) Diethylenglycolmonoethylether umfasst;
c. eine zweite Verbindung, die Folgendes umfasst: zu 5% bis 30% (Gew./Gew.) Isopropanol; zu 25% bis 45% (Gew./Gew.) Caprylsäure-/Caprinsäuretriglyceride; und q.s. 100% (Gew./Gew.) mit Mineralöl; und/oder
(vii)
a. zu 0,01% bis 10% (Gew./Gew.) (Z)-Endoxifen freie Base oder Salze oder Solvate davon;
b. eine erste Verbindung, die zu 15% bis 45% (Gew./Gew.) Diethylenglycolmonoethylether umfasst;
c. eine zweite Verbindung, die Folgendes umfasst: zu 5% bis 15% (Gew./Gew.) Isopropanol; zu 25% bis 45% (Gew./Gew.) Caprylsäure-/Caprinsäuretriglyceride; und q. s. 100% (Gew./Gew.) mit Mineralöl.

7. Topische Zusammensetzung nach Anspruch 1, wobei die topische Zusammensetzung Folgendes umfasst:
(i) 1% (Gew./Gew.) Endoxifen, 20% (Gew./Gew.) Diethylsebacat, 39% (Gew./Gew.) Dimethylsulfoxid, 10% (Gew./Gew.) Diisopropyladipat, 10% (Gew./Gew.) Dipropylenglycol und 20% (Gew./Gew.) PEG 300;
(ii) 1% (Gew./Gew.) Endoxifen, 40% (Gew./Gew.) Diethylenglycolmonoethylether, 30% (Gew./Gew.) Caprinsäuretriglycerid, 19% (Gew./Gew.) Sojaöl und 10% (Gew./Gew.) Isopropanol;
(iii) 1% (Gew./Gew.) Endoxifen, 20% (Gew./Gew.) Diethylsebacat, 10% (Gew./Gew.) Diisopropyladipat, 29% (Gew./Gew.) PEG 300 und 40% (Gew./Gew.) Diethylenglycolmonoethylether;
(iv) 1% (Gew./Gew.) Endoxifen, 45% (Gew./Gew.) Dimethylsulfoxid, 10% (Gew./Gew.) Dipropylenglycol, 29% (Gew./Gew.) PEG 300, 10% (Gew./Gew.) Isopropanol und 5% (Gew./Gew.) Polyethylenglycoldodecylether (Brij^{™} L4);
(v) 1% (Gew./Gew.) Endoxifen, 20% (Gew./Gew.) Diethylsebacat, 30% (Gew./Gew.) Dimethylsulfoxid, 9% (Gew./Gew.) Diisopropyladipat, 10% (Gew./Gew.) Dipropylenglycol und 30% (Gew./Gew.) Diethylenglycolmonoethylether;
(vi) 1% (Gew./Gew.) Endoxifen, 20% (Gew./Gew.) Diethylsebacat, 10% (Gew./Gew.) Diisopropyladipat, 19% (Gew./Gew.) Diethylenglycolmonoethylether, 40% (Gew./Gew.) Caprinsäuretriglycerid und 10% (Gew./Gew.) Isopropanol;
(vii) 1% (Gew./Gew.) Endoxifen, 20% (Gew./Gew.) Dimethylsulfoxid, 40% (Gew./Gew.) Caprinsäuretriglycerid, 29% (Gew./Gew.) Sojaöl und 10% (Gew./Gew.) Isopropanol;
(viii) 1% (Gew./Gew.) Endoxifen, 19% (Gew./Gew.) Diethylenglycolmonoethylether, 40% (Gew./Gew.) Caprinsäuretriglycerid, 10% (Gew./Gew.) Isopropanol und 30% (Gew./Gew.) Mineralöl;
(ix) 1% (Gew./Gew.) Endoxifen, 30% (Gew./Gew.) Diethylenglycolmonoethylether, 30% (Gew./Gew.) Caprinsäuretriglycerid, 10% (Gew./Gew.) Isopropanol, 25% (Gew./Gew.) Mineralöl und 4% (Gew./Gew.) Cetylalkohol;
(x) 1% (Gew./Gew.) Endoxifen, 29% (Gew./Gew.) Diethylenglycolmonoethylether, 30% (Gew./Gew.) Caprinsäuretriglycerid, 10% (Gew./Gew.) Isopropanol, 20% (Gew./Gew.) Mineralöl, 5% (Gew./Gew.) Cetylalkohol und 5% (Gew./Gew.) Stearinsäure;
(xi) 1% (Gew./Gew.) Endoxifen, 19% (Gew./Gew.) Diethylenglycolmonoethylether, 40% (Gew./Gew.) Caprinsäuretriglycerid, 10% (Gew./Gew.) Isopropanol und 20% (Gew./Gew.) Mineralöl; oder
(xii) 1% (Gew./Gew.) Endoxifen, 10% (Gew./Gew.) Diethylsebacat, 19% (Gew./Gew.) Diethylenglycolmonoethylether, 40% (Gew./Gew.) Caprinsäuretriglycerid, 10% (Gew./Gew.) Isopropanol und 10% (Gew./Gew.) Mineralöl.

8. Topische Zusammensetzung nach einem der vorhergehenden Ansprüche, die ferner Folgendes umfasst:
a. einen pharmazeutisch akzeptablen Hilfsstoff;
b. ein Verdickungsmittel, einen Penetrationsverbesserer, ein Emolliens, ein Tensid, ein Antioxidans, ein antimikrobielles Mittel, einen Hautpflegewirkstoff, ein Mittel mit kontrollierter Freisetzung oder eine Kombination davon; und/oder
c. ein zweites Therapeutikum; wobei das zweite Therapeutikum optional aus der Gruppe ausgewählt ist, die aus Folgenden besteht: Bicalutamid, Enzalutamid, Abirateronacetat, einem onkologischen Wirkstoff wie etwa Antineoplastika wie etwa Capecitabin (Xeloda), Carboplatin (Paraplatin), Cisplatin (Platinol), Cyclophosphamid (Neosar), Docetaxel (Docefrez, Taxotere), Doxorubicin (Adriamycin), pegyliertem liposomalem Doxorubicin (Doxil), Epirubicin (Ellence), Fluorouracil (5-FU, Adrucil), Gemcitabin (Gemzar), Methotrexat (mehrere Markenbezeichnungen), Paclitaxel (Taxol), proteingebundenem Paclitaxel (Abraxane), Vinorelbin (Navelbine), Eribulin (Halaven), Ixabepilon (Ixempra), Goserelinacetat, Trastuzumab, Ado-Trastuzumab, Bevacizumab, Everolimus, Checkpoint-Inhibitoren (wie etwa Pembrolizumab (Keytruda^{™}), Nivolumab (Opdivo^{™}), Atezolizumab (Tecentriq^{™}), Durvalumab (Imfinzi^{™}) und Avelumab (Bavencio^{™}), und Inhibitoren von ABC-Binding-Cassette-Reportern wie etwa BCRP-Inhibitoren und P-gp-Inhibitoren.

9. Topische Zusammensetzung nach einem der vorhergehenden Ansprüche, wobei die topische Zusammensetzung:
a. einen Wassergehalt von weniger als 1% aufweist oder wobei die Zusammensetzung eine Dielektrizitätskonstante von weniger als 50 aufweist oder beides; und/oder
b. bei Umgebungstemperatur mindestens 18 Monate lang stabil ist.

10. Topische Zusammensetzung nach einem der vorhergehenden Ansprüche, wobei die Endoxifen oder ein Salz oder ein Solvat davon umfassende topische Zusammensetzung in einer Einzeldosis von Endoxifen oder einem Salz oder einem Solvat davon von 0,01 mg, 0,05 mg, 0,1 mg, 0,25 mg, 0,5 mg, 0,75 mg, 1 mg, 1,5 mg, 2 mg, 3 mg, 4 mg, 5 mg, 6 mg, 7 mg, 8 mg, 9 mg, 10 mg, 15 mg, 20 mg, 25 mg, 50 mg, 75 mg, 100 mg, und 200 mg formuliert ist.

11. Topische Zusammensetzung nach einem der Ansprüche 1 bis 10 zur Verwendung in einem Verfahren zur Behandlung eines Individuums, das eine hormonabhängige Erkrankung der Brustdrüse, eine hormonabhängige Erkrankung der Geschlechtsorgane oder beides hat oder bei dem ein Risiko für diese besteht.

12. Topische Zusammensetzung zur Verwendung nach Anspruch 11, wobei:
a. die hormonabhängige Erkrankung der Brustdrüse oder die hormonabhängige Erkrankung der Geschlechtsorgane eine gutartige Erkrankung der Brustdrüse, Brustdrüsenhyperplasie, Brustdrüsenatypie, atypische duktale Hyperplasie, atypische lobuläre Hyperplasie, höhere Dichte der Brustdrüse, Gynäkomastie, McCune-Albright-Syndrom, ein verfrühtes Eintreten der Pubertät, ein DCIS, LCIS, Brustkrebs, Endometriumkrebs, Eierstockkrebs, Gebärmutterkrebs, Gebärmutterhalskrebs, Vaginalkrebs oder Vulvakrebs ist;
b. das Individuum Prostatakrebs hat; und wobei bei dem Individuum eine Chemotherapie eingeleitet wurde oder in Kürze eingeleitet wird;
c. das Individuum, das die hormonabhängige Erkrankung der Brustdrüse oder hormonabhängige Erkrankung der Geschlechtsorgane hat oder bei dem ein Risiko für diese besteht, gegenüber Tamoxifen refraktär oder gegen Tamoxifen resistent ist; und/oder
d. das Individuum eine geringe Endoxifen-Plasmakonzentration, bezogen auf das Tamoxifenmetabolitenprofil des Individuums im Vergleich mit einer Endoxifen-Plasmakonzentration in einem Tamoxifenmetaboliten-Referenzprofil, im Anschluss an eine vorherige Verabreichung einer Tamoxifen umfassenden Zusammensetzung zeigt.

13. Topische Zusammensetzung zur Verwendung nach Anspruch 11 oder Anspruch 12, wobei:
a. die topische Zusammensetzung Endoxifen oder ein Salz oder ein Solvat davon in einer Einzeldosis im Bereich von 0,01 mg bis 200 mg umfasst;
b. die topische Zusammensetzung (Z)-Endoxifen oder ein Salz oder ein Solvat davon umfasst in:
(i) einer Einzeldosis im Bereich von 0,01 mg bis 100 mg;
(ii) einer Dosis von 2,0 mg, 6 mg oder 10 mg;
(iii) einer Dosis von 1,0 mg, 3,0 mg oder 5 mg pro Brustdrüse;
c. die topische Zusammensetzung einmal täglich, zweimal täglich, dreimal täglich, viermal täglich, jeden zweiten Tag, zweimal wöchentlich, wöchentlich, vierzehntägig, zweimal monatlich, monatlich, vierteljährlich, einmal alle sechs Monate oder jährlich verabreicht wird; und/oder
d. das Verfahren das Verabreichen der topischen Zusammensetzung, um das Endoxifen im Plasma des Individuums bei einer Steady-State-Konzentration von ≤ 30 nM zu halten.

14. Topische Zusammensetzungen zur Verwendung nach einem der Ansprüche 11 bis 13, wobei das Verfahren das Verabreichen der topischen Zusammensetzung entweder allein oder in Kombination mit einem zweiten Therapeutikum umfasst; wobei das zweite Therapeutikum optional aus der Gruppe ausgewählt ist, die aus Folgenden besteht: Bicalutamid, Enzalutamid, Abirateronacetat, einem onkologischen Wirkstoff wie etwa Antineoplastika wie etwa Capecitabin (Xeloda), Carboplatin (Paraplatin), Cisplatin (Platinol), Cyclophosphamid (Neosar), Docetaxel (Docefrez, Taxotere), Doxorubicin (Adriamycin), pegyliertem liposomalem Doxorubicin (Doxil), Epirubicin (Ellence), Fluorouracil (5-FU, Adrucil), Gemcitabin (Gemzar), Methotrexat (mehrere Markenbezeichnungen), Paclitaxel (Taxol), proteingebundenem Paclitaxel (Abraxane), Vinorelbin (Navelbine), Eribulin (Halaven), Ixabepilon (Ixempra), Goserelinacetat, Trastuzumab, Ado-Trastuzumab, Bevacizumab, Everolimus, Checkpoint-Inhibitoren (wie etwa Pembrolizumab (Keytruda^{™}), Nivolumab (Opdivo^{™}), Atezolizumab (Tecentriq^{™}), Durvalumab (Imfinzi^{™}) und Avelumab (Bavencio^{™}), und Inhibitoren von ABC-Binding-Cassette-Reportern wie etwa BCRP-Inhibitoren und P-gp-Inhibitoren.

## Revendications

1. Composition topique comprenant :
a. de l'endoxifène ou un sel ou un solvate de celui-ci ;
b. un premier composé ; et
c. un deuxième composé ; et
dans laquelle le premier composé comprend :
i. de l'éther monoéthylique de diéthylène glycol, et le deuxième composé comprend un activateur de pénétration sélectionné dans le groupe constitué du diméthylsulfoxyde (DMSO), du sébaçate de diéthyle, de l'adipate de diisopropyle, du dipropylène glycol, de polyéthylènes glycols, de l'isopropanol, du t-butanol, du dodécyléther de polyéthylène glycol, de l'alcool cétylique, d'une huile minérale, d'un triglycéride caprylique, d'un triglycéride caprique, de triglycérides capryliques/capriques et de l'acide stéarique, ou d'une combinaison de ceux-ci ; ou
ii. du DMSO et le deuxième composé comprend un activateur de pénétration sélectionné dans le groupe constitué de l'éther monoéthylique de diéthylène glycol, du sébaçate de diéthyle, de l'adipate de diisopropyle, du dipropylène glycol, de polyéthylènes glycols, de l'isopropanol, du t-butanol, du dodécyléther de polyéthylène glycol, de l'alcool cétylique, d'une huile minérale, d'un triglycéride caprylique, d'un triglycéride caprique, de triglycérides capryliques/capriques et de l'acide stéarique, ou d'une combinaison de ceux-ci.

2. Composition topique selon la revendication 1, dans laquelle :
a. la concentration totale du premier composé et du deuxième composé est de jusqu'à environ 95 %, jusqu'à environ 90 %, jusqu'à environ 85 %, jusqu'à environ 80 %, jusqu'à environ 75 %, jusqu'à environ 70 %, jusqu'à environ 65 %, jusqu'à environ 60 %, jusqu'à environ 55 %, jusqu'à environ 50 %, jusqu'à environ 45 %, jusqu'à environ 40 %, jusqu'à environ 35 %, jusqu'à environ 30 %, jusqu'à environ 25 %, jusqu'à environ 20 %, jusqu'à environ 15 %, jusqu'à environ 10 % ou jusqu'à environ 5 % p/p de la composition topique ;
b. la concentration totale du premier composé et du deuxième composé est dans la plage de 10 % à 90 % p/p de la composition ;
c. la concentration totale du premier composé est dans la plage de 10 % à 90 % (p/p) de la composition topique ; et la concentration totale du deuxième composé est dans la plage de 5 % à 80 % (p/p) de la composition topique ; et/ou
d. le rapport du premier composé au deuxième composé :
(i) est dans la plage de 1:9 à 9:1 ;
(ii) est dans la plage de 1:4 à 4:1 ;
(iii) est dans la plage de 1:2 à 2:1 ; et/ou
(iv) est d'environ 1:1.

3. Composition topique selon la revendication 1 ou la revendication 2, dans laquelle l'endoxifène ou un sel ou un solvate de celui-ci comprend : (a) au moins 40 % de (Z)-endoxifène sous forme de base libre (p/p) relativement à l' endoxifène total dans la composition finale ; ou b) du (Z)-endoxifène et du (E)-endoxifène selon un rapport Z:E dans la plage de 99:1 à 1:99, de 90:10 à 10:90, de 85:15 à 15:85, de 80:20 à 20:80, de 75:25 à 25:75, de 70:30 à 30:70, de 65:35 à 35:65, de 60:40 à 40:60, de 55:45 à 45:55 et d'environ 50:50.

4. Composition topique selon l'une quelconque des revendications précédentes, la composition topique comprenant :
a. 0,01 % à 20 % (p/p) d'endoxifène sous forme de base libre ou d'un sel ou d'un solvate de celui-ci ; et/ou
b. 0,01 % à 10 % (p/p) de (Z)-endoxifène sous forme de base libre ou d'un sel ou d'un solvate de celui-ci.

5. Composition topique selon l'une quelconque des revendications précédentes, dans laquelle le sel d'endoxifène est le gluconate d'endoxifène, le chlorhydrate d'endoxifène ou le citrate d'endoxifène, ou un solvate de ceux-ci.

6. Composition topique selon l'une quelconque des revendications précédentes, comprenant :
(i)
0,01 % à 10 % (p/p) de (Z)-endoxifène sous forme de base libre ou de sels ou de solvates de celui-ci ;
(ii)
a. 0,01 % à 10 % (p/p) de (Z)-endoxifène sous forme de base libre ou de sels ou de solvates de celui-ci ;
b. un premier composé comprenant 10 % à 90 % (p/p) d'éther monoéthylique de diéthylène glycol ;
c. un deuxième composé comprenant 5 % à 80 % (p/p) d'un activateur de pénétration sélectionné dans le groupe constitué du DMSO, du sébaçate de diéthyle, de l'adipate de diisopropyle, du dipropylène glycol, de polyéthylènes glycols, de l'isopropanol, du t-butanol, du dodécyléther de polyéthylène glycol, de l'alcool cétylique, d'une huile minérale, d'un triglycéride caprylique, d'un triglycéride caprique, de triglycérides capryliques/capriques et de l'acide stéarique, ou d'une combinaison de ceux-ci ;
(iii)
a. 0,01 % à 10 % (p/p) de (Z)-endoxifène sous forme de base libre ou de sels ou de solvates de celui-ci ;
b. un premier composé comprenant de l'éther monoéthylique de diéthylène glycol ;
c. un deuxième composé comprenant de l'isopropanol et une huile minérale ;
(iv)
a. 0,01 % à 10 % (p/p) de (Z)-endoxifène sous forme de base libre ou de sels ou de solvates de celui-ci ;
b. un premier composé comprenant de l'éther monoéthylique de diéthylène glycol ;
c. un deuxième composé comprenant de l'isopropanol et des glycérides capryliques/capriques ;
(v)
a. 0,01 % à 10 % (p/p) de (Z)-endoxifène sous forme de base libre ou de sels ou de solvates de celui-ci ;
b. un premier composé comprenant 10 % à 90 % (p/p) d'éther monoéthylique de diéthylène glycol ;
c. un deuxième composé comprenant 5 % à 30 % (p/p) d'isopropanol ; 10 % à 80 % (p/p) de triglycérides capryliques/capriques ou d'huile minérale, ou les deux ;
(vi)
a. 0,01 % à 10 % (p/p) de (Z)-endoxifène sous forme de base libre ou de sels ou de solvates de celui-ci ;
b. un premier composé comprenant 10 % à 90 % (p/p) d'éther monoéthylique de diéthylène glycol ;
c. un deuxième composé comprenant 5 % à 30 % (p/p) d'isopropanol ; 25 % à 45 % (p/p) de triglycérides capryliques/capriques ; et de l'huile minérale q.s. 100 % (p/p) ; et/ou
(vii)
a. 0,01 % à 10 % (p/p) de (Z)-endoxifène sous forme de base libre ou de sels ou de solvates de celui-ci ;
b. un premier composé comprenant 15 % à 45 % (p/p) d'éther monoéthylique de diéthylène glycol ;
c. un deuxième composé comprenant 5 % à 15 % (p/p) d'isopropanol ; 25 % à 45 % (p/p) de triglycérides capryliques/capriques ; et de l'huile minérale q.s. 100 % (p/p).

7. Composition topique selon la revendication 1, la composition topique comprenant :
(i) 1 % (p/p) d'endoxifène, 20 % (p/p) de sébaçate de diéthyle, 39 % (p/p) de diméthylsulfoxyde, 10 % (p/p) d'adipate de diisopropyle, 10 % (p/p) de dipropylène glycol et 20 % (p/p) de PEG 300 ;
(ii) 1 % (p/p) d'endoxifène, 40 % (p/p) d'éther monoéthylique de diéthylène glycol, 30 % (p/p) de triglycéride caprique, 19 % (p/p) d'huile de soja et 10 % (p/p) d'isopropanol ;
(iii) 1 % (p/p) d'endoxifène, 20 % (p/p) de sébaçate de diéthyle, 10 % (p/p) d'adipate de diisopropyle, 29 % (p/p) de PEG 300 et 40 % (p/p) d'éther monoéthylique de diéthylène glycol ;
(iv) 1 % (p/p) d'endoxifène, 45 % (p/p) de diméthylsulfoxyde, 10 % (p/p) de dipropylène glycol, 29 % (p/p) de PEG 300, 10 % (p/p) d'isopropanol et 5 % (p/p) de dodécyléther de polyéthylène glycol (Brij^{™} L4) ;
(v) 1 % (p/p) d'endoxifène, 20 % (p/p) de sébaçate de diéthyle, 30 % (p/p) de diméthylsulfoxyde, 9 % (p/p) d'adipate de diisopropyle, 10 % (p/p) de dipropylène glycol et 30 % (p/p) d'éther monoéthylique de diéthylène glycol ;
(vi) 1 % (p/p) d'endoxifène, 20 % (p/p) de sébaçate de diéthyle, 10 % (p/p) d'adipate de diisopropyle, 19 % (p/p) d'éther monoéthylique de diéthylène glycol, 40 % (p/p) de triglycéride caprique et 10 % (p/p) d'isopropanol ;
(vii) 1 % (p/p) d'endoxifène, 20 % (p/p) de diméthylsulfoxyde, 40 % (p/p) de triglycéride caprique, 29 % (p/p) d'huile de soja et 10 % (p/p) d'isopropanol ;
(viii) 1 % (p/p) d'endoxifène, 19 % (p/p) d'éther monoéthylique de diéthylène glycol, 40 % (p/p) de triglycéride caprique, 10 % (p/p) d'isopropanol et 30 % (p/p) d'huile minérale ;
(ix) 1 % (p/p) d'endoxifène, 30 % (p/p) d'éther monoéthylique de diéthylène glycol, 30 % (p/p) de triglycéride caprique, 10 % (p/p) d'isopropanol, 25 % (p/p) d'huile minérale et 4 % (p/p) d'alcool cétylique ;
(x) 1 % (p/p) d'endoxifène, 29 % (p/p) d'éther monoéthylique de diéthylène glycol, 30 % (p/p) de triglycéride caprique, 10 % (p/p) d'isopropanol, 20 % (p/p) d'huile minérale, 5 % (p/p) d'alcool cétylique et 5 % (p/p) d'acide stéarique ;
(xi) 1 % (p/p) d'endoxifène, 19 % (p/p) d'éther monoéthylique de diéthylène glycol, 40 % (p/p) de triglycéride caprique, 10 % (p/p) d'isopropanol et 20 % (p/p) d'huile minérale ; ou
(xii) 1 % (p/p) d'endoxifène, 10 % (p/p) de sébaçate de diéthyle, 19 % (p/p) d'éther monoéthylique de diéthylène glycol, 40 % (p/p) de triglycéride caprique, 10 % (p/p) d'isopropanol et 10 % (p/p) d'huile minérale.

8. Composition topique selon l'une quelconque des revendications précédentes, comprenant en outre :
a. un excipient pharmaceutiquement acceptable ;
b. un agent épaississant, un activateur de pénétration, un émollient, un tensioactif, un antioxydant, un antimicrobien, un agent actif de soin de la peau, un agent à libération contrôlée, ou une combinaison de ceux-ci ; et/ou
c. un deuxième agent thérapeutique ; optionnellement dans laquelle le deuxième agent thérapeutique est sélectionné dans le groupe constitué du bicalutamide, de l'enzalutamide, de l'acétate d'abiratérone, d'un médicament oncologique tel que des antinéoplasiques tels que la capécitabine (Xeloda), le carboplatine (Paraplatin), le cisplatine (Platinol), le cyclophosphamide (Neosar), le docétaxel (Docefrez, Taxotere), la doxorubicine (Adriamycin), la doxorubicine liposomale pégylée (Doxil), l'épirubicine (Ellence), le fluorouracile (5-FU, Adrucil), la gemcitabine (Gemzar), le méthotrexate (multiples noms de marque), le paclitaxel (Taxol), le paclitaxel lié à une protéine (Abraxane), la vinorelbine (Navelbine), l'éribuline (Halaven), l'ixabépilone (Ixempra), l'acétate de goséréline, le trastuzumab, l'ado-trastuzumab, le bévacizumab, l'évérolimus, d'inhibiteurs de point de contrôle (tels que le pembrolizumab (Keytruda^{™}), le nivolumab (Opdivo^{™}), l'atézolizumab (Tecentriq^{™}), le durvalumab (Imfinzi^{™}) et l'avélumab (Bavencio^{™})), et d'inhibiteurs de reporters ATP-Binding Cassette (ABC) tels que les inhibiteurs de BCRP et les inhibiteurs de P-gp.

9. Composition topique selon l'une quelconque des revendications précédentes, la composition topique :
a. ayant une teneur en eau inférieure à 1 % ou la composition ayant une constante diélectrique inférieure à 50, ou les deux ; et/ou
b. est stable à la température ambiante pendant au moins 18 mois.

10. Composition topique selon l'une quelconque des revendications précédentes, la composition topique comprenant de l'endoxifène ou un sel ou un solvate de celui-ci étant formulée à une dose unitaire d'endoxifène ou d'un sel ou d'un solvate de celui-ci de 0,01 mg, 0,05 mg, 0,1 mg, 0,25 mg, 0,5 mg, 0,75 mg, 1 mg, 1,5 mg, 2 mg, 3 mg, 4 mg, 5 mg, 6 mg, 7 mg, 8 mg, 9 mg, 10 mg, 15 mg, 20 mg, 25 mg, 50 mg, 75 mg, 100 mg et 200 mg.

11. Composition topique selon l'une quelconque des revendications 1 à 10, destinée à une utilisation dans une méthode de traitement d'un sujet atteint ou à risque d'être atteint d'une pathologie mammaire hormonodépendante, d'une pathologie hormonodépendante de l'appareil génital, ou des deux.

12. Composition topique destinée à une utilisation selon la revendication 11, dans laquelle :
a. la pathologie mammaire hormonodépendante ou la pathologie hormonodépendante de l'appareil génital est une affection bénigne du sein, une hyperplasie, une atypie, une hyperplasie canalaire atypique, une hyperplasie lobulaire atypique, une augmentation de la densité mammaire, une gynécomastie, un syndrome de McCune-Albright, une puberté précoce, un carcinome intracanalaire in situ (CIIS), un carcinome lobulaire in situ (CLIS), un cancer du sein, un cancer de l'endomètre, un cancer de l'ovaire, un cancer de l'utérus, un cancer du col de l'utérus, un cancer du vagin ou un cancer de la vulve ;
b. le sujet est atteint d'un cancer de la prostate ; et le sujet a commencé ou est sur le point de commencer une chimiothérapie ;
c. le sujet est atteint ou à risque d'être atteint d'une pathologie mammaire hormonodépendante ou d'une pathologie hormonodépendante de l'appareil génital réfractaire au tamoxifène ou résistante au tamoxifène ; et/ou
d. le sujet présente un faible taux plasmatique d'endoxifène basé sur le profil de métabolites du tamoxifène du sujet comparativement à un taux plasmatique d'endoxifène dans un profil de métabolites du tamoxifène de référence après administration préalable d'une composition comprenant du tamoxifène.

13. Composition topique destinée à une utilisation selon la revendication 11 ou la revendication 12, dans laquelle :
a. la composition topique comprend de l'endoxifène ou un sel ou un solvate de celui-ci à une dose unitaire dans la plage de 0,01 mg à 200 mg ;
b. la composition topique comprend du (Z)-endoxifène ou un sel ou un solvate de celui-ci à :
i) une dose unitaire dans la plage de 0,01 mg à 100 mg ;
(ii) une dose de 2,0 mg, 6 mg ou 10 mg ;
(iii) une dose de 1,0 mg, 3,0 mg ou 5 mg par sein ;
c. la composition topique est administrée une fois par jour, deux fois par jour, trois fois par jour, quatre fois par jour, tous les deux jours, deux fois par semaine, une fois par semaine, toutes les deux semaines, deux fois par mois, une fois par mois, tous les trois mois, une fois tous les six mois, ou une fois par an ; et/ou
d. la méthode comprend l'administration de la composition topique pour maintenir le taux plasmatique d'endoxifène du sujet à un niveau stable de ≤ 30 nM.

14. Composition topique destinée à une utilisation selon l'une quelconque des revendications 11 à 13, la méthode comprenant l'administration de la composition topique seule ou en association avec un deuxième agent thérapeutique ; le deuxième agent thérapeutique étant optionnellement sélectionné dans le groupe constitué du bicalutamide, de l'enzalutamide, de l'acétate d'abiratérone, d'un médicament oncologique tel que des antinéoplasiques tels que la capécitabine (Xeloda), le carboplatine (Paraplatin), le cisplatine (Platinol), le cyclophosphamide (Neosar), le docétaxel (Docefrez, Taxotere), la doxorubicine (Adriamycin), la doxorubicine liposomale pégylée (Doxil), l'épirubicine (Ellence), le fluorouracile (5-FU, Adrucil), la gemcitabine (Gemzar), le méthotrexate (multiples noms de marque), le paclitaxel (Taxol), le paclitaxel lié à une protéine (Abraxane), la vinorelbine (Navelbine), l'éribuline (Halaven), l'ixabépilone (Ixempra), l'acétate de goséréline, le trastuzumab, l'ado-trastuzumab, le bévacizumab, l'évérolimus, d'inhibiteurs de point de contrôle (tels que le pembrolizumab (Keytruda^{™}), le nivolumab (Opdivo^{™}), l'atézolizumab (Tecentriq^{™}), le durvalumab (Imfinzi^{™}), l'avélumab (Bavencio^{™})), et d'inhibiteurs de reporters ATP-Binding Cassette (ABC) tels que les inhibiteurs de BCRP et les inhibiteurs de P-gp.
